(19) Europäisches Patentamt
European Patent Office
Office européen des brevets

(11) EP 1 640 379 A1

(12) EUROPEAN PATENT APPLICATION
published in accordance with Art. 158(3) EPC

(43) Date of publication:
**29.03.2006 Bulletin 2006/13**

(21) Application number: **04746992.9**

(22) Date of filing: **29.06.2004**

(51) Int Cl.:
*C07H 1/00 (1974.07)* *C07H 15/04 (1974.07)*
*C07H 15/18 (1974.07)* *C07H 15/203 (1985.01)*
*C07H 3/06 (1974.07)*

(86) International application number:
**PCT/JP2004/009523**

(87) International publication number:
**WO 2005/000861 (06.01.2005 Gazette 2005/01)**

(84) Designated Contracting States:
**AT BE BG CH CY CZ DE DK EE ES FI FR GB GR HU IE IT LI LU MC NL PL PT RO SE SI SK TR**

(30) Priority: **30.06.2003 JP 2003187878**

(71) Applicant: **MITSUBISHI CHEMICAL CORPORATION**
**Tokyo 108-0014 (JP)**

(72) Inventor: **KANIE, Osamu**
**Machida-shi**
**Tokyo 1948511 (JP)**

(74) Representative: **Alcock, David et al**
**D Young & Co**
**120 Holborn**
**London EC1N 2DY (GB)**

# (54) METHOD OF SYNTHESIZING SUGAR CHAIN

(57) An object of the present invention is to provide a method for efficiently chemically synthesizing biomolecules including a nucleotide (nucleic acid), a peptide (protein), or a sugar chain, as representative examples. The present invention provides a method of solid-phase synthesis of sugar chain(s) for synthesizing multiple types of sugar chains in at least one sugar chain synthesis reaction system comprising multiple types of monosaccharide units, which is **characterized in that** it comprises changing the temperature in the sugar chain synthesis reaction system depending on the temperature rising rate that has been determined based on a decrease in side reaction(s) in the reaction system as an indicator.

FIG. 1

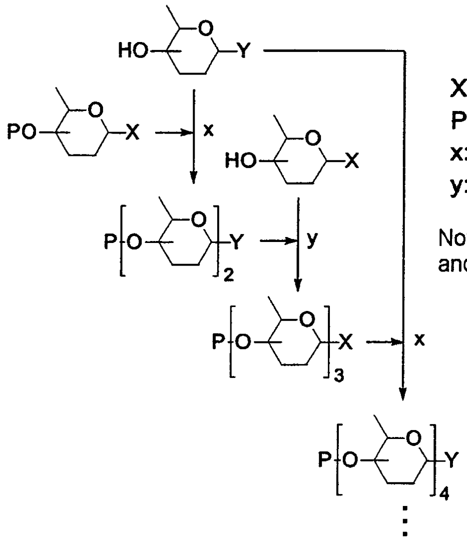

X,Y: leaving group
P: protecting group
x: activating condition to X
y: activating condition to Y

Note: Y is stable under condition x, and X is stable under condition y.

EP 1 640 379 A1

## Description

### TECHNICAL FIELD

The present invention relates to a chemical synthesis of sugar chain(s) that constitutes a group of biological components having important functions, and particularly to a solid-phase synthesis of sugar chain(s).

### BACKGROUND ART

The chemical synthesis of an oligosaccharide chain and a complex thereof has been reported (Paulsen H. (1982) *Angew. Chem. Int. Ed. Engl.* 21, 155-173; Toshima K., Tatsuta K. (1993) *Chem. Rev* 93, 1503-1531; and Boons G.-J. (1996) *Tetrahedron, 52,* 1095-1121). However, in the current chemical synthesis, isolation and purification operations by chromatography, whose operations are complicated and which requires a long reaction time, are necessary after completion of a reaction, and thus operators are required to be familiar with such operations (Kanie O., Hindsgaul O. (1992) *Curr. Opin. Struc. Biol.* 2, 674-681). A solid-phase synthesis is one means for solving such problems. The solid-phase synthesis method is also used for the synthesis of peptides or nucleotides, and it has high effectiveness. In recent years, it has been considered that the stereoselective formation of a glycosidic linkage attended with a coupling reaction and the tactful use of a protecting group for site selective glycosylation are essential for the synthesis of an oligosaccharide chain, but that these are difficult tasks to be achieved. However, as a result of previous studies, several successful cases have been reported (Fréchet J. M., Schuerch C. (1972) *Carbohydr. Res.* 22, 399-412; Chiu S.-H. L., Anderson L. (1976) *Carbohydr. Res.* 50, 227-238; Yan L., Taylor C. M., Goodnow R. Jr., Kahne D. (1994) *J. Am. Chem. Soc.* 116, 6953-6954; Redemann J., Schmidt R. R. (1997) *J. Org. Chem.* 62, 3650-3653; Heckel A., Mross E., Jung K.-H., Rademann J., Schmidt R. R. (1998) Synlett 171-173; Nicolaou K. C., Winssinger N., Pastor J., DeRoose F. (1997) *J. Am. Chem. Soc.* 119, 449-450; Nicolaou K. C., Watanabe N., Li J., Pastor J., Wissinger N. (1998) *Angew Chem. Int. Ed. Engl.* 37, 1559-1561; Rodebaugh R., Joshi S., Fraser-Reid B., Geysen H. M. (1997) *J. Org. Chem.* 62, 5660-5661; Danishefsky S. J., McClure K. F., Randolph J. T., Ruggeri R. B. (1993) *Science,* 260, 1307-1309; Zheng C., Seeberger P. H., Danishefsky S. J. (1998) *J. Org. Chem.* 63, 1126-1130; Ito Y, Kanie O., Ogawa T. (1996) *Angew. Chem. Int. Ed. Engl.* 35, 2510-2512; Douglas S. P., Whitfield D. M., Krepinsky J. J. (1995) *J. Am. Chem. Soc.* 117, 2116-2117; Verduyn R., van der Klein P. A. M., Douwes M., van der Marel G. A., van Boom J. H. (1993) *J. R. Neth. Chem. Soc.* 112, 464-466; Shimizu H., Ito Y, Kanie O., Ogawa T. (1996) *Bioorg. Med. Chem. Lett. 6,* 2841-2846; Adoinolfi M., Barone G., DeNapoli L., Ladonisi A., Piccialli G. (1996) *Tetrahedron Lett.* 37, 5007-5010; Seberger P. H., Beebe X., Sukenick G. D., Pochapsky S., Danishefsky S. J. (1997) *Angew. Chem. Int. Ed. Engl.,* 36, 491-493; Kanemitsu T., Kanie O., Wong C.-H. (1998) *Angew. Chem. Int. Ed. Engl.* 37, 3415-3418; and WO02/16384).

Based on such a large number of findings, a sugar chain library, which is considered to be useful for efficient sugar chain synthesis, has also been synthesized in large numbers. For example, Hindsgaul et al. have radically reconsidered the basis of sugar chain chemistry and have attempted the glycosylation reaction (random glycosylation) of an unprotected sugar receptor with a Bn-protected sugar donor (imidate). As a result, they have found that a random condensate mixture with no site selectivity can easily be obtained. This method is not directed to the synthesis of a single compound, but the sequence depends on the order of the reaction. Moreover, the given mixture has been screened with glycosyltransferase, and it has been shown that it specifically picks up a known reaction (Kanie O., Barresi F., Ding Y, Labbe J., Otter A., Forsberg L. S., Ernst B., Hindsgaul O. (1995) *Angew Chem. Int. Ed. Engl.* 34, 2720-2722; and Ding Y, Kanie O., Labbe J., Palcic M. M., Ernst B., Hindsgaul O. "Synthesis and Biological Activity of Oligosaccharide Libraries" in Glycoimmunology, Eds. by Alavi, A., Axford, J.S., (1995) *Avd. Exp. Med. Biol.* 376, Plenum press, New York, pp.259-269).

Kahne and Still et al. have reported the synthesis of a library by the split and mix method involving glycosylation and induction of an amino group on a solid-phase. Tagging has been performed for each step, and after deprotection on a solid-phase, a ligand structure to lectin, which is stronger than the known ligands, has been discovered by assay and decoding (Liang R., Yan L., Loebach J., Ge M., Uozumi Y, Sekanina K., Horan N., Gildersleeve J., Thompson C., Smith A., Biswas K., Still W.C., Kahne D. (1996) *Science* 274, 1520-1522). Boons et al. have synthesized a trisaccharide library by a synthetic method tactfully using double bond isomerization of allyl-vinyl for the synthesis of a sugar chain, and thus they have demonstrated the possibility of the method in the future. In this method, they have used a solution reaction and have succeeded in obtaining a library, wherein a sugar binding position is determined by the split and mix method (Boons G.-J., Heskamp B., Hout F. (1996) *Angew Chem. Int. Ed. Engl.* 35, 2845-2847). Boons et al. have also attempted a chemical species-selective glycosylation reaction on a solid-phase, and at the same time, they have synthesized a library. They have reported 12 trisaccharide libraries (Johnson M., Arles C., Boons G.-J. (1998) *Tetrahedron Lett.* 39, 9801-9804; and Zhu T., Boons G.-J. (1998) *Angew. Chem. Int. Ed. Engl.* 37, 1898-1900). Ichikawa et al. have activated glycal by IDCP and have then conducted the coupling thereof with an unprotected diol monosaccharide receptor. As a result, they have synthesized a 2-deoxy sugar trisaccharide library, which is stereoselective but does not have site

selectivity (Izumi M., Ichikawa Y. (1998) *Tetrahedron Lett.* 39, 2079-2082). Wong et al. have used orthogonally protected monosaccharide (Baranay, G, Merrifield, R.B., (1977) J. Am.Chem. Soc. 116, 7363-7365) as a parent nucleus and have deprotected protecting groups in the order of necessity, so as to synthesize a pentasaccharide protected sugar library, which was highly branched from a linear trisaccharide (Wong C.-H., Ye X.-S., Zhang Z. (1998) *J. Am. Chem. Soc.* 120, 7137-7138). In addition, they have examined carefully the reactivity of a protecting group at an anomeric position (thioglycoside) in a chemical species selective glycosylation reaction and have completed one-pot glycosylation by armed-disarmed glycosylation. Based on such one-pot glycosylation, they have conducted selection of a reaction via the use of computers and elimination of an orthogonal protecting group, thereby opening the way for automation of a sugar chain library (Zhang Z., Ollmann I. R., Ye X.-S., Wischnat R., Baasov T., Wong C.-H. (1999) *J. Am. Chem. Soc.* 121, 734-753). In 1994, Armstrong et al. have sharply focused attention on a sugar chain library, and as a result of osmium oxidation and reductive cyclization of diene having carboxylic acid at the terminus thereof, they have announced a stereoisomeric library of a hydroxyl group of a reducing terminal sugar of C-glycosyl disaccharide (Armstrong R. W., Sutherlin D. P. (1994) *Tetrahedron Lett.* 35, 7743-7745).

Moreover, WO96/06102 and Australian Patent Publication No. AU334649 (issue date: March 16, 1996) describe a method for constructing a sugar chain library. In WO96/06102 and Australian Patent Publication No. AU3346495 (issue date: March 16, 1996), a sugar receptor that does not have a protecting group is used, a protected sugar donor is allowed to chemically react with the sugar receptor, so as to quickly synthesize a sugar chain library at a random site.

Furthermore, US. Patent No. 5,721,099 describes a method for constructing nucleic acid, a peptide, a sugar chain or the like, on a resin by organic synthesis chemical means. In US. Patent No. 5,721,099, molecular coding is carried out on a single resin. A library is synthesized by the spit and mix method, and thus a resin mixture consisting of a single resin and a single compound is given. At the end of the synthesis, all deprotections are carried out, and the resultant product is directly used for screening for a biomolecule such as a protein. Since only a trace amount of a synthetic substance is synthesized, only hitted beads are decoded, so as to specify a compound structure on the beads.

However, it is difficult to say that the aforementioned large number of synthesis examples and construction examples of sugar libraries have been successful. For example, in order to carry out a stereoselective or site selective glycosylation reaction, a large number of monosaccharide units are required. However, since enormous manpower and cost are required therefor, it has been difficult to comprehensively synthesize them.

For such reasons, it is strongly desired that an efficient method of solid-phase synthesis of sugar chain(s) for easily synthesizing a sugar chain library containing a sufficient number of sugar chains be established.

On the other hand, as described above, a solid-phase reaction method that is commonly used for the synthesis of a sugar chain or the like has been widely used also for the synthesis of a peptide or nucleotide, and its effectiveness is highly evaluated. That is to say, using a solid-phase for such reactions, isolation and purification operations and other operations are simplified, resulting in high efficiency. Thus, even persons other than experts are able to deal with such operations. Further, this method greatly contributes to the development of devices or the like.

For example, with regard to peptide synthesis and nucleotide (nucleic acid) synthesis, basic synthesis methods have been almost established, and a large number of synthesizers have been commercially available (refer to US. Patent No. 5,462,748, for example). However, all of these synthesizers are of large size and require a large amount of solution. Thus, there is still room for improvements such as miniaturization of devices, reduction in the amount of reagent, or cost reduction. In addition, a device for synthesizing a sugar chain has not yet been commercially available. Although there have been several attempts (refer to WO02/16384, for example), all of such synthesizers have been problematic, and thus they have not come into practical use. For the aforementioned reasons, it is strongly desired that a method for efficiently carrying out a solid-phase reaction using a small amount of liquid, and a small-sized device used therefor, be developed, and in particular that a device for synthesizing a sugar chain be developed.

## DISCLOSURE OF THE INVENTION

It is an object of the present invention to provide a method for efficiently chemically synthesizing biomolecules including a nucleotide (nucleic acid), a peptide (protein), or a sugar chain, as representative examples. It is a particular object of the present invention to provide a method, which constitutes a basis of a device for synthesizing a sugar chain, regarding which no commercially available synthesizers exist.

In order to achieve the aforementioned objects, the present inventors have conducted intensive studies. The inventors have first synthesized various types of monosaccharide units that enable the synthesis of a sugar chain, and the efficiency of the combinatorial chemistry of a sugar chain. Subsequently, the inventors have focused attention on the control of the temperature rising rate in a reaction system, when multiple types of sugar chains are synthesized by a method of solid-phase synthesis of sugar chain(s) using the synthesized multiple types of monosaccharide units. They have found that a sugar chain can be efficiently chemically synthesized by changing the temperature in the sugar chain synthesis reaction system depending on the temperature rising rate that has been determined based on a decrease in side reaction(s) in the reaction system as an indicator. Moreover, the present inventors have also found that the solid-phase synthesis of biomolecules such as sugar chain(s) can be efficiently carried out by performing a reaction that is controlled by diffusion without using processes such as stirring. The present invention has been completed based on these findings.

That is to say, the present invention provides the following features of the invention:

(1) A method of solid-phase synthesis of sugar chain(s) for synthesizing multiple types of sugar chains in at least one sugar chain synthesis reaction system comprising multiple types of monosaccharide units, which is characterized in that it comprises changing the temperature in the sugar chain synthesis reaction system depending on the temperature rising rate that has been determined based on a decrease in side reaction(s) in the reaction system as an indicator.

(2) The method of solid-phase synthesis of sugar chain(s) according to (1) above, which comprises the following steps:

(a) a step of allowing a monosaccharide unit represented by the following formula (2):

$$HO\text{-}A^2\text{-}Y \qquad (2)$$

(wherein $A^2$ represents a monosaccharide skeleton wherein a hydroxyl group that is not involved in the reaction is protected by a protecting group; and Y represents a leaving group that is stable under conditions for activating a leaving group X),
to react with a monosaccharide unit represented by the following formula (1):

$$P\text{-}L\text{-}O\text{-}A^1\text{-}X \qquad (1)$$

(wherein P represents a solid-phase; L represents a linker, O represents an oxygen atom at the nonreducing terminus of the monosaccharide; $A^1$ represents a monosaccharide skeleton wherein a hydroxyl group that is not involved in the reaction is protected by a protecting group; and X represents a leaving group that is stable under conditions for activating the leaving group Y) under conditions for activating the leaving group X, so as to obtain sugars represented by the following formula (3):

$$P\text{-}L\text{-}O\text{-}A^1\text{-}O\text{-}A^2\text{-}Y \qquad (3);$$

and
(b) a step of allowing a monosaccharide unit represented by the following formula (4):

$$HO\text{-}A^3\text{-}X' \qquad (4)$$

(wherein $A^3$ represents a monosaccharide skeleton wherein a hydroxyl group that is not involved in the reaction is protected by a protecting group; X' represents a leaving group that is stable under conditions for activating the leaving group Y or a protecting group of a hydroxyl group at position 1),
to react with the sugars represented by formula (3) obtained in the above-described step (a) under conditions for activating the leaving group Y, so as to obtain sugars represented by the following formula (5):

$$P\text{-}L\text{-}O\text{-}A^1\text{-}O\text{-}A^2\text{-}O\text{-}A^3\text{-}X' \qquad (5).$$

(3) The method of solid-phase synthesis of sugar chain(s) according to (2) above, wherein, in step (b), the monosaccharide unit represented by formula (4), wherein X' represents a protecting group of a hydroxyl group at position 1, is used to synthesize a sugar chain wherein 3 sugars are connected with one another.

(4) The method of solid-phase synthesis of sugar chain(s) according to (2) above, which comprises repeatedly performing steps (a) and (b) on the sugars represented by formula (5) obtained in step (b).

(5) The method of solid-phase synthesis of sugar chain(s) according to any one of (2) to (4) above, wherein the monosaccharide units represented by formulas (1), (2), and (4) are allowed to simultaneously react in a single reaction system, so as to carry out the reaction.

(6) The method of solid-phase synthesis of sugar chain(s) according to any one of (2) to (5) above, wherein the leaving group X is one of a phenylthio group or a fluorine group, and the leaving group Y is the other of a phenylthio group or a fluorine group.

(7) The method of solid-phase synthesis of sugar chain(s) according to (6) above, wherein the phenylthio group is activated with N-iodosuccinimide-trifluoromethanesulfonic acid (NIS-TfOH) or dimethyl(methylthio)sulfonium triflate (DMTST), and wherein the fluorine group is activated with $Sn(ClO_4)_2$, $Sn(OTf)_2$, $Cp_2Hf(ClO_4)_2$, or $Cp_2Hf(OTf)_2$.

(8) The method of solid-phase synthesis of sugar chain(s) according to any one of (2) to (7) above, wherein the protecting group is a benzyl group.

(9) The method of solid-phase synthesis of sugar chain(s) according to any one of (2) to (8) above, wherein monosaccharide skeletons represented by $A^1$, $A^2$, and $A^3$ are the monosaccharide skeletons of 3 types of sugars selected from among monosaccharides existing in a living body.

(10) The method of solid-phase synthesis of sugar chain(s) according to any one of (2) to (9) above, wherein monosaccharide skeletons represented by $A^1$, $A^2$, and $A^3$ are the monosaccharide skeletons of mannose, glucose, galactose, xylitose, glucosamine, galactosamine, glucuronic acid, fructose, or sialic acid.

(11) A monosaccharide library used for carring out the method of solid-phase synthesis of sugar chain(s) according to any one of (1) to (10) above, which comprises at least one monosaccharide derivative selected from the group consisting of
phenyl 1-thio-3,4,6-tri-O-benzyl-β-D-mannopyranoside,
phenyl 1-thio-2,4,6-tri-O-benzyl-β-D-mannopyranoside,
phenyl 1-thio-2,3,6-tri-O-benzyl-β-D-mannopyranoside,
phenyl 1-thio-2,3,4-tri-O-benzyl-β-D-mannopyranoside,
phenyl 1-thio-3,4,6-tri-O-benzyl-β-D-galactopyranoside,
phenyl 1-thio-2,4,6-tri-O-benzyl-β-D-galactopyranoside,
phenyl 1-thio-2,3,6-tri-O-benzyl-β-D-galactopyranoside,
phenyl 1-thio-2,3,4-tri-O-benzyl-β-galactopyranoside,
phenyl 2,3-di-O-benzyl-1-thio-β-D-xylopyranoside,
phenyl 3,4-di-O-benzyl-1-thio-β-D-xylopyranoside,
phenyl 2,4-di-O-benzyl-1-thio-β-D-xylopyranoside,
phenyl 1-thio-2,3-di-O-benzyl-β-L-fucopyranoside,
phenyl 2-azido-4,6-di-O-benzyl-2-deoxy-1-thio-β-D-galactopyranoside,
phenyl 2-azido-3,6-di-O-benzyl-2-deoxy-1-thio-β-D-galactopyranoside,
phenyl 2-azido-3,4-di-O-benzyl-2-deoxy-1-thio-β-D-galactopyranoside,
phenyl 3,4,6-tri-O-benzyl-1-thio-β-D-glucopyranoside,
phenyl 2,4,6-tri-O-benzyl-1-thio-β-D-glucopyranoside,
phenyl 2,3,6-tri-O-benzyl-1-thio-β-D-glucopyranoside,
phenyl 2,3,4-tri-O-benzyl-1-thio-β-D-glucopyranoside,
phenyl 2-azido-4,6-di-O-benzyl-2-deoxy-1-thio-β-D-glucopyranoside,
phenyl 2-azido-3,4-di-O-benzyl-2-deoxy-1-thio-β-D-glucopyranoside,
phenyl 2-azido-3,6-di-O-benzyl-2-deoxy-1-thio-β-D-glucopyranoside,
methyl(phenyl 1-thio-2,3-di-O-benzyl-(3-D-glucopyranoside)uronate,
methyl(phenyl 5-acetamide-4,7,8,9-tetra-O-acetyl-3,5-dideoxy-2-thio -β-D-glycero-D-galacto-2-nonulopyranoside) onate,
methyl(phenyl 5-acetamide-4,7,8,9-tetra-O-acetyl-3,5-dideoxy-2-thio -α-D-glycero-D-galacto-2-nonulopyranoside) onate,
methyl(phenyl 5-acetamide-3,5-dideoxy-2-thio-β-D-glycero-D-galacto -2-nonulopyranoside)onate,
methyl(phenyl 5-acetamide-3,5-dideoxy-2-thio-α-D-glycero-D-galacto -2-nonulopyranoside)onate,
and compounds obtained by substituting a phenylthio group with a fluorine group in the aforementioned compounds.

(12) A method for carrying out a solid-phase reaction in a pore of a solid material with a size necessary for capillary phenomenon, which is characterized in that the reaction is carried out in a state where an excess amount of liquid-phase does not exist on the outer surface of the solid material.

(13) The method for carrying out a solid-phase reaction according to (12) above, which is characterized in that the reaction is carried out by diffusive mixing.

(14) The method for carrying out a solid-phase reaction according to (12) or (13) above, wherein the solid material is a resin particle having a pore therein.

(15) The method for carrying out a solid-phase reaction according to any one of (12) to (14) above, wherein the solid-phase reaction is a chemical reaction or a biochemical reaction.

(16) The method for carrying out a solid-phase reaction according to any one of (12) to (15) above, wherein the solid-phase reaction is a sugar chain synthesis reaction.

(17) The method for carrying out a solid-phase reaction according to any one of (12) to (16) above, wherein the solid-phase reaction is the solid-phase synthesis reaction of sugar chain(s) according to any one of (1) to (10) above.

(18) A library, which is composed of sugar chains consisting of all combinations of 3 types of sugars selected from the monosaccharides existing in a living body, which are synthesized by the method of solid-phase synthesis of sugar chain(s) according to any one of (1) to (10) above or the solid-phase reaction method according to (17) above.

(19) The library according to (18) above, wherein the monosaccharides existing in a living body are mannose,

glucose, galactose, xylitose, glucosamine, galactosamine, glucuronic acid, fructose, or sialic acid.

(20) A reaction device used for carrying out the solid-phase reaction method according to any one of (12) to (17) above, which comprises at least the following (1) and (2):

(1) a reaction unit for carrying out a solid-phase reaction, which has an introduction unit for injecting or discharging a liquid-phase and a space for accommodating a solid material; and

(2) a temperature-controlling unit for controlling the temperature of the reaction unit.

BRIEF DESCRIPTION OF THE DRAWINGS

Figure 1 shows the summary of an orthogonal method used in the present invention.

Figure 2 shows a monosaccharide derivative library used in the present invention.

Figure 3 shows the pore of a resin bead.

Figure 4 shows the aspect of a conventional solid-phase reaction method and the aspect of the diffusive mixing solid-phase reaction method of the present invention.

Figure 5 shows $2^3$ anomeric isomers.

Figure 6 shows the analysis results of MALDI-TOFMS obtained when the temperature was increased from -15°C to 10°C over 3 hours (8.3°C/hour) in a glycosylation reaction by activation of glycosyl fluoride on a solid-phase.

Figure 7 shows the analysis results of MALDI-TOFMS obtained when the temperature was increased from -15°C to 10°C over 4.5 hours (5.6°C/hour) in a glycosylation reaction by activation of glycosyl fluoride on a solid-phase.

Figure 8 shows the analysis results of MALDI-TOFMS obtained when the temperature was increased from -15°C to 10°C over 6 hours (4.2°C/hour) in a glycosylation reaction by activation of glycosyl fluoride on a solid-phase.

Figure 9 shows the analysis results of MALDI-TOFMS obtained when the temperature was increased from -15°C to 10°C over 4.5 hours (5.6°C/hour) in a glycosylation reaction by activation of thioglycoside on a solid-phase.

Figure 10 shows the analysis results of MALDI-TOFMS obtained when the temperature was increased from -15°C to 10°C over 6 hours (4.2°C/hour) in a glycosylation reaction by activation of thioglycoside on a solid-phase.

Figure 11 shows the analysis results of MALDI-TOFMS obtained when the temperature was increased from -15°C to 10°C over 12 hours (2.1°C/hour) in a glycosylation reaction by activation of thioglycoside on a solid-phase.

Figure 12 shows the results of a reaction course study, wherein a glycosylation reaction is carried out by a method comprising swelling, with a receptor solution, a resin to which a donor binds and adding a promoter thereto.

Figure 13 shows the results of a reaction course study, wherein a glycosylation reaction is carried out by a method comprising swelling, with a mixed solution consisting of a receptor and a promoter, a resin to which a donor binds.

Figure 14 shows the MALDI-TOFMS of 2,3-di-*O*-benzyl-L-fucopyranosyl-(1→2)3,4,6-tri-*O*-benzyl-α-D-galactopyranosyl fluoride, which binds to a resin.

Figure 15 shows the MALDI-TOFMS of 2,3-di-*O*-benzyl-L-fucopyranosyl-(1→3)2,4,6-tri-*O*-benzyl-α-D-galactopyranosyl fluoride, which binds to a resin.

Figure 16 shows the MALDI-TOFMS of 2,3-di-*O*-benzyl-L-fucopyranosyl-(1→4)2,3,6-tri-*O*-benzyl-α-D-galactopyranosyl fluoride, which binds to a resin.

Figure 17 shows the MALDI-TOFMS of 2,3-di-*O*-benzyl-L-fucopyranosyl-(1→6)2,3,4-tri-*O*-benzyl-α-D-galactopyranosyl fluoride, which binds to a resin.

Figure 18 shows the MALDI-TOFMS of a trisaccharide derivative synthesized on a solid-phase.

Figure 19 shows the MALDI-TOFMS of a trisaccharide derivative synthesized on a solid-phase.

Figure 20 shows the MALDI-TOFMS of a trisaccharide derivative synthesized on a solid-phase.

Figure 21 shows the MALDI-TOFMS of a trisaccharide derivative synthesized on a solid-phase.

Figure 22 shows the MALDI-TOFMS of a trisaccharide derivative synthesized on a solid-phase.

Figure 23 shows the MALDI-TOFMS of a trisaccharide derivative synthesized on a solid-phase.

Figure 24 shows the MALDI-TOFMS of a trisaccharide derivative synthesized on a solid-phase.

Figure 25 shows the MALDI-TOFMS of a trisaccharide derivative synthesized on a solid-phase.

BEST MODE FOR CARRYING OUT THE INVENTION

The embodiments of the present invention will be described below. However, the descriptions of constitutional elements given below are representative examples of the embodiments of the present invention. Thus, the present invention is not limited to such contents. It is to be noted that a solid-phase synthesis reaction of sugar chain(s), a solid-phase used, a liquid-phase used, and others, which will be described below, can be selected as appropriate from known methods described in *"Koso Gosei Handbook* (Solid-Phase Synthesis Handbook) (published by Novabiochem

(Merck))," for example, unless otherwise specified.

(1) Method of solid-phase synthesis of sugar chain(s) involving controlled temperature rising rate

The method of solid-phase synthesis of sugar chain(s) of the present invention is a method for synthesizing multiple types of sugar chains in at least one sugar chain synthesis reaction system comprising multiple types of monosaccharide units, which is characterized in that it comprises changing the temperature in the above-described sugar chain synthesis reaction system depending on the temperature rising rate that has been determined based on a decrease in side reaction(s) in the reaction system as an indicator.

In the case of the conventional sugar chain synthesis reaction, it has been known that the reaction temperature of a reaction system needs to be minutely controlled because the optimal reaction temperature differs depending on the type of sugar to be reacted. This is because if such a reaction temperature were not sufficiently controlled, unreacted products, eliminated products, and others would be generated, so that a sufficient amount of reaction product could not be obtained. Various types of sugars exist to be used, and such sugars have stereoisomers or positional isomers. Since reactivity differs even depending on such factors, if the optimal reaction temperature were determined for every sugar to be used for the reaction, enormous quantities of studies would be required. Hence, when a large number of reactions have previously been carried out, the optimal reaction temperature has not been determined for every sugar to be used for the reactions. Instead, an average temperature, which is considered to be very common, has been determined, and the reactions have been carried out. Accordingly, it has previously been difficult to obtain a sufficient amount of reaction product in many cases.

As a result of intensive studies directed towards solving such problems, the present inventors have found that when a reaction is carried out using at least one sugar chain synthesis reaction system comprising multiple types of monosaccharide units, and preferably using two or more sugar chain synthesis reaction systems, if the temperatures of these reaction systems are changed depending on the temperature rising rate that has been determined based on a decrease in side reaction(s) in the reaction system as an indicator, the solid-phase synthesis reaction of sugar chain(s) can efficiently be carried out without the need for examining the optimal reaction temperature for each monosaccharide unit. That is to say, the inventors have found that by changing the reaction temperature, each monosaccharide unit contained in each reaction system can be reacted at the point of its optimal temperature, so that a sugar chain synthesis reaction can be carried out. In addition, as a result of further studies by the inventors, it was revealed that if the aforementioned temperature rising rate is determined based on a decrease in side reaction(s) occurring in the reaction system as an indicator, such a solid-phase reaction of sugar chain(s) can be more efficiently carried out.

The thus completed method of solid-phase synthesis of sugar chain(s) of the present invention brings on great effects, whereby many types of sugar chains can be efficiently synthesized without the need for examining all the reaction temperatures in a solid-phase synthesis reaction of sugar chain(s), which has previously caused a serious problem.

A sugar chain synthesis reaction itself in the present invention can be carried out by means known to persons skilled in the art. Specifically, a solution containing a second monosaccharide unit, a solution containing a third monosaccharide unit, and as desired, a solution containing a fourth or more monosaccharide unit, are successively added to a first monosaccharide unit binding to a solid-phase in a suitable solvent that is inactive for the reaction, and thus they are allowed to react with one another, so that such monosaccharide units can be allowed to successively bind to one another. It is to be noted that when the term "sugar chain" is simply used in the present specification, it means a chain product formed by binding two or more monosaccharides to each other.

The types of multiple types of monosaccharide units used in the present invention are not particularly limited, as long as they are monosaccharides. Preferred examples may include those containing a monosaccharide skeleton selected from among monosaccharides existing in a living body. Particularly preferred monosaccharide skeletons may includes the monosaccharide skeletons of mannose, glucose, galactose, xylitose, glucosamine, galactosamine, glucuronic acid, fructose, and sialic acid. In the present invention, using a plurality of these monosaccharide units, multiple types of sugar chains are synthesized in at least one sugar chain synthesis reaction system. Such a sugar chain synthesis reaction system may be either a single reaction system, or two or more reaction systems.

When the reaction is carried out in a single reaction system, one or two or more types of solutions containing monosaccharide units are added to such a single reaction system containing a solid-phase to which one or two or more types of monosaccharide units bind, so as to carry out sugar chain synthesis reaction(s), thereby synthesizing sugar chain(s) consisting of two monosaccharides. Subsequently, one or two or more types of solutions containing monosaccharide units are added to this reaction system, so as to carry out the next sugar chain synthesis reaction(s), thereby synthesizing sugar chain(s) consisting of three monosaccharides. Thereafter, by repeating the aforementioned steps, sugar chain(s) having a desired length can be synthesized. However, when multiple types of sugar chains are simultaneously synthesized in a single reaction system as described above, an operation to separate and purify each sugar chain is required in each step of the reaction or at the final stage.

When the reaction is carried out in two or more reaction systems, for example, two sets of two types of reaction

systems each containing a solid-phase, to which each different monosaccharide unit (e.g., X1 and X2) binds, are prepared (four reaction systems in total), and solutions each containing a different monosaccharide unit (e.g., Y1 and Y2) are added to the reaction systems, so as to carry out a sugar chain synthesis reaction, thereby synthesizing a sugar chain consisting of two monosaccharides. Thereby, a disaccharide consisting of X1 and Y1, a disaccharide consisting of X1 and Y2, a disaccharide consisting of X2 and Y1, and a disaccharide consisting of X2 and Y2, are generated in the four reaction systems. Thereafter, each of the thus obtained reaction products is divided into a half, and a solution containing a different unit (e.g., Z1 and Z2) is added to each reaction system, so as to carry out a sugar chain synthesis reaction, thereby synthesizing a sugar chain consisting of three monosaccharides. Thereby, all of 8 combinations (X1, X2, Y1, Y2, Z1, and Z2) of reaction products can be obtained in each reaction system. Since it is not necessary to separate and purify the sugar chain of interest after completion of the reaction, the method for carrying out the reaction in multiple reaction systems is preferably used in the present invention. When 8 types of sugar chains are intended to be synthesized as described above, for example, 4 reaction systems are previously prepared for the first reaction, and 8 reaction systems are previously prepared for the second reaction. Thereafter, the reactions are preferably carried out in these reaction systems simultaneously.

One characteristic of the present invention is that the temperature in the aforementioned sugar chain synthesis reaction system is changed depending on the temperature rising rate that has been determined based on a decrease in side reaction(s) in the reaction system as an indicator. As described in the examples of the present specification, it was revealed that by-products are generated, as the temperature rising rate increases, and that the reaction efficiency of the sugar chain synthesis reaction thereby decreases. Accordingly, in the present invention, the temperature of a sugar chain synthesis reaction system is changed depending on the temperature rising rate that has been determined based on a decrease in side reaction(s) in the reaction system as an indicator, so as to efficiently carry out the sugar chain synthesis reaction.

Herein, the term "side reaction" is used to mean a reaction of generating by-products that hinder the reaction efficiency of a desired sugar chain synthesis reaction, and such by-products mean elimination reaction products (eliminated products), hydrolysates, or the like. An example of such eliminated products is glycal generated as a result of an intramolecular reaction occurring instead of a glycosylation reaction that is a bimolecular reaction of interest. That is, the expression "based on a decrease in side reaction(s) as an indicator" is used in the present invention to mean that "a state, in which the presence or quantities of by-products are low to such an extent that they do not inhibit the reaction of interest, is used as an indicator," for example. A state, in which the quantities of eliminated products are sufficiently low, is particularly preferable. In addition, since if the reaction efficiency is poor, large quantities of unreacted products remain, it is more preferable that a decrease in such unreacted products be also used as an indicator. The presence or quantities of by-products can be detected and confirmed by the mass spectrometry method (MS), the nuclear magnetic resonance spectrometry method (NMR), high performance liquid chromatography (HPLC), determination of absorbance, determination of optical rotation, etc.

Any method for controlling the temperature may be applied, as long as it is able to carry out a sugar chain synthesis reaction, while changing the temperatures of the aforementioned single reaction system or two or more reaction systems depending on the determined temperature rising rate. Specifically, the temperature may be controlled using a commonly used known thermoregulator or the like, which can be used for chemical reactions or the like. When a single reaction system is used, the temperature of the reaction system may be changed depending on the temperature rising rate that has previously been determined. When two or more reaction systems are used, the temperature of each reaction system may be changed depending on the temperature rising rate that has previously been determined, or the temperatures of multiple reaction systems may be controlled by a single thermoregulator. In particular, when a sugar chain library is synthesized, a method for controlling the temperatures of multiple reaction systems by a single thermoregulator, so as to simultaneously synthesize a large number of sugar chains, is preferably applied.

The temperature rising rate or the reaction initiation temperature is appropriately determined depending on the type of monosaccharide used or the type of leaving group used. If the reaction temperature is high, side reaction(s) are likely to progress. On the other hand, if the reaction initiation temperature or the temperature rising rate is too low, the reaction time becomes longer. In a preferred example of the present invention, a monosaccharide unit having a fluorine group or a phenylthio group as a leaving group is allowed to react with another monosaccharide unit having a hydroxyl group under conditions for activating the fluorine group or the phenylthio group. At this time, the temperature rising rate is, for example, 8°C/hour or less, preferably 7°C/hour or less, and more preferably 5.6°C/hour or less. The preferred range of the temperature rising rate differs depending on a leaving group or monosaccharide used. Thus, such a preferred range may be determined as appropriate by conducting experiments. The reaction initiation temperature differs depending on a monosaccharide unit used. Such a reaction initiation temperature may be determined depending on a monosaccharide with the highest reactivity (side reaction(s) are likely to occur) from among monosaccharides existing in the reaction system, and the temperature may be then increased from the thus determined temperature. In the case of fucose with the highest reactivity (for which the reaction temperature is set at low) from among 9 types of monosaccharides existing in a living body used as a preferred example in the present invention, the reaction can be initiated from the

temperature between approximately -30°C and -15°C. Thus, by determining the temperature rising rate and reaction initiation temperature that are suitable for each monosaccharide, the generation of side reaction(s) is suppressed as much as possible, and a sugar chain synthesis reaction can be carried out with high efficiency.

A sugar chain is considered to be a third biopolymer following a nucleic acid and a protein, and it plays an extremely important role in the biological functions of multicellular organisms, such as cell differentiation, immunity, canceration, or infection. Chemical synthesizers are commercially available regarding nucleic acids and proteins, and such synthesizers have greatly contributed to the progress and advancement of science. However, chemical synthesizers for sugar chains are not commercially available, and thus early development of such synthesizers is desired [Plante O. J., Palmacci E. R., Seeberger P. H. (2001) *Science,* 291, 1523-1527]. A solid-phase synthesis constitutes a basis for synthesis by chemical means. As a preliminary step toward the development of such synthesizers, the efficiency of an elementary reaction or the efficiency of a total process should be carried out. In order to achieve this purpose, the method of solid-phase synthesis of sugar chain(s) of the present invention is extremely useful. Moreover, in the method of the present invention, as stated above, a sugar chain synthesis reaction itself can be carried out by known methods. Among such known methods, an orthogonal glycosylation method that is based on the independency of reactivity (mutual selectivity) (hereinafter referred to as "orthogonal method" at times) is the most suitable method [Kanie O., Ito Y, Ogawa T., (1994) *J. Am. Chem. Soc.,* 116, 12073-12074. b; Kanie O., Ito Y, Ogawa T., (1996) *Tetrahedron Lett.,* 37, 4551-4554. c; and Ito Y, Kanie O., Ogawa T., (1996) *Angew. Chem. Int. Ed. Engl., 35,* 2510-2512].

A specific example of the solid-phase synthesis reaction of sugar chain(s) of the present invention by the aforementioned orthogonal glycosylation method is a reaction comprising steps (a) and (b) mentioned below. The summary of the method is shown in Figure 1.

(a) a step of allowing a monosaccharide unit represented by the following formula (2) (hereinafter referred to as a "receptor" at time):

$$HO\text{-}A^2\text{-}Y \qquad (2)$$

(wherein $A^2$ represents a monosaccharide skeleton wherein a hydroxyl group that is not involved in the reaction is protected by a protecting group; and Y represents a leaving group that is stable under conditions for activating a leaving group X),
to react with a monosaccharide unit represented by the following formula (1) (hereinafter referred to as a "donor" at time):

$$P\text{-}L\text{-}O\text{-}A^1\text{-}X \qquad (1)$$

(wherein P represents a solid-phase; L represents a linker, O represents an oxygen atom at the nonreducing terminus of the monosaccharide; $A^1$ represents a monosaccharide skeleton wherein a hydroxyl group that is not involved in the reaction is protected by a protecting group; and X represents a leaving group that is stable under conditions for activating the leaving group Y) under conditions for activating the leaving group X, so as to obtain sugars represented by the following formula (3):

$$P\text{-}L\text{-}O\text{-}A^1\text{-}O\text{-}A^2\text{-}Y \qquad (3);$$

and
(b) a step of allowing a monosaccharide unit represented by the following formula (4):

$$HO\text{-}A^3\text{-}X' \qquad (4)$$

(wherein $A^3$ represents a monosaccharide skeleton wherein a hydroxyl group that is not involved in the reaction is protected by a protecting group; X' represents a leaving group that is stable under conditions for activating the leaving group Y or a protecting group of a hydroxyl group at position 1),
to react with the sugars represented by formula (3) obtained in the above-described step (a) under conditions for activating the leaving group Y, so as to obtain sugars represented by the following formula (5):

$$P\text{-}L\text{-}O\text{-}A^1\text{-}O\text{-}A^2\text{-}O\text{-}A^3\text{-}X' \qquad (5).$$

That is to say, the orthogonal method is a continuous and efficient synthesis method that utilizes the properties of the aforementioned two types of leaving groups (the aforementioned X and Y), which have different activation conditions and each one of which is stable under the activation conditions of the other group. In the present specification, such two

types of leaving groups are considered to "have an orthogonal relationship."

In the aforementioned reaction, when a sugar chain consisting of 3 connected sugars is synthesized, a monosaccharide unit represented by formula (4) wherein X' represents a protecting group of a hydroxyl group at position 1 can be used in step (b).

In addition, in the aforementioned reaction, when a sugar chain consisting of 4 or more connected sugars is synthesized, the reaction in step (a) and the reaction in step (b) are repeatedly carried out on the sugars represented by formula (5) obtained in step (b), so as to synthesize a sugar chain having a desired length.

A solid-phase that is commonly used for the solid-phase synthesis of sugar chains can be used as a solid-phase represented by P. Specific examples of such a solid-phase may include: various types of resins such as a polystyrene resin, a polyacrylamide resin, a polyether resin, or a mixed resin thereof; porous glasses; beads; micro flow channels (fused silica capillary, microchip, or the like); and sepharose. All of these materials may have functional groups such as an amino group, a bromomethyl group, a hydroxyl group, a thiol group, or a carboxyl group, which constitute scaffolds for connecting a linker, a spacer, or the like during the solid-phase synthesis, and may further have a suitable side chain, a modifying substance, or the like.

Any type of linker can be used herein as a linker represented by L, as long as it is a group that is capable of connecting a solid-phase with an oxygen atom at the nonreducing terminus of a monosaccharide and that is capable of selective cleavage under moderate conditions that do not affect products obtained after the synthesis reaction.

More specifically, as a solid-phase represented by P and a linker represented by L used herein, suitable products can be selected from among solid-phases and linkers that are commonly used for the sugar chain synthesis reaction described, for example, in Kanemitsu T. and Kanie O., Combinatorial Chemistry & High Throughput screening, 5, 339-360 (2002).

$A^1$, $A^2$, and $A^3$ represent monosaccharide skeletons, wherein a hydroxyl group that is not associated with the reaction is protected by a protecting group, and they may be identical to or different from one another. Specific examples of such monosaccharide skeletons are as described above. The type of protecting group for a hydroxyl group is not particularly limited, as long as it is able to protect the hydroxyl group during the sugar chain synthesis reaction. Protecting groups for a hydroxyl group that are known to persons skilled in the organic synthesis field can be used as appropriate. Specific examples of such protecting groups for a hydroxyl group are as follows.

(Ether type)

Methyl group, methoxymethyl group, methylthiomethyl group, benzyloxymethyl group, t-butoxymethyl group, 2-methoxyethoxymethyl group, 2,2,2-trichloroethoxymethyl group, bis(2-chloroethoxy)methyl group, 2-(trimethylsilyl)ethoxymethyl group, tetrahydropyranyl group, 3-bromotetrahydropyranyl group, tetrahydrothiopyranyl group, 4-methoxytetrahydropyranyl group, 4-methoxytetrahydrothiopyranyl group, 4-methoxytetrahydrothiopyranyl-S,S-dioxide group, tetrahydrofuranyl group, tetrahydrothiofuranyl group;

1-ethoxyethyl group, 1-methyl-1-methoxyethyl group, 1-(isopropoxy)ethyl group, 2,2,2-trichloroethyl group, 2-(phenylselenyl)ethyl group, t-butyl group, allyl group, cinnamyl group, p-chlorophenyl group, benzyl group, p-methoxybenzyl group, o-nitrobenzyl group, p-nitrobenzyl group, p-halobenzyl group, p-cyanobenzyl group, 3-methyl-2-picolyl-N-oxide group, diphenylmethyl group, 5-dibenzosuberyl group, triphenylmethyl group, α-naphthyldiphenylmethyl group, p-methoxyphenyldiphenylmethyl group, p-(p'-bromophenacyloxy)phenyldiphenylmethyl group, 9-anthryl group, 9-(9-phenyl)xanthenyl group, 9-(9-phenyl-10-oxo)anthryl group, benzoisothiazolyl-S,S-dioxide group;

Trimethylsilyl group, triethylsilyl group, isopropyldimethylsilyl group, t-butyldimethylsilyl group (TBDMS group), (triphenylmethyl)dimethylsilyl group, t-butyldiphenylsilyl group, methyldiisopropylsilyl group, methyldi-t-butylsilyl group, tribenzylsilyl group, tri-p-xylylsilyl group, triisopropylsilyl group, triphenylsilyl group;

(Ester type)

Formate, benzoylformate, acetate, chloroacetate, dichloroacetate, trichloroacetate, trifluoroacetate, methoxy acetate, triphenylmethoxy acetate, phenoxy acetate, p-chlorophenoxy acetate, 2,6-dichloro-4-methylphenoxy acetate, 2,6-dichloro-4-(1,1,3,3-tetramethylbutyl)phenoxy acetate, 2,4-bis(1,1-dimethylpropyl)phenoxy acetate, chlorodiphenylacetate, p-phenylacetate, 3-phenylpropionate, 3-benzoylpropionate, isobutylate, monosuccinate, 4-oxopentanoate, pivaloate, adamantoate, crotonate, 4-methoxycrotonate, (E)-2-methyl-2-butenoate, benzoate, o-(dibromomethyl)benzoate, o-(methoxycarbonyl)benzoate, p-phenylbenzoate, 2,4,6-trimethylbenzoate, p-benzoate, α-naphthoate;

(Carbonate type)

Methyl carbonate, ethyl carbonate, 2,2,2-trichloroethyl carbonate, isobutyl carbonate, vinyl carbonate, allyl carbonate, cinnamyl carbonate, p-nitrophenyl carbonate, benzyl carbonate, p-methoxybenzyl carbonate, 3,4-dimethoxybenzyl carbonate, o-nitrobenzyl carbonate, p-nitrobenzyl carbonate, S-benzylthio carbonate;

(Other type)

N-phenyl carbamate, N-imidazolyl carbamate, borate, nitrate, N,N,N',N'-tetramethyl phosphorodiamidate, 2,4-dinitrophenyl sulfonate.

A method for introducing the aforementioned protecting group for a hydroxyl group and a deprotection method thereof are known to persons skilled in the art. Such methods are described in Teodora W. Green, Protective Groups in Organic Synthesis, John & Wiley & Sons Inc. (1981), for example.

In the present invention, a benzyl group can be particularly preferably used.

Specific examples of the combination of the leaving group X (or X') that is stable under conditions for activating the leaving group Y, and the leaving group Y that is stable under conditions for activating the leaving group X, may include:

(1) A case where the leaving groups X (or X') is one of an arylthio group or arylseleno group, or fluorine group, and the leaving group Y is the other of an arylthio group or arylseleno group, or fluorine group; and
(2) A case where the leaving groups X (or X') is an ethylthio group ($-SC_2H_5$), and the leaving group Y is a pyridylsulfone group ($-SO_2Pyr$).

Herein, an aryl (Ar) portion in the arylthio group (-S-Ar) or arylseleno group (-Se-Ar) is not particularly limited, as long as it is suitable for activation conditions. Aryl containing 6 or more carbon atoms is preferable. Examples may include phenyl and naphthalene. Phenyl is the most preferable.

Among the aforementioned cases, the case (1) is particularly preferable. In this case, a phenylthio group can be activated with N-iodosuccinimide-trifluoromethanesulfonic acid (NIS-TfOH) or dimethyl(methylthio)sulfonium triflate (DMTST) or the like. In the case of using an activator formed by combining NIS with TfOH (hereinafter referred to as a "promoter" at time), these substances are reacted with each other to generate iodonium ions ($I^+$) in a reaction system, so as to conduct activation. Activators formed by combination of other types of reagents can also used herein as activators, as long as such combination enables highly efficient generation of iodonium ions ($I^+$). DMTST can be directly added to a reaction system as an activator, or for example, MeSSMe (dimethyl disulfide) and MeOTf (methyltrifluoromethanesulfonate (methyltriflate)) can be added to a reaction system, so as to generate DMTST in the system.

Moreover, a fluorine group can be activated with $Sn(ClO_4)_2$ (stannous periodate), $Sn(OTf)_2$ (tin trifluoromethanesulfonate (II)), $Cp_2Hf(ClO_4)_2$ (hafnocene periodate (II)), $Cp_2Hf(OTf)_2$ (hafnocene trifluoromethanesulfonate (II)), etc. $Sn(ClO_4)_2$ can be generated as a result of addition of $SnCl_2$ (tin chloride) and $AgClO_4$ (silver perchlorate) to a reaction system, and a fluorine group can be then activated therewith. Likewise, $Sn(OTf)_2$ can be generated as a result of addition of $SnCl_2$ and AgOTf (silver triflate) to a reaction system, and a fluorine group can be then activated therewith. Also, $Cp_2Hf(ClO_4)_2$ can be generated as a result of addition of $Cp_2HfCl_2$ (hafnocene chloride (II)) and $AgClO_4$ to a reaction system, and a fluorine group can be then activated therewith. Moreover, $Cp_2Hf(OTf)_2$ can be generated as a result of addition of $Cp_2HfCl_2$ and AgOTf to a reaction system, and a fluorine group can be then activated therewith.

The thus synthesized sugar chain binding to a solid-phase (P) can be obtained by washing the solid-phase with a suitable solvent as necessary, and then cleaving it out of the solid-phase using a suitable compound such as sodium methoxide.

In the aforementioned orthogonal method, two types of leaving groups associated with a coupling reaction can be selectively activated. For example, X (arylthio group) can be activated with NIS-TfOH, DMTST, or the like, and Y (fluorine group) can be activated with $Sn(ClO_4)_2$, $Sn(OTf)_2$, $Cp_2Hf(ClO_4)_2$, $Cp_2Hf(OTf)_2$, or the like (Figure 1). Herein, X and Y can be activated in any given order. According to such an orthogonal method, synthesis is carried out in a direction opposite to that of biosynthesis; namely in a direction from the nonreducing terminus to the reducing terminus. At this time, since the leaving group on the receptor side is stable under conditions for activating the donor, a protecting group is unnecessary. Moreover, according to the orthogonal method, a coupling product has already had a leaving group that is used for the next reaction. Thus, this method is advantageous in that it involves the smallest number of steps in the synthesis of a sugar chain.

The effectiveness of the aforementioned orthogonal method can be further increased by applying the method to the method of solid-phase synthesis of sugar chain(s) of the present invention. That is to say, when a synthesis method of initiating the synthesis from the nonreducing terminus (method A) is compared with a synthesis method of initiating the synthesis from the reducing terminus (method B), it is necessary for method B to protect (cap) a functional group (hydroxyl group) derived from an unreacted product in a coupling reaction. Thus, in method B, one cycle consists of 3 steps consisting of coupling, capping, and deprotection. On the other hand, in method A, by-products (hydrolysates and elimination reaction products) are accumulated on a solid-phase for every coupling reaction. However, since such by-products are not reacted under the next coupling reaction conditions, capping is not necessary. Thus, in the case of method A, one cycle consists of 1 step even in a solid-phase reaction. A solid-phase synthesis method has been originally

developed for the purpose of achieving high efficiency, and the method of solid-phase synthesis of sugar chain(s) of the present invention is obtained by improving the above method more efficiently. Hence, it is anticipated that the combined use of the solid-phase synthesis method with the orthogonal method enables more comprehensive efficiency of the reaction process. Moreover, by the combined use of the orthogonal method with a solid-phase reaction method via diffusive mixing, which will be described later in the present specification, advantages such as reduction in the reaction time, the saving of a solvent, or electricity saving are provided, and thus, such combined use is also useful for the development of a solid-phase synthesizer of sugar chains.

(2) Monosaccharide library

In order to freely synthesize sugar chains that play various important roles in living bodies, monosaccharide units which are appropriately protected are necessary. In addition, it is extremely useful to produce a monosaccharide library comprising a large number of monosaccharide units. As described in (1) above, since the comprehensive efficiency of the process is important for highly efficient synthesis of sugar chains, the type of a monosaccharide unit used is important.

Moreover, stereoisomers ($\alpha/\beta$) are generated in a coupling reaction in the synthesis of a sugar chain. As a common synthesis method, a method for selectively synthesizing a stereoisomer of interest is generally applied. However, in a case where combinatorial chemistry is carried out for the purpose of actively creating new drugs, for example, while departing from chemical synthesis directed towards reconstruction of biological components, the presence of a protecting group acting as a control factor necessary for stereoselective coupling not only doubles the number of necessary monosaccharide units, but also multiplies the number of reaction steps necessary for the synthesis of such derivatives. Thus, since molecules become complicated, the type of an available reaction is also restricted. Accordingly, in such combinatorial chemistry, it is preferred that stereo-nonselective synthesis is first carried out, so as to efficiently synthesize a stereoisomer mixture, and thereafter, stereoisomers are separated by HPLC or the like, for example. The method or monosaccharide library of the present invention is preferably used for such means.

As specific examples of monosaccharide units contained in a library, in the method of solid-phase synthesis of sugar chain(s) of the present invention, and particularly in the above method with the combination of the orthogonal method, for example, it is desired that a phenylthio group be used as the above X, that a fluorine group be used as the aforementioned Y, and that benzyl groups be used as all of hydroxyl groups that are not associated with the reaction. Furthermore, since X has an orthogonal relationship with Y, it is desired that a synthetic product be synthesized in the form of a stable phenylthioglycoside product, and that it be then appropriately converted to a fluoride product for use. As a protecting group used herein, an alkyl group that does not interfere with groups adjacent thereto is preferable, and a benzyl group that is commonly used in an organic synthesis reaction is most preferable. In the case of 2-amino sugar, it is preferable that an azido group that does not interfere with groups adjacent thereto be used, and that it be given in the form of 2-azido sugar.

When a monosaccharide library is produced while taking into consideration the synthesis of a sugar chain library, in the case of sialic acid, glucuronic acid, and fucose, which exist only in the nonreducing termini of human sugar chains other than polysaccharides, derivatives wherein only a hydroxyl group at a site binding to a solid-phase is released may be synthesized. In the case of other monosaccharides, namely, glucose, galactose, mannose, xylose, glucosamine, and galactosamine, derivatives wherein only the hydroxyl group of each monosaccharide is released may be synthesized. As with other monosaccharide derivatives, in the case of sialic acid, glucuronic acid, and fucose also, each of hydroxyl groups can be distinguished from one another.

An example of a monosaccharide unit (phenylthioglycoside form), which has been designed by the present inventors, and is utilized in the solid-phase synthesis of sugar chain(s) that is combined with the orthogonal method of the present invention, is shown in Figure 2. A monosaccharide library comprising at least one monosaccharide unit selected from the group consisting of the compound described in Figure 2 (phenylthioglycoside form) and a compound obtained by substituting a phenylthio group with a fluorine group in the above compound (fluoride form) is useful as a monosaccharide library used for applying the method of solid-phase synthesis of sugar chain(s) of the present invention described in (1) above. When steric control is required, a compound obtained by substituting the group with an acyl group can be arbitrarily used. The monosaccharide unit shown in Figure 2 is essential for construction of sugar chains or a sugar chain library comprising the same from 9 types of monosaccharides constituting sugar chains existing in living bodies by chemical synthesis method.

Study papers regarding known compounds from among the monosaccharide units shown in Figure 2 are mentioned below.

Man-2-OH: Marzabadi C. H.; Spilling C. D. (1993) J. *Org. Chem.* 58, 3761-6.

Man-3-OH: Oshitari T.; Shibasaki M.; Yoshizawa T.; Tomita M.; Takao K.; Kobayashi S. (1997) *Tetrahedron* 53, 10993-11006

Man-4-OH: Lemanski G.; Ziegler T. (2000) *Helvetica Chim. Acta* 83, 2655-2675.

Man-6-OH: Lemanski G.; Ziegler T. (2000) *Tetrahedron* 56, 563-579.
Gal-2-OH: Marzabadi C. H.; Spilling C. D. (1993) *J. Org. Chem.* 58, 3761-6.
Gal-3-OH: Kanie O.; Ito Y; Ogawa T. (1996) *Tetrahedron Lett.* 37, 4551-4554.
Gal-4-OH: Greenberg W. A.; Priestley E. S.; Sears P. S.; Alper P. B.; Rosenbohm C.; Hendrix M.; Hung S. -C.; Wong C. -H. (1999) *J. Am. Chem. Soc.* 121, 6527-6541.
Gal-6-OH: Magaud D.; Granjean C.; Doutheau A.; Anker D.; Shevchik V.; Cotte-Pattat N.; Robert-Baudouy J. (1998) *Carbohydr. Res.* 314, 189-199.
Glc-2-OH: Ennis S. C.; Cumpstey L; Fairbanks A. J.; Butters T. D.; Mackeen M.; Wormald M. R. (2002) *Tetrahedron* 58, 9403-9411.
Glc-4-OH: Motawia M. S.; Olsen C. E.; Enevoldsen K.; Marcussen J.; Moeller B. L. (1995) *Carbohydr. Res.* 277, 109-23.
Glc-6-OH: Pfaeffli P. J.; Hixson S. H.; Anderson L. (1972) *Carbohydr. Res.* 231, 195-206.
GlcN-4-OH: Martin-Lomas M.; Flores-Mosquera M.; Chiara J. L. (2000) *Europ. J. Org. Chem.* 1547-1562.
GlcN-6-OH: Takahashi S.; Kuzuhara H.; Nakajima M. (2001) *Tetrahedron* 57*,* 6915-6926.
Sia: Marra A.; Sinay P. (1989) *Carbohydr. Res.,* 187 35-42.

(3) Solid-phase reaction method via diffusive mixing

The present invention further relates to a method for carrying out a solid-phase reaction in a pore of a solid material with a size necessary for capillary phenomenon, which is characterized in that the reaction is carried out in a state where an excess amount of liquid-phase does not exist on the outer surface of the solid material.

Solid-phase synthesis is a synthesis method that is essential for the synthesis of polymeric biomolecules such as a nucleotide, a peptide, or a sugar chain. Also, such solid-phase synthesis constitutes a basis for combinatorial chemistry, which has quickly received attention in recent years as means for synthesizing lead compounds for medicaments. Solid-phase synthesis is advantageous in that a reaction treatment and a column chromatography operation that are required for every reaction process in a common solution reaction are replaced with a washing operation, so as to simplify the reaction process, and as a result, reduction in operation time can be achieved.

An example of a solid-phase that provides a site for carrying out the reaction in solid-phase synthesis is a resin that is an inorganic or organic polymer. An actual reaction site is an internal pore thereof (Figure 3). Since such pores have a wide surface area, they can be used as reaction sites. Molecules in such pores move due to diffusion. However, there has been no such recognition in the conventional solid-phase synthesis, and thus the solid-phase synthesis reaction has been carried out by swelling a resin with an excess amount of solvent, as continuation of a solution reaction, followed by stirring, shaking, or solution sending (Figure 4; "conventional method" ). For example, one reactant is retained on a solid-phase, and other reactants are given in the form of a solution (liquid-phase). Since the amount of the solvent in the liquid-phase is excessively higher than the amount necessary for swelling the resin under such conditions, the reactant should be moved via diffusion from such an excess amount of liquid-phase to the inside of the resin that is a reaction site (solid-phase) for the reactant, that is, the inside of pores. Moreover, the reactant given in the form of a liquid-phase is uniform, and the movement of molecules in pores is conducted via diffusion. Thus, external physical force such as stirring is not effective. It has been considered that these matters decrease the reaction efficiency of a solid-phase reaction in the conventional methods.

Hence, the present inventors have conducted intensive studies regarding the aforementioned two points: that since the actual reaction site for a solid-phase reaction is a pore, it is considered that the reaction is controlled by diffusion; and that since the movement of a reactant from a liquid-phase into a pore is also controlled by diffusion, it is considered that an excess amount of liquid-phase rather decreases the reaction efficiency. As a result, the inventors have confirmed that the efficiency of the conventional solid-phase reaction is significantly improved by the improvement of the aforementioned two points. That is, such two improvements are (a) a reaction due to diffusive control (a solid-phase reaction via diffusive mixing) and (b) a reaction using the minimum necessary amount of a solvent. The effects obtained by the improvement (a) result in miniaturization of a synthesizing robot caused by a decrease in the liquid amount or elimination of a stirring or shaking device, and the effects obtained by the improvement (b) result in a reduction in the reaction time due to concentration of a reactant contained in a liquid-phase. In addition, these improvements simultaneously achieve energy conservation, reduction in waste liquid, and the improvement of living environment (research environment).

As a result of such improvements and studies, the present inventors have established the solid-phase reaction method of the present invention for carrying out a solid-phase reaction in a pore of a solid material with a size necessary for capillary phenomenon, which is characterized in that the reaction is carried out in a state where an excess amount of liquid-phase does not exist on the outer surface of the solid material. The above method is also characterized in that it does not need external force such as stirring or shaking, and that the reaction is carried out only via diffusive mixing.

The solid-phase reaction of the present invention involving diffusive mixing is the principle of a solid-phase reaction, and it is not limited to reactions associated with sugars, but it can also be applied to all types of reactions performed on solid-phases. The aforementioned solid-phase reaction method involving diffusive mixing can be preferably

applied to chemical reactions or biochemical reactions. Specific examples of such reactions may include a sugar chain synthesis reaction, a peptide synthesis reaction, a nucleic acid synthesis reaction, a reaction of synthesizing analogs or complexes thereof, an organic chemical synthesis reaction, and an enzyme reaction. This method is applied more preferably to a sugar chain synthesis reaction, and particularly preferably to the solid-phase synthesis reaction of sugar chain(s) of the present invention described in (1) above.

Specifically, in the case of a solid-phase synthesis reaction of sugar chain(s) for example, a reaction of allowing a second monosaccharide to react with and bind to a first monosaccharide binding to a solid-phase is carried out by adding a liquid-phase containing the second monosaccharide to the solid-phase to which the first monosaccharide binds, and then allowing the above liquid-phase to react with the above solid-phase. In the case of other types of chemical reactions or biochemical reactions also, a first substance associated with the reaction is previously allowed to bind to a solid-phase, and a liquid-phase containing a second substance to be allowed to react with the first substance is then added to the above solid-phase, so as to carry out the reaction.

Any type of solid material can be used as a solid-phase in the present invention, as long as it has a pore with a size necessary for capillary phenomenon and the aforementioned solid-phase reaction can be carried out in the pore. In the case of carrying out a method of solid-phase synthesis of sugar chain(s) for example, a solid material preferably has a certain functional group on the surface thereof, and further, a linker capable of binding to a substrate associated with the reaction binds to the functional group. As such a functional group or a linker, those described in detail regarding the method of solid-phase synthesis of sugar chain(s) described in (1) above can be used. With regard to the material of a solid as well, those described in detail regarding the method of solid-phase synthesis of sugar chain(s) described in (1) above can be used. Specific examples may include a resin, a silica, and a glass (porous glass). Examples of a resin may include polystyrene, TG (TengaGel (manufactured by Novabiochem)), and PEGA (Acrylamide-PEG Co-polymer (manufactured by Novabiochem)). These materials are size and shape-controlled so that they have suitable pores therein, and the thus size and shape-controlled products can be preferably used. The size of a pore existing in these products is not particularly limited, as long as it is a size enough to generate capillary phenomenon. Such a size may be suitably selected depending on the reaction to be applied, the solvent used, or the like.

The size and shape of a solid material may be suitably selected depending on the reactor or device used. Resins or silicas that are size and shape-controlled into various shapes are commercially available. Resin beads that are size and shape-controlled into the form of beads (particles) are preferably used in terms of handlability. In the case of resin beads for example, the grain diameter thereof is between approximately 1 and 1,000 μm. In addition, those with an indefinite shape can also be used. Resin beads having various grain diameters and pore sizes are commercially available. Those suitable for the reaction of interest may be selected from among such commercially available products and may be used. An example is given in Table 1 shown later.

Moreover, a solid material having a monolith structure can also be preferably used. The term "monolith" is used to mean a continuous structure or a monolithic rock-like product. Thus, this term means a continuum composed of a single material having a certain texture. For example, in the case of "monolithic silica" composed of silica, a silica skeleton and a void have a continuous structure in a single article. With such a structure, when compared with a product filled with spherical particles for example, monolithic silica has a higher porosity, and it also has many perforations because of its continuous structure. Thus, monolithic silica is preferable for the method of the present invention. It is also possible to form a solid material having a monolithic structure even from other materials such as polystyrene monolith. Such materials can also be used in the present invention by selecting as appropriate those with a form or size suitable for a reactor or device used.

Furthermore, an article having artificially formed pores can also be used in the present invention. For example, an article comprising a channel or the like that is artificially formed to provide the same effects as those of the aforementioned pore, can also be used. More specifically, a micro flow channel structure (hereinafter referred to as a "microchannel" at times) or a chip equipped with such a microchannel can be used, for example.

The term "microchannel" is used herein to mean a channel having a sectional form whereby micro effects appear, namely, a certain change appears in the behavior of a liquid, when the liquid is introduced into the channel (flow channel). In the present invention, a microchannel causing at least capillary phenomenon among such micro effects is used. Such appearance of micro effects differs depending on the physical properties of a solvent used as a liquid-phase. With regard to the sectional shape, that is, the shape of a face that is vertical to the main flow direction of the channel, the length of the shortest interval (which corresponds to a short side in the case of a rectangle, and a minor axis in the case of an ellipse) is generally 5 mm or shorter, preferably 500 μm or shorter, and more preferably 200 μm or shorter. The lower limit of this length is not particularly limited, as long as it is a length having functions as a microchannel. In addition, the term "chip" is used to mean a member that is a portion containing a flow channel structure. Moreover, the term "microchip" is used to mean a chip having a micro flow channel structure consisting of the aforementioned microchannel. The size and shape of such a chip is not particularly limited, and they are determined as appropriate depending on purpose. Such a chip can be produced by commonly used known means.

In all the cases of the aforementioned shapes, a solid material having a large void due to pores existing therein,

namely, a solid material having a high porosity, is preferable as a solid material used in the present invention. A solid material having a small pore size and a high porosity is particularly preferable. This is because since a reaction is carried out on the surface of a pore of a solid material in the method of the present invention, if a pore size is constant, a solid material having a high porosity and a large surface area brings on high efficiency in the reaction.

In the present invention, the expression "the reaction is carried out in a state where an excess amount of liquid-phase does not exist on the outer surface of the solid material" does not necessarily mean that no liquid-phase exists, but means that since the reaction efficiency decreases if an excess amount of liquid-phase is used, such an excess amount of liquid-phase is unnecessary. That is, if the reaction efficiency of interest can be sufficiently achieved, an excess amount of liquid-phase may exist to a certain extent. Conditions under which the reaction is efficiently carried out only via diffusive mixing without using external force such as stirring, shaking, or continuous solution sending, are all included.

As a liquid-phase, a liquid amount that at least fills pores to such an extent that diffusion can sufficiently occur in the pores is necessary. That is, if the amount of such a liquid is too small or too large, the reaction efficiency decreases, and it results in a waste of solvent. Thus, it is adequate that a minimum necessary amount of liquid-phase be used. Theoretically, it is most efficient to carry out the reaction with the maximum liquid amount that fills up pores of a solid material.

In the case of a solid material having properties whereby it does not swell even if a liquid-phase is added, such as silica beads or monolithic silica, such a maximum liquid amount that fills up pores of a solid material is theoretically the same as the amount of voids of pores existing in the solid material (hereinafter referred to as a "void amount" at times). Such a void amount can be obtained by a commonly used known measurement method. On the other hand, in the case of a solid material having properties whereby it swells by addition of a liquid-phase, such as resin beads, a necessary liquid amount (hereinafter referred to as a "swollen liquid amount" ) can be easily determined by the means mentioned below, for example. In the case of the former solid material with properties whereby it does not swell also, since the void amount determined by measurement or calculation is not necessarily identical to the liquid amount that is actually required in some cases, it is preferable to confirm the optimum liquid amount by the same above method.

Such a liquid amount necessary for swelling a resin can be determined with reference to Table 1 shown below or Bayer E. Angew. Chem. Int. Ed. Engl. (1991) 30, 113. It is easily confirmed in an experiment as follows.

First, a certain amount of resin is weighed, and a solvent used is then added dropwise to the resin. This time, the amount of a solvent added is measured. The swelling of a resin is generally completed for approximately 10 minutes. Thus, using this period of time as a guideline, the solvent is added dropwise. When more than a necessary amount of solvent has been added, the friction among beads decreases, and a "liquefaction" phenomenon takes place. The amount of a solvent obtained at this time may be determined as a solvent amount used for the reaction. This time, if resin beads are weighed using a suitable experimental container such as a cylinder, and addition of a solvent is carried out in this container, the resin beads swell as the solvent is added dropwise. However, when the amount exceeds the swollen liquid amount, it results in a state where an unnecessary excess amount of solvent is just laminated on the layer of the resin beads. Thus, the swollen liquid amount (that is a point at which pores are filled with solvent and do not swell any more) can be easily confirmed in an experiment.

Table 1

| Resin | Swelling ml/g | | | | | |
|---|---|---|---|---|---|---|
| | DMF | THF | MeOH | Tol. | $H_2O$ | $CH_2Cl_2$ |
| Polystyrene (200-400 mesh) | 3 | 8 | 2 | 7 | - | 7 |
| Polystyrene (100-200 mesh) | 3 | 8 | 2 | 7 | - | 7 |
| TG (90 μ m) | 5 | 6 | 4 | 5 | 4 | 5 |
| TG(130 μ m) | 5 | 6 | 4 | 5 | 4 | 5 |
| PEGA | 11 | 13 | 13 | 12 | 16 | 13 |

As a result of the experiment carried out by the present inventors, it was found that when a reactant (which is specifically a monosaccharide unit, an activator, or the like, associated with the reaction in the case of a sugar chain synthesis method, for example) necessary for the swollen liquid amount of a solid phase such as a resin is dissolved therein, if the amount of a reactant is the same, it becomes unnecessary for the reactant to move via diffusion between the inside of a pore of the resin as a reaction site and an excess amount of liquid-phase. As a result, reduction in the reaction time could be achieved. In addition, in the case of using multiple reactants, all the reactants may be added simultaneously, or the reactants may be added sequentially in several times and may be then mixed in a pore.

Any type of solvent can be used as a liquid-phase, as long as it is suitable for each solid-phase reaction. A solvent, which is not involved in the reaction of interest and does not react with a solid-phase as a resin, can be used. Also, a solvent that is likely to cause capillary phenomenon with the combination of a solid-phase, is preferably selected.

Using the thus selected solid-phase and liquid-phase, the solid-phase reaction method of the present invention can be carried out.

The solid-phase reaction itself can be carried out by commonly used known means. In the method of the present invention, the reaction is carried out without using external force such as stirring, shaking, or continuous solution sending, and preferably by leaving it at rest. As a result of leaving the reaction system at rest, the reaction can be carried out via diffusive mixing. The device preferably has a temperature-controlling function, a solution-sending function, or the like.

Any type of reactor (reaction vessel) can be used, as long as it does not prevent the diffusive mixing and the reaction but is able to maintain a state that is sufficient for promoting the reaction. A reactor suitable for the reaction of interest, the solid-phase used, the liquid-phase used, or the like, may be selected. A reactor capable of opening and closing at a moderate degree is preferable for the purpose of preventing the liquid-phase from being dried.

In the case of using a beads-like (granular) solid material as a solid-phase, a reaction vessel has a structure whereby it is filled with such beads at a suitable density. Preferably, a reaction vessel has a structure capable of adding and recovering a liquid-phase is used. Specifically, a reaction vessel having a structure similar to those of columns generally used for purification or separation of substances is used (for example, "the present invention" in Figure 4). Moreover, in the case of using a solid material having a monolithic structure also, such a solid material is preferably accommodated in a reaction vessel suitable for the size or shape thereof.

On the other hand, in the case of using an article having artificially formed pores, such as a chip having a micro flow channel structure (microchannel), such a chip itself can be used as a reaction vessel. A structure whereby a liquid-phase is supplied to a channel equipped in the chip as a result of capillary phenomenon, and whereby the liquid-phase can be recovered after completion of the reaction, can be adopted. Moreover, the aforementioned beads-like or monolithic-structured solid material may be filled in the channel of a chip with such a structure, or the internal wall of the channel may also be coated therewith.

The method of solid-phase synthesis of sugar chain(s) described in (1) above is a method for carrying out a solid-phase synthesis of sugar chain(s) at high efficiency by controlling the temperature rising rate, thereby changing the temperature in a reaction system. Thus, by combining the above method with a method capable of efficiently carrying out a solid-phase reaction with a small amount of solvent, such as the present method, the synthesis can be carried out more efficiently. In particular, in a case where a sugar chain library is intended to be constructed by combinatorial chemistry as described above, for example, it is necessary to carry out the reaction with a large number of combinations even at a small amount, thereby synthesizing many types of sugar chains. For such a purpose, it is extremely preferable to combine the method of solid-phase synthesis of sugar chain(s) described in (1) above that is capable of simultaneously promoting a large number of reactions at one time with this solid-phase reaction method capable of carrying out the reaction only by leaving it at rest with a small amount of liquid-phase (which does not need a device associated with stirring, shaking, continuous solution sending, or the like).

Hereafter, a case where the present method is used with the combination of the method of solid-phase synthesis of sugar chain(s) of the present invention described in detail in (1) above, will be more specifically explained.

First, a resin (P-L-O-$A^1$-X) to which a monosaccharide unit (donor) binds is prepared. Subsequently, a method comprising allowing this resin to swell with a solution containing a monosaccharide unit (receptor; HO-$A^2$-Y) allowed to bind to the resin and then adding an activator (promoter) of a leaving group, so as to carry out the reaction (method 1), or a method comprising allowing this resin to swell with a mixed solution consisting of a monosaccharide unit (HO-$A^2$-Y) allowed to bind to the resin (P-L-O-$A^1$-X) and an activator of a leaving group, so as to carry out the reaction (method 2), is repeated, thereby synthesizing a sugar chain having any given length on the resin. As such method 1 or method 2, any type of method can be selected depending on the proceeding of the reaction.

The thus obtained resin to which a sugar chain binds is washed with a suitable solvent, as appropriate, as described above, and the sugar chain is then cleaved out of the resin, so as to obtain a sugar chain of interest.

In the case of the solid-phase reaction method of the present invention involving diffusive mixing, there is no need to conduct physical operations for mixing, such as stirring or shaking, in the solid-phase reaction. That is to say, the solid-phase reaction can be achieved by diffusion of molecules in resin pores. Thereby, miniaturization of a reaction device can be achieved. The conventional synthesizer is composed of a reaction vessel, a temperature-controlling device, a dispenser, a stirrer/mixer, and a gas-substituting device. In contrast, as a reactor for carrying out the method of the present invention, (i) a device composed of a reaction vessel, a temperature-controlling device, a dispenser, and a gas-substituting device, or (ii) a device composed of a reaction vessel, a temperature-controlling device, a flow channel converter, and a solution-sending pump, can be used.

That is to say, the present invention provides a reaction device for carrying out a solid-phase reaction via diffusive mixing, which comprises at least the following (1) and (2):

(1) a reaction unit for carrying out a solid-phase reaction, which has an introduction unit for injecting or discharging a liquid-phase and a space for accommodating a solid material; and
(2) a temperature-controlling unit for controlling the temperature of the reaction unit.

(4) Library obtained by the method of solid-phase synthesis of sugar chain(s) of the present invention

A library composed of sugar chains consisting of all types of combinations of 3 types of sugars selected from among monosaccharides existing in a living body, which have been synthesized by the method of solid-phase synthesis of sugar chain(s) of the present invention described in (1) above, is also included in the scope of the present invention. Preferred examples of monosaccharides existing in a living body that are used herein may include mannose, glucose, galactose, xylitose, glucosamine, galactosamine, glucuronic acid, fructose, and sialic acid.
It is considered that the importance of a sugar chain library increases in the future. The desired target is a trisaccharide library consisting of combinations obtained by arbitrarily selecting 3 types of monosaccharides from 9 types of monosaccharides existing in a living body. The thus obtained sugar chain library can be directly used as a probe for studies of functions, or can be used as a medicament lead. Otherwise, it can also be used as a library that imitates sugar chains in a living body to screen for a medicament.
A sugar chain in a living body generally consists of 1 to 5 connected monosaccharides. It has been known that such a sugar chain is associated with complicated recognition phenomena such as intercellular recognition in an immune reaction or the like, or recognition of viruses or bacteria. The library of the present invention that is composed of sugar chains consisting of all types of combinations of 3 types of sugars selected from among monosaccharides existing in a living body is useful as a library that averagely imitates sugar chains actually existing in a living human body. The number of combinations of trisaccharides consisting of 9 types of monosaccharides existing in a living body is 140,824. However, the range covered by the library can also be limited.
In the present invention, based on the orthogonal glycosylation method as described above in the present specification, a sugar chain is synthesized according to the diffusive mixing solid-phase reaction method as described above in the present specification, using certain monosaccharide units as described above in the present specification, so as to obtain a mixture consisting of stereoisomers ($2^3$=8) at position of acetal (anomeric). The mixture can be then separated by HPLC, as necessary (Figure 5).
The present invention will be more specifically described in the following examples. However, the present invention is not limited by the examples. As common methods and reagents regarding chemical reactions and sugar chain synthesis reactions used in the following examples, those described in general experimental protocols such as *"Koso Gosei Handbook* (Solid-Phase Synthesis Handbook), published by Novabiochem (Merck)" can be used.

EXAMPLES

Merk Art 5715 Silicagel 60 F254 was used for TLC, and detection was carried out using a UV absorption and coloring reagent (10%$H_2SO_4$-ethanol). Merk Art 7734 Silica gel 60 70-230 mesh was used for column chromatography. $^1$H and $^{13}$C-NMR were measured using BRUKER AVANCE 500 or JEOL EX-270. The chemical shift value was indicated as a $\delta$ value (ppm) to an internal standard substance (tetramethylsilane). MALDI-TOFMS was measured using Perseptive Voyger™, and 2,5-dihydrobenzoic acid was used as a matrix. HORIBA FT720 was used for FT-IR. As a resin, Novasyn$^R$ TG amino resin HL (0.29mmo1/g loading) was used. Aldrich 1,2-dichloroethane anhydrous (99.8%) was used as $C_2H_4Cl_2$ for the reaction. EtCN was distilled away under a reduced pressure with $CaH_2$. For a solid-phase reaction, Bohdan$^R$ Miniblock™ was used.

Example 1: Protected monosaccharide library

In the monosaccharide library shown in Figure 1, fourteen types of monosaccharides including Man-2-OH, Man-3-OH, Man-4-OH, Man-6-OH, Gal-2-OH, Gal-3-OH, Gal-4-OH, Gal-6-OH, Glc-2-OH, Glc-4-OH, Glc-6-OH, GlcN-4-OH, GlcN-6-OH, and Sia, are known compounds, as described above in the present specification.
With regard to the synthesis of Man-2-OH, Man-3-OH, Man-4-OH, and Man-6-OH, the following publications can be referred to: Kametani T.; Kawamura K.; Honda T. (1987) J. Am. Chem. Soc. 109, 3010-17; Lemanski G.; Ziegler, T. (2000) Tetrahedron 56, 563-579; Lemanski G; Ziegler T. (2000) Helvetica Chim. Acta 83, 2655-2675; Oshitari T.; Shibasaki M.; Yoshizawa T.; Tomita M.; Takao K.; Kobayashi S. (1997) Tetrahedron 53, 10993-11006; Marzabadi C. H.; Spilling C. D. (1993) J. Org. Chem. 58, 3761-6; Street I. P.; Withers S. G. (1986) Can. J. Chem. 64, 1400-3; Muragata T.; K., Masami; S., Y; S., H.; Suzuki S.; Ogawa T Jpn. Kokai Tokkyo Koho (1994), 44 pp. CODEN: JKXXAF JP 06293790 A2 19941021 Heisei. Patent written in Japanese. Application: JP 93-81955 19930408. CAN 123:83943 AN 1995:662332.
With regard to the synthesis of Sia, the following publications can be referred to: Dasgupta F. (Glycomed, Inc., USA). U.S. (1992), 9 pp. CODEN: USXXAM US 5138044 A 19920811 Patent written in English. Application: US

90-566682 19900813. CAN 118:81335 AN 1993:81335 CAPLUS (Copyright 2003 ACS); Marra A.; Sinay P. (1989) *Carbohydr. Res.,* 187 35-42; Sharma N. M.; Eby R. (1984) *Carbohydr. Res.,* 127 201-210.

Monosaccharides other than the aforementioned monosaccharides were synthesized as follows.

(1) Synthesis of fluoride of galactose derivative phenyl 2-*O*-chloroacetyl-3,4,6-tri-*O*-benzyl-1-thio-β-D-galactopyranoside

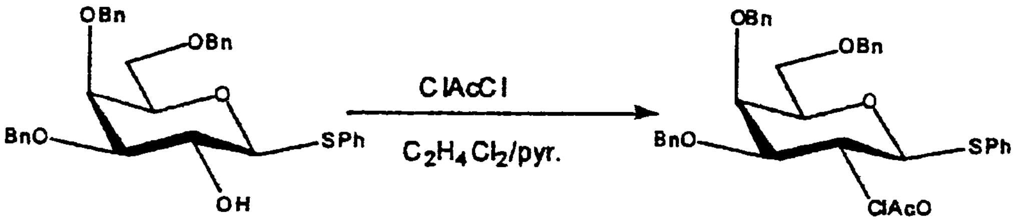


28 38

Compound 28 (300 mg, 0.55 mmol) was dissolved in $CH_2Cl_2$ (5.4 mL) and pyridine (0.3 mL), and thereafter, chloroacetyl chloride (88 μL, 1.11 mmol) was added to the solution under cooling on ice. The obtained mixture was stirred at room temperature for 4 hours. After completion of the reaction, the reaction solution was extracted with $CH_2Cl_2$, washed with HCl, and then dehydrated with $MgSO_4$, followed by vacuum concentration. The resultant was purified by silica gel column chromatography (toluene : ethyl acetate = 15 : 1), so as to obtain compound 38 (323 mg, 0.52 mmol) at a yield of 94%. TLC toluene : ethyl acetate=6 : 1 *Rf*=0.62. $^1$H-NNM ($CDCl_3$):δ7.50-7.22 (20H, m, Ph x 4), 5.45 (1H, t, *J*=9.5Hz, H-2), 4.93, 4.68, 4.63, 4.56, 4.50, 4.43 (6H, each d, *J*= 12.0Hz, benzyl methylene x 3), 4.68 (1H, d, H-1), 4.00 (1H, t, *J*=2.8Hz, H-4), 3.97 (1H, d, -$CH_2$Cl), 3.90 (1H, d, *J*=15.0Hz, -$CH_2$Cl), 3.66-3.63 (3H, m, H-3, H-5, H-6a), 3.58 (1H, dd, *J*=9.5Hz, *J*=3.0Hz, H-6b).

2-*O*-chloroacetyl-3,4,6-tri-*O*-benzyl-β-D-galactopyranosyl fluoride (39)

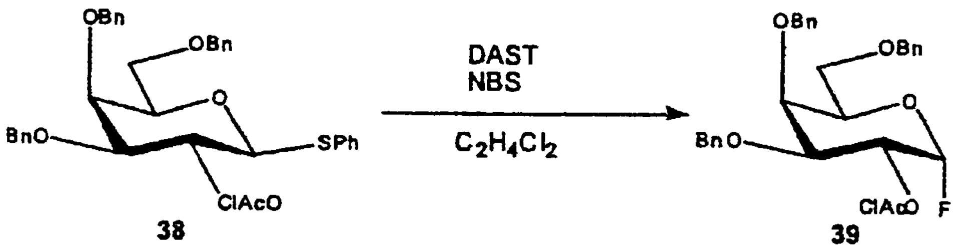


38 39

Compound 38 (308 mg, 0.50 mmol) was dissolved in $CH_2Cl_2$ (4.0 mL), and thereafter, DAST (79 μL, 0.60 mmol) and NBS (107 mg, 0.60 mmol) were added to the solution under nitrogen stream at -15°C. The obtained mixture was stirred for 1 hour. After completion of the reaction, the reaction solution was extracted with $CH_2Cl_2$, washed with $NaHCO_3$, and dehydrated with $MgSO_4$, followed by vacuum concentration. The resultant was purified by silica gel column chromatography (toluene : ethyl acetate = 20 : 1), so as to obtain compound 39 (180 mg, 0.34 mmol) at a yield of 68%. TLC toluene : ethyl acetate=6 : 1 *Rf*=0.60. $^1$H-NMR ($CDCl_3$):δ.68-7.26 (15H, m, Ph x 3), 5.80 (1H, dd, $J_{1,2}$=2.8Hz, $J_{1,F}$=54.6Hz, H-1), 5.41 (1H, dd, *J*=10.5Hz, *J*=25.0Hz, H-2), 4.93, 4.72, 4.63, 4.57, 4.50, 4.44 (6H, each d, *J*=11.0Hz, benzyl methylene x 3), 4.13 (1H, t, *J*=6.5Hz), 4.08 (1H, d, H-4), 4.05-4.01 (2H, m, -$CH_2$Cl), 3.96 (1H, dd, *J*=2.5Hz, *J*=10.5Hz, H-3), 3.60-3.59 (2H, m, H-6a, H-6b).

3,4,6-tri-*O*-benzyl-β-D-galactopyranosyl fluoride (40)

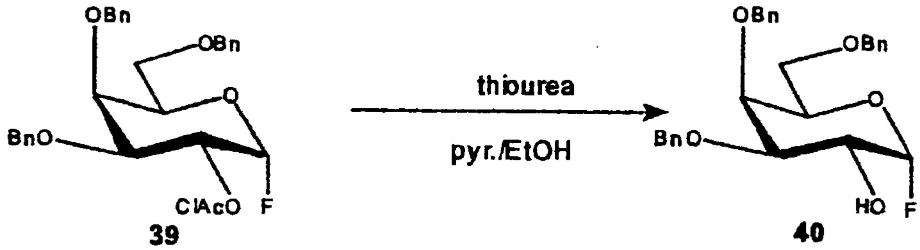


39

40

Compound 39 (146 mg, 0.28 mmol) was dissolved in pyridine (6.0 mL) and ethanol (1.0 mL), and thereafter, thiourea (21 mg, 0.28 mmol) was added to the solution. The obtained mixture was stirred at 80°C for 1 hour. After completion of the reaction, the reaction solution was extracted with $CH_2Cl_2$, washed with HCl and $NaHCO_3$, and dehydrated with $MgSO_4$, followed by vacuum concentration. The resultant was purified by silica gel column chromatography (toluene : ethyl acetate=10 : 1), so as to obtain compound 40 (94 mg, 0.21 mmol) at a yield of 75%. TLC toluene : ethyl acetate=6 : 1 *Rf*=0.31. $^1$H-NMR ($CDCl_3$):δ7.40-7.22 (15H, m, Ph x 3), 5.65 (1H, dd, $J_{1,2}$=2.8Hz, $J_{1,F}$=54.7Hz, H-1), 4.87, 4.73, 4.61, 4.56, 4.51, 4.44 (6H, each d, *J*=11.8Hz, benzyl methylene × 3), 4.20 (1H, dd, *J*=10.1Hz, *J*=25.4Hz, H-2), 4.12 (1H, t, *J*=6.8Hz, H-5), 4.06 (1H, d,*J*=1.6Hz, H-4), 3.75 (1H, dd, *J*=2.6Hz, *J*=10.2Hz, H-3), 3.65-3.57 (2H, m, H-6a, H-6b). $^{13}$C-NMR ($CDCl_3$) :δ108.3, 106.2, 7.6, 74.7, 73.5, 73.1, 72.2, 71.9, 68.3, 60.7

phenyl 3-*O*-chloroacetyl-2,4,6-tri-*O*-benzyl-1-thio-β-D-galactopyranoside (41)

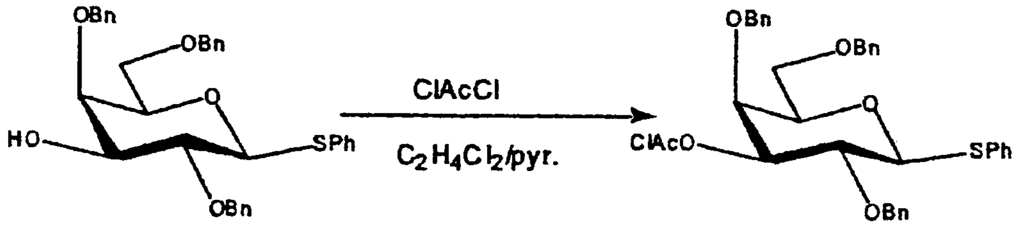


32

41

Compound 32 (140 mg, 0.25 mmol) was dissolved in $CH_2Cl_2$ (2.7 mL) and pyridine (0.15 mL), and thereafter, chloroacetyl chloride (41 μL, 0.51 mmol) was added to the solution under cooling on ice. The obtained mixture was stirred at room temperature for 1.5 hours. After completion of the reaction, the reaction solution was extracted with $CH_2Cl_2$, washed with HCl, and then dehydrated with $MgSO_4$, followed by vacuum concentration. The resultant was purified by silica gel column chromatography (toluene : ethyl acetate = 20 : 1), so as to obtain compound 41 (145 mg, 0.23 mmol) at a yield of 91%. TLC toluene : ethyl acetate=6 : 1 *Rf*=0.70. $^1$H-NMR ($CDCl_3$):δ7.58-7.15 (20H, m, Ph x 4), 4.98 (1H, dd, *J*=3.0Hz, *J*=9.7Hz, H-3), 4.83, 4.68, 4.57, 4.53, 4.52, 4.45 (6H, each d, *J*=11.1Hz, benzyl methylene × 3), 4.59 (1H, d, *J*=7.5Hz, H-1), 4.05 (1H, d, *J*=2.9Hz, H-4), 3.95 (1H, t, *J*=9.6Hz, H-2), 3.74 (1H, t, *J*=6.6Hz, H-6a), 3.66 (1H, d, *J*=11.8Hz, $-CH_2Cl$), 3.58 (1H, d, *J*=11.8Hz, $-CH_2Cl$), 3.63-3.54 (H-4, H-5, H-6b).

3-*O*-chloroacetyl-2,4,6-tri-*O*-benzyl-β-D-galactopyranosyl fluoride(42)

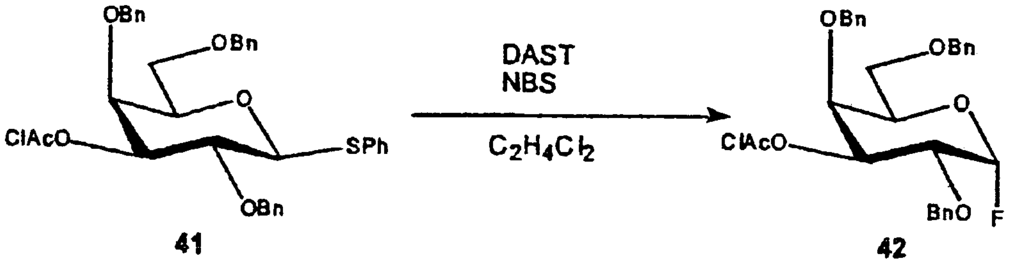


41 42

Compound 41 (132 mg, 0.21 mmol) was dissolved in $CH_2Cl_2$ (2.5 mL), and thereafter, DAST (34 μL, 0.25 mmol) and NBS (46 mg, 0.25 mmol) were added to the solution under nitrogen stream at -15°C. Thereafter, the obtained mixture was stirred for 1 hour. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 20 : 1), so as to obtain compound 42 (110 mg, 0.21 mmol) at a yield of 98%. TLC toluene : ethyl acetate=6 : 1 *Rf=0.68.* $^1$H-NMR ($CDCl_3$):δ7.40-7.17 (15H, m, Ph x 3), 5.60 (1H, dd, *J=2.7Hz, J=53.2Hz,* H-1 b), 5.28 (1H, dd, *J=4.7Hz, J=10.8Hz,* H-3), 5.05 (1H, dd, *J=6.9Hz,* H-1b) 4.83, 4.69, 4.66, 4.58, 4.53, 4.46 (6H, each d, *J=11.*7Hz, benzyl methylene x 3), 4.22 (1H, t, *J*=6.8Hz, H-5), 4.14 (1H, d, *J*=2.5Hz, H-4), 4.04 (1H, dd, *J=2.8Hz, J*=10.3Hz, H-3), 4.01 (1H, t, *J=2.5Hz,* H-2), 3.94-3.89 (2H, m, H-6a, H-6b), 3.81 (1H, d, *J*= 14.8Hz, $CH_2Cl$), 3.74 (1H, d, *J=14.8Hz,* $CH_2Cl$).

2,4,6-tri-O-benzyl-β-D-galactopyranosyl fluoride (43)

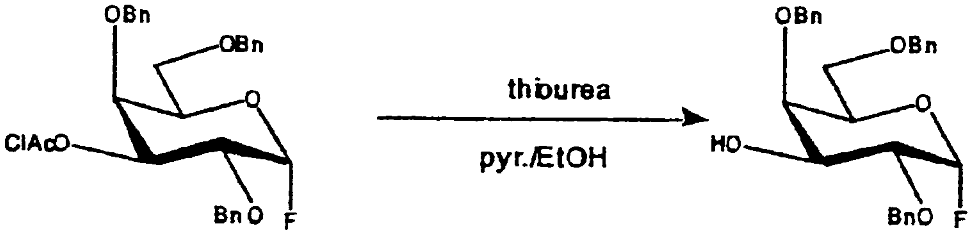


42 43

Method 1

Compound 42 (101 mg, 0.19 mmol) was dissolved in pyridine (6.0 mL) and ethanol (1.0 mL), and thereafter, thiourea (16 mg, 0.21 mmol) was added thereto. The obtained mixture was stirred at 80°C for 1 hour. After completion of the reaction, the reaction solution was extracted with $CH_2Cl_2$, washed with HCl and $NaHCO_3$, and then dehydrated with $MgSO_4$, followed by vacuum concentration. The resultant was purified by silica gel column chromatography (toluene : ethyl acetate = 12:1), so as to obtain compound 43 (67 mg, 0.15 mmol) at a yield of 77%.

Method 2

Compound 32 (191 mg, 0.35 mmol) was dissolved in $CH_2Cl_2$ (2.5 mL), and thereafter, DAST (233 μL, 1.72 mmol) and NBS (69 mg, 0.39 mmol) were added to the solution under nitrogen stream at -15°C. The obtained mixture was stirred for 1 hour. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 12:1), so as to obtain compound 43 (60 mg, 0.13 mol) at a yield of 38%. TLC toluene : ethyl acetate=6:1 *Rf*=0.40. $^1$H-NMR ($CDCl_3$):δ7.35-7.16 (15H, m, Ph x 3), 5.60 (1H, dd, $J_{1,2}$=2.5Hz, $J_{1,F}$=53.7Hz, H-1), 4.81, 4.73, 4.68, 4.62, 4.52, 4.43 (6H, each d, *J*=11.4Hz, benzyl methylene x 3), 4.15 (1H, t, *J*=6.8Hz, H-5), 4.07 (1H, dd, *J*=3.2Hz, *J*=9.6Hz, H-3), 3.99 (1H, d, *J*=2.6Hz, H-2), 3.80 (1H, dd, *J*=9.9Hz, *J*=25.2Hz, H-2), 3.61-3.58 (1H,. m, H-6a, H-6b). $^{13}$C-NMR ($CDCl_3$) :δ108.1, 104.3, 76.5, 75.9, 75.2, 73.4, 73.0, 71.6, 69.8, 68.2 phenyl 4-*O*-chloroacetyl-2,3,6-tri-*O*-benzyl-1-thio-β-D-galactopyranoside (44)

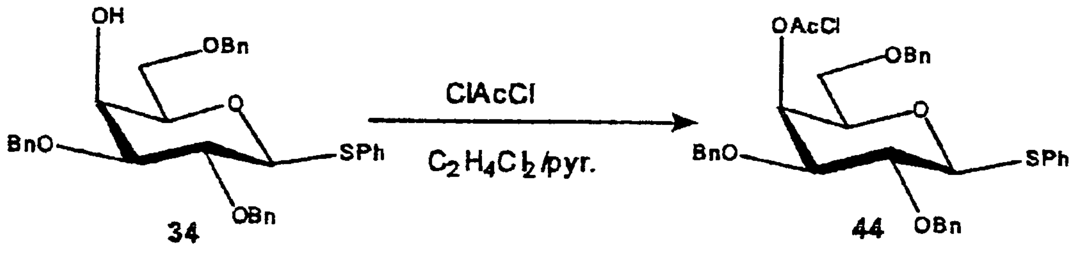


Compound 34 (150 mg, 0.28 mmol) was dissolved in $CH_2Cl_2$ (2.7 mL) and pyridine (0.15 mL), and thereafter, chloroacetyl chloride (44 μL, 0.55 mmol) was added to the solution under cooling on ice. The obtained mixture was stirred at room temperature for 2 hours. After completion of the reaction, the reaction solution was extracted with $CH_2Cl_2$, washed with HCl, and then dehydrated with $MgSO_4$, followed by vacuum concentration. The resultant was purified by silica gel column chromatography (toluene : ethyl acetate = 20 : 1), so as to obtain compound 44 (160 mg, 0.26 mmol) at a yield of 94%. TLC toluene : ethyl acetate=6 : 1 *Rf*=0.70. $^1$H-NMR ($CDCl_3$):δ7.61-7.13 (20H, m, Ph x 4), 5.70 (1H, d, *J*=3.0Hz, H-4), 4.76, 4.75, 4.72, 4.64, 4.53, 4.52 (6H, each d, benzyl methylene x 3), 4.60 (1H, *J*=7.8Hz, H-1), 4.04 (1H, d, *J*= 14.9Hz, -CH2Cl), 3.98 (1h, d, *J*=14.9Hz, -$CH_2$Cl), 3.78 (1H, t, *J*=6.8Hz, H-5), 3.66 (1H, dd, *J*=3.2Hz, *J*=9.1Hz, H-3), 3.63-3.58 (2H, m, H-6a, H-6b), 3.51 (1H, t, H-2).

4-*O*-chloroacetyl-2,3,6-tri-*O*-benzyl-β-D-galactopyranosyl fluoride (45)

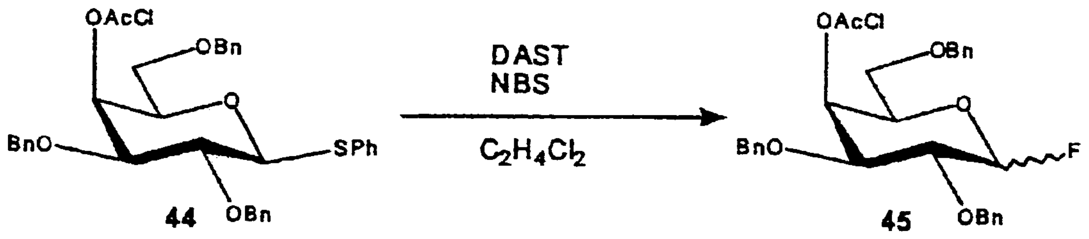


Compound 44 (146 mg, 0.23 mmol) was dissolved in $CH_2Cl_2$ (2.5 mL), and thereafter, DAST (38 μL, 0.28 mmol) and NBS (50 mg, 0.28 mmol) were added to the solution under nitrogen stream at -15°C. The obtained mixture was then stirred for 1 hour. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 20:1), so as to obtain compound 45 (117 mg, 0.22 mmol) at a yield of 94%. TLC toluene : ethyl acetate=6 : 1 *Rf*=0.69. $^1$H-NMR ($CDCl_3$):δ7.51-7.29 (15H, m, Ph x 3), 5.73 (1H, d, *J*=2.4Hz, H-4), 5.60 (1H, dd, $J_{1,2}$=2.6Hz, $J_{1,F}$=53.0Hz, H-1), 4.82, 4.77, 4.67, 4.58, 4.54, 4.30 (6H, each d, *J*=11.8Hz, benzyl methylene x 3), 4.27 (1H, t, *J=6.5Hz,* H-5), 4.03 (1H, d, *J*=15.3Hz, -$CH_2$Cl), 3.99-3.94 (2H, m, H-3, -$CH_2$Cl), 3.71 (1H, dd, *J*=9.7Hz, *J*=25.3Hz, H-2), 3.54 (1H, dd, *J*=6.2Hz, *J=9.0Hz,* H-6a), 3.45 (1H, t, *J*=9.3Hz, H-6b).

2,3,6-tri-*O*-benzyl-β-D-galactopyranosyl fluoride (46)

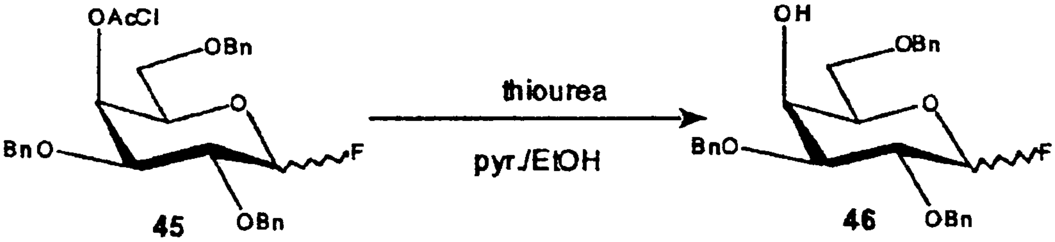


45 46

Method 1

Compound 45 (108 mg, 0.20 mmol) was dissolved in pyridine (6.0 mL) and ethanol (1.0 mL), and thereafter, thiourea (16 mg, 0.21 mmol) was added thereto. The obtained mixture was stirred at 80°C for 1 hour. After completion of the reaction, the reaction solution was extracted with $CH_2Cl_2$, washed with HCl and $NaHCO_3$, and then hydrated with $MgSO_4$, followed by vacuum concentration. The resultant was purified by silica gel column chromatography (toluene : ethyl acetate = 12 : 1), so as to obtain compound 46 (51 mg, 0.11 mmol) at a yield of 55%.

Method 2

Compound 34 (120 mg, 0.22 mmol) was dissolved in $CH_2Cl_2$ (2.5 mL), and thereafter, DAST (146 μL, 1.11 mmol) and NBS (43 mg, 0.24 mmol) were added to the solution under nitrogen stream at -15°C. The obtained mixture was stirred for 1 hour. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 12:1), so as to obtain compound 46 (40 mg, 0.09 mmol) at a yield of 40%. TLC toluene : ethyl acetate=6 : 1 *Rf*=0.47. $^1$H-NMR ($CDCl_3$):7.37-7.17(15H, m, Phx3), 5.40 (1H, dd, $J_{1,2}$=2.6Hz, $J_{1,F}$=53.0Hz, H-1α), 5.15 (1H, dd, *J=7.2Hz, J=52.7Hz,* H-1(β), 4.83, 4.78, 4.74, 4.71, 4.60, 4.55 (6H, each d, *J=11.4Hz,* benzyl methylene x 3), 4.09 (1H, s, H-4), 4.07 (1H, t, *J=5.7Hz,* H-5), 3.91 (1H, dd, *J*=2.5Hz, *J=9.8Hz,* H-3), 3.81-3.64 (3H, m, H-2, H-6a, H-6b). $^{13}$C-NMR ($CDCl_3$) δ108.1, 104.3, 76.5, 75.9, 73.4, 73.0, 71.6, 69.8, 68.2.

Phenyl 6-*O*-chloroacetyl-2,3,4-tri-*O*-benzyl-1-thio-β-D-galactopyranoside (47)

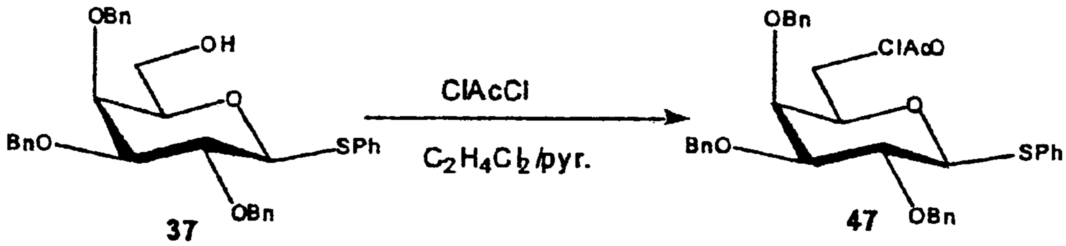


37 47

Compound 37 (150 mg, 0.28 mmol) was dissolved in $CH_2Cl_2$ (2.7 mL) and pyridine (0.15 mL), and thereafter, chloroacetyl chloride (44 μL, 0.55 mmol) was added to the solution under cooling on ice. The obtained mixture was stirred at room temperature for 2 hours. After completion of the reaction, the reaction solution was extracted with $CH_2Cl_2$, washed with HCl, and then dehydrated with $MgSO_4$, followed by vacuum concentration. The resultant was purified by silica gel column chromatography (toluene : ethyl acetate = 15:1), so as to quantitatively obtain compound 47 (171 mg, 0.28 mmol). TLC toluene : ethyl acetate=6:1 *Rf*=0.52. $^1$H-NMR ($CDCl_3$):δ7.56-7.18 (20H, m, Ph x 4), 5.00, 4.83, 4.79, 4.74, 4.63, 4.62 (6H, each d, *J*=11.8Hz, benzyl methylene x 3), 4.74 (1H, d, H-1), 4.35 (1H, dd, *J*=7.1Hz, *J*=11.2Hz, H-6a), 4.13 (1H, dd, *J=5.5Hz, J*=11.3Hz, H-6b), 3.95-3.87 (3H, m, H-3, -$CH_2$Cl), 3.82 (1H, d, *J*=2.4Hz, H-4), 3.61-3.59 (1H, m, H-2, H-5).

6-*O*-chloroacetyl-2,3,4-tri-*O*-benzyl-β-D-galactopyranosyl fluoride (48)

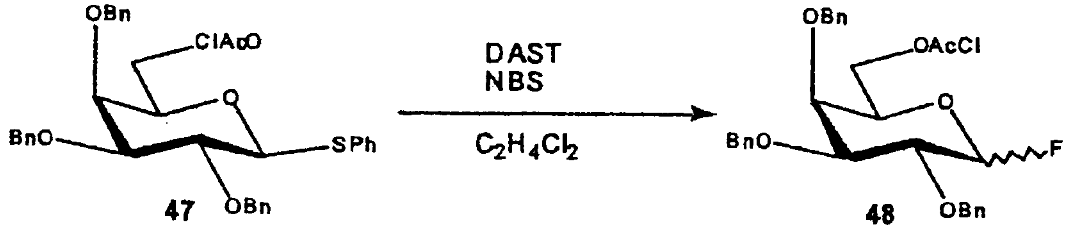


47 48

Compound 47 (171 mg, 0.27 mmol) was dissolved in $CH_2Cl_2$ (2.5 mL), and thereafter, DAST (44 μL, 0.33 mmol) and NBS (59 mg, 0.33 mmol) were added to the solution under nitrogen stream at -15°C. The obtained mixture was stirred for 1 hour. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 20:1), so as to obtain compound 48 (119 mg, 0.2 2mmol) at a yield of 82%. TLC toluene : ethyl acetate=6 : 1 *Rf*=0.50.

2,3,6-Tri-*O*-benzyl-β-D-galactopyranosyl fluoride (49)

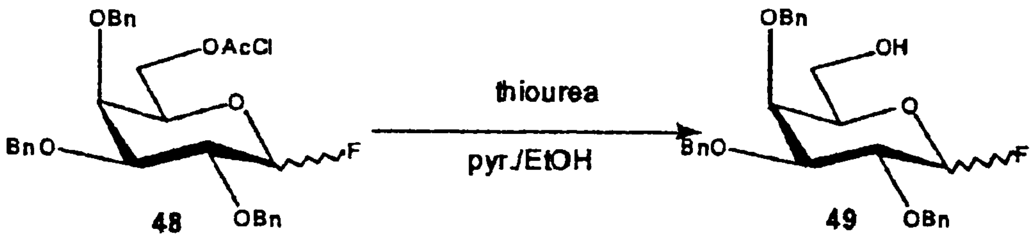


48 49

Compound 48 (119 mg, 0.23 mmol) was dissolved in pyridine (6.0 mL) and ethanol (1.0 mL), and thereafter, thiourea (20.6 mg, 0.27 mmol) was added thereto. The obtained mixture was stirred at 80°C for 1 hour. After completion of the reaction, the reaction solution was extracted with $CH_2Cl_2$, washed with HCl and $NaHCO_3$, and then dehydrated with $MgSO_4$, followed by vacuum concentration. The resultant was purified by silica gel column chromatography (toluene : ethyl acetate = 10:1), so as to obtain compound 49 (78 mg, 0.17 mmol) at a yield of 76%.

Compound 49α: TLC toluene : ethyl acetate=6 : 1 *Rf*= 0.47. $^1$H-NMR ($CDCl_3$):δ7.41-7.15 (15H, m, Ph x 3), 5.60 (1H, dd, *J*=2.3Hz, *J*=53.8Hz, H-1), 4.95, 4.88, 4.83, 4.72, 4.68, 4.64 (6H, each d, *J*=11.6Hz, benzyl methylene x 3), 4.12 (1H, dd, H-2), 4.08-3.91 (3H, m, H-3, H-4, H-5), 3.75 (1H, dd, *J*=9.6Hz, *J*=5.8Hz, H-6a), 3.52 (1H, dd, *J*=5.6Hz, H-6b). $^{13}$C-NMR ($CDCl_3$) :δ104.4 (C-1), 78.3 (C-2), 75.9, 73.7, 72.9, 61.9 (C-6).

Compound 49β: TLC toluene : ethyl acetate=6 : 1 *Rf*= 0.40. $^1$H-NMR ($CDCl_3$):δ7.38-7.25 (15H, m, Ph x 3), 5.20 (1H, dd, *J*=6.6Hz, *J*=53.1Hz, H-1), 4.94, 4.86, 4.85, 4.80, 4.74, 4.69, 4.64 (6H, each d, J=11.6Hz, benzyl methylene x 3), 3.98 (1H, m, H-2), 3.87-3.81 (2H, m, H-4, H-6a), 3.60 (1H, d, H-3), 3.55-3.50 (2H, m, H-5, H-6b). $^{13}$C-NMR ($CDCl_3$) : δ108.5 (C-1), 80.4, 79.1 (C-2), 76.5, 72.5, 61.9(c-6)

# Gal-F

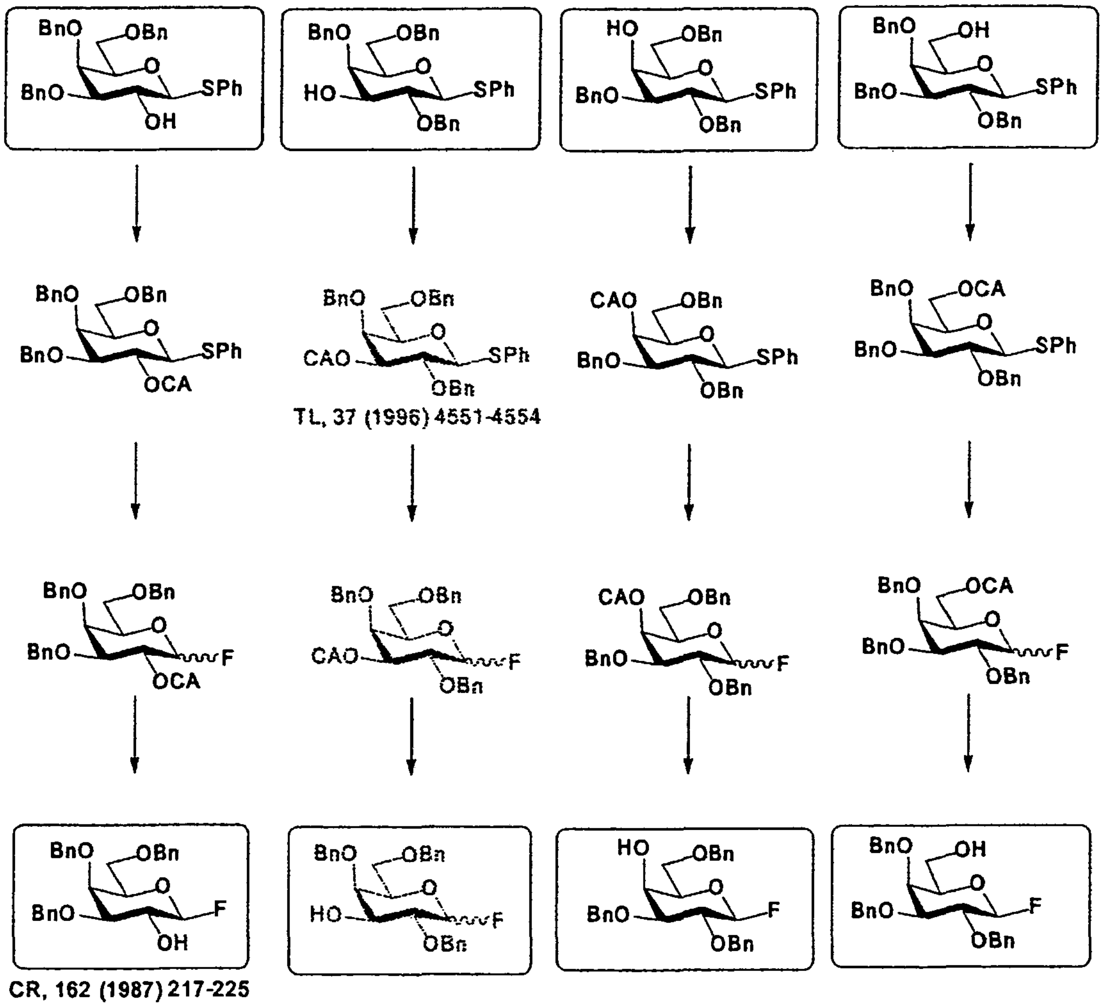


CR, 162 (1987) 217-225

Greenberg W. A.; Priestley E. S.; Sears P. S.; Alper P. B.; Rosenbohm C.; Hendrix M.; Hung S.-C.; Wong C.-H. (1999) *J. Am. Chem. Soc.* 121, 6527-6541.
Kametani T.; Kawamura K.; Honda T. (1987) *J. Am. Chem. Soc.* 109, 3010-17.
Lemanski G.; Ziegler T. (2000) *Tetrahedron* 56, 563-579.
Liptak A.; Jodal I.; Harangi J.; Nanasi P. (1983) *Acta Chim. Hung.* 113, 415-422.
Lemanski G.; Ziegler T. (2000) *Helvetica Chim. Acta* 83, 2655-2675.
Kanie O.; Ito, Y.; Ogawa T. (1996) *Tetrahedron Lett.* 37, 4551-4554.
Oshitari T.; Shibasaki M.; Yoshizawa T.; Tomita M.; Takao K.; Kobayashi S. (1997) *Tetrahedron* 53, 10993-11006.
Magaud D.; Granjean C.; Doutheau A.; Anker D.; Shevchik V.; Cotte-Pattat N.; Robert-Baudouy J. (1998) *Carbohydr. Res.* 314, 189-199.
Marzabadi C. H.; Spilling C. D. (1993) *J. Org. Chem.* 58, 3761-6.
Street I. P.; Withers S. G. (1986) *Can. J. Chem.* 64, 1400-3.
Muragata T.; Kaneko M.; Saito Y.; Saito H.; Suzuki S.; Ogawa T..
Jpn. Kokai Tokkyo Koho (1994), 44 pp. CODEN: JKXXAF JP 06293790 A2 19941021 Heisei. Patent written in Japanese. Application: JP 93-81955 19930408. CAN 123:83943 AN 1995:662332.

(2) Synthesis of xylose derivative

Phenyl 2,3,4-tri-*O*-acetyl-1-thio-β-D-xylopyranoside (50)

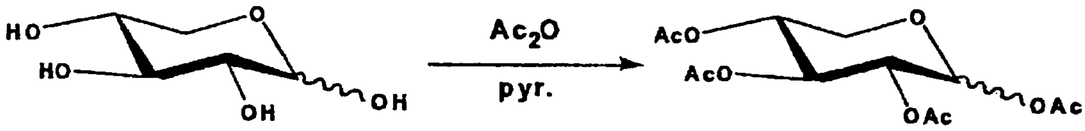


D-xylose 50

D-xylose (10 g, 0.06 mol) was dissolved in pyridine (30 mL), and thereafter, $Ac_2O$ (30mL) was added thereto under cooling on ice. The obtained mixture was stirred at room temperature for 12 hours. After completion of the reaction, toluene was added to the reaction solution, and the obtained mixture was then subjected to vacuum concentration, so as to quantitatively obtain compound 50 (17.6 g, 0.06 mol). TLC toluene : ethyl acetate=4:1 *Rf*=0.42.

Phenyl 2,3,4 -tri-*O*-acetyl-1-thio-β-D-xylopyranoside (51)

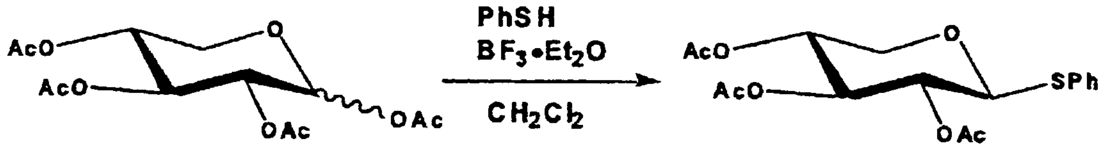


50 51

Compound 50 (23 g, 0.07 mol) was dissolved in $CH_2Cl_2$ (20 mL). Thereafter, PhSH (8.2 mL, 0.08 mol) and $BF_3Et_2O$ (18.3 mL, 0.14 mol) were added thereto under nitrogen stream, while cooling on ice. The obtained mixture was stirred for 2 hours. After completion of the reaction, the reaction solution was extracted with $CH_2Cl_2$, and then dehydrated with $MgSO_4$, followed by vacuum concentration. The resultant was purified by silica gel column chromatography (hexane : ethyl acetate = 4:1), so as to obtain compound 51 (22 g, 0.064 mol) at a yield of 83%. TLC toluene : ethyl acetate=4:1 *Rf*=0.50.

Phenyl 1-thio-β-D-xylopyranoside (52)

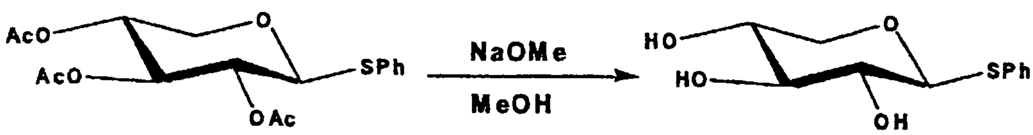


51 52

Compound 51 (22 g, 0.06 mol) was dissolved in methanol (50 mL), and thereafter, 0.5M $CH_3ONa$/methanol (0.5 mL) was added to the solution. The obtained mixture was stirred for 1 hour. After completion of the reaction, an ion exchange resin was added to the reaction solution, so as to return the pH thereof to neutral. After confirming it with a pH test paper, the resultant was filtrated. The obtained filtrate was subjected to vacuum concentration, so as to quantitatively obtain compound 52 (14 g, 0.06 mol). TLC $CH_2Cl_2$ : methanol t=4:1 *Rf*=0.49.

Phenyl 3,4-di-*O*-benzyl-1-thio-β-D-xylopyranoside (53), phenyl 2,4-di-*O*-benzyl-1-thio-β-D-xylopyranoside (54), and phenyl 2,3-di-*O*-benzyl-1-thio-β-D-xylopyranoside (55)

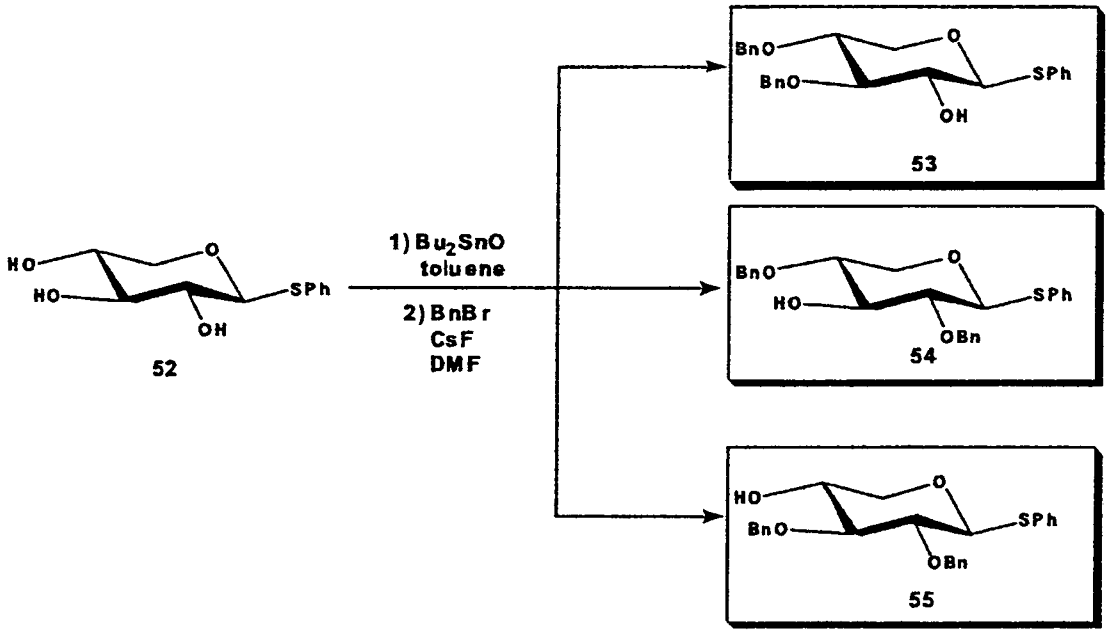


Compound 52 (300 mg, 1.2 mmol) was dissolved in toluene (10 mL), and thereafter, $Bu_2SnO$ (340 mg, 1.3 mmol) was added to the solution. The obtained mixture was heated to reflux for 3 hours, followed by vacuum concentration. The obtained residue was dissolved in DMF (10 mL), and thereafter, CsF (900 mg, 7.2 mmol) and BnBr (800 μL, 7.2.mmol) were added thereto. The obtained mixture was then stirred for 2 hours. After completion of the reaction, the reaction solution was extracted with ethyl acetate, washed with $NaHCO_3$, and then dehydrated with $MgSO_4$, followed by vacuum concentration. The resultant was purified by silica gel column chromatography (toluene : ethyl acetate = 10: 1), so as to obtain compound 53 (180 mg, 36.1%), compound 54 (250 mg, 50.1%), and compound 55 (26 mg, 5.3%).
Compound (53): TLC toluene : ethyl acetate=2:1 *Rf*=0.73. $^1$H-NMR ($CDCl_3$)δ: 7.51-7.23 (15H, m, Ph x 3), 5.30 (1H, d, $J_{1,2}$=4.7Hz, H-1), 4.79, 4.75, 4.64, 4.60 (4H, d. benzyl methylene x 2), 4.41 (1H, dd, $J_{4,5}$a=11.9Hz, $J_{5a5b}$=11.9Hz, H-5a), 3.71 (1H, t, $J_{2,3}$=4.9Hz, H-2), 3.64 (1H, q, H-4), 3.51 (1H, dd, $J_{4,5b}$=5.2Hz, $J_{5a,5b}$=11.5Hz, H-5b), 3.10 (1H, d, H-3). $^{13}$C-NMR ($CDCl_3$) δ: 86.7, 77.8, 73.5, 73.4, 71.9, 68.0, 65.4, 64.7.
Compound (54): TLC toluene : ethyl acetate=2 : 1 *Rf*=0.79. $^1$H-NMR ($CDCl_3$) δ: 7.54-7.21 (15H, m, Ph x 3), 4.92 (1H, d, $J_{1,2}$=6.2Hz, H-1), 4.83, 4.76, 4.65, 4.61 (4H, d, benzyl methylene x 2), 4.30 (1H, dd, $J_{4,5a}$=11.8Hz, $J_{5a,5b}$=3.1Hz, H-5a), 3.72 (1H, t, *J*=5.90Hz, H-2), 3.62 (1H, t, $J_{3,4}$=6.1Hz, H-3), 3.58 (1H, q, H-4), 3.57 (1H, dd, $J_{4,5b}$=6.0Hz, $J_{5a,5b}$=2.9Hz). $^{13}$C-NMR ($CDCl_3$) δ: 88.1, 80.4, 77.9, 77.3, 76.7, 75.2, 73.1, 67.5.
Compound (55): TLC toluene : ethyl acetate=2 : 1 *Rf=0.75.* $^1$H-NMR ($CDCl_3$)δ: 7.54-7.21 (15H, m, Ph x 3), 4.71 (1H, d, $J_{1,2}$=5.1Hz), 4.92, 4.74, 4.68, 4.61 (4H, d, benzyl methylene x 2), 4.04 (1H, dd, $J_{4,5}$=5.0Hz, $J_{5a,5b}$=11.6Hz, H-5a), 3.72 (1H, t, $J_{2,3}$=8.8Hz, H-2), 3.51 (1H, q, H-4), 3.30 (1H, t, $J_{3,4}$=9.3 Hz, H-3), 3.20 (1H, t, $J_{5a,5b}$ =11.1Hz, H-5b). $^{13}$C-NMR ($CDCl_3$)δ: 88.9, 79.2, 75.9, 73.9, 72.4, 70.8.

**PhS-β-Xyl & Xylo-F**

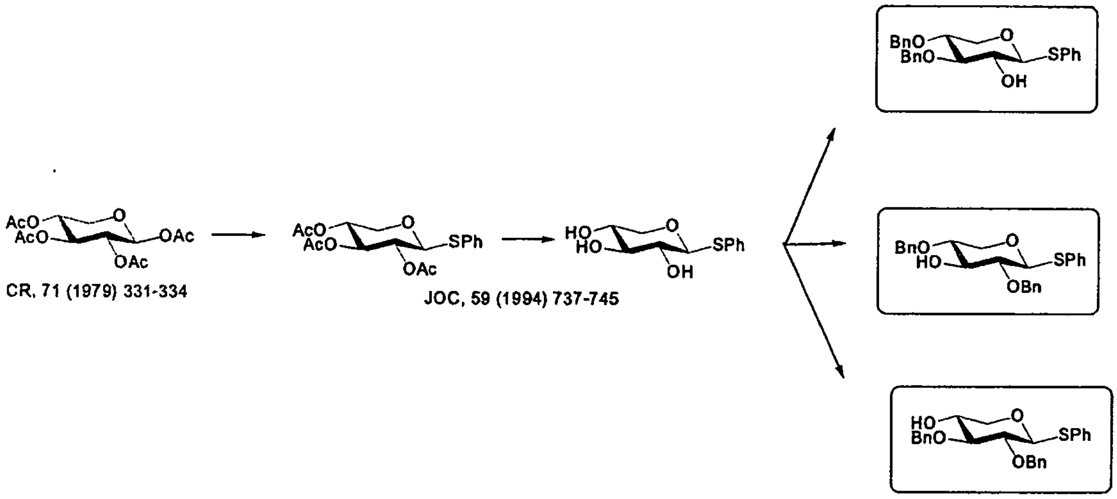


Lee E.; Bruzzi A.; O'Brien E.; O'Colla P. S. (1979) *Carbohydr. Res.* 71, 331-4.
Lopez R.; Fernandez-Mayoralas A. (1994) *J. Org. Chem.* 59, 737-45.

(3) Synthesis of fucose derivative

1,2,3,4,6-penta-*O*-acetyl-β-L-fucopyranoside (56)

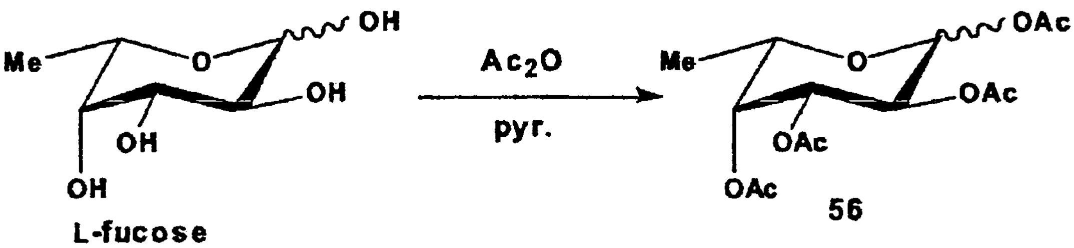


L-fucose

56

L-fucose (10 g, 0.06 mol) was dissolved in pyridine (30 mL), and thereafter, $Ac_2O$ (30 mL) was added to the solution under cooling on ice. The obtained mixture was stirred at room temperature for 12 hours. After completion of the reaction, toluene was added to the reaction solution, followed by vacuum concentration, so as to quantitatively obtain compound 56 (17.6 g, 0.06 mol). TLC toluene : ethyl acetate=4 : 1 *Rf*=0.42.

Phenyl 2,3,4,6-terta-*O*-acetyl-1-thio-β-L-fucopyranoside (57)

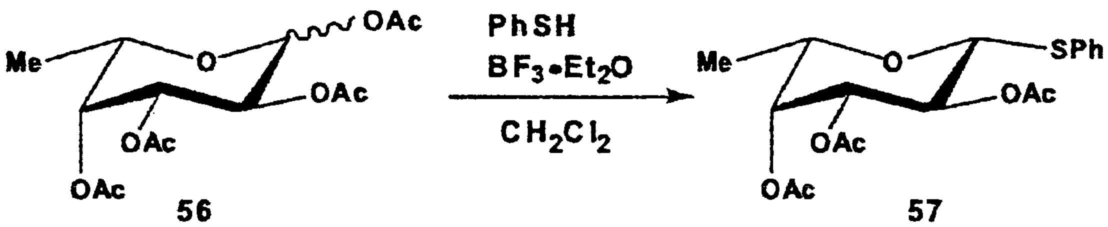

56 57

Compound 56 (22 g, 0.076 mol) was dissolved in $CH_2Cl_2$ (30 mL). Thereafter, PhSH (8.6 mL, 0.083 mol) and $BF_3Et_2O$ (19.3 mL, 0.152 mol) were added to the solution under nitrogen stream, while cooling on ice. The obtained mixture was stirred for 2 hours. After completion of the reaction, the reaction solution was extracted with $CH_2Cl_2$, and then dehydrated with $MgSO_4$, followed by vacuum concentration. The resultant was purified by silica gel column chromatography (hexane : ethyl acetate = 2:1), so as to obtain compound 57 (22 g, 0.064 mol) at a yield of 85%. TLC toluene : ethyl acetate=4:1 *Rf*=0.50.

Phenyl 1-thio-β-L-fucopyranoside (58)

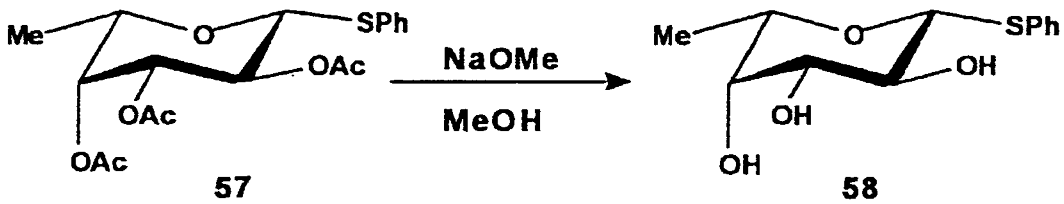

57 58

Compound 57 (18 g, 0.047 mol) was dissolved in methanol (50 mL), and thereafter, sodium methoxide/methanol were added to the solution. The obtained mixture was stirred for 1 hour. After completion of the reaction, an ion exchange resin was added to the reaction solution, so as to return the pH thereof to neutral. After confirming it with a pH test paper, the resultant was filtrated. The obtained filtrate was subjected to vacuum concentration, so as to quantitatively obtain compound 58 (12 g, 0.047 mol). TLC $CH_2Cl_2$ : methanol=4:1 *Rf*=0.49.

Phenyl 3,4-O-benzylidene-1-thio-β-L-fucopyranoside (59)

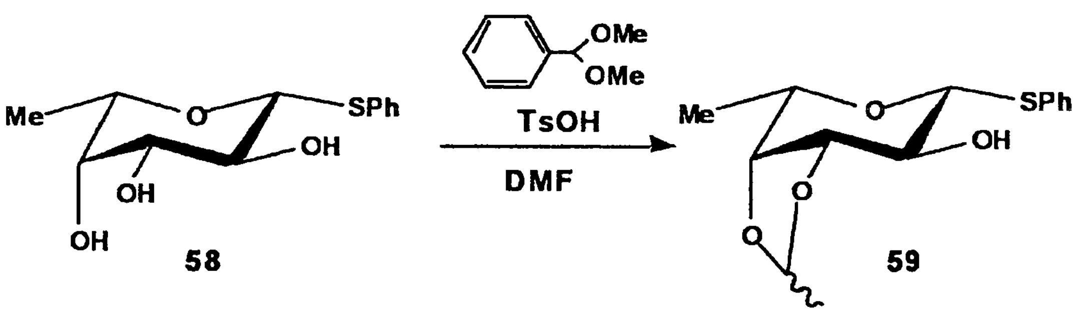

58 59

Compound 58 (2.0 g, 5.9 mmol) was dissolved in DMF (7.0 mL). Thereafter, benzaldehyde dimethyl acetal (1.33 mL, 8.9 mmol) and TsOH (112 mg, 0.59 mmol) were added to the solution. The obtained mixture was stirred at 45°C for 1 hour. After completion of the reaction, the reaction solution was neutralized with triethylamine, followed by

vacuum concentration. The resultant was purified by silica gel column chromatography (hexane : ethyl acetate = 3 : 1), so as to obtain compound 59 (1.84g (a,b mixture), 5.1 mmol) at a yield of 87%.

Compound 59a: TLC toluene : ethyl acetate=4 : 1 *Rf*=0.52. $^1$H-NMR ($CDCl_3$):δ7.54-7.2.6 (10H, m, Ph x 2), 6.19 (1H, s, benzylidene methine), 5.17 (1H, dd, *J=6.8Hz, J=10.4Hz,* H-2), 4.67 (1H, d, *J*=10.0Hz, H-1), 4.48 (1H, t, *J=5.7Hz,* H-4), 4.11 (1H, dd, *J=2.9Hz, J=5.3Hz,* H-3), 3.87 (1H, q, H-5), 1.38 (3H, d, *J=6.5Hz,* H-6).

Compound 59b: TLC toluene : ethyl acetate=4 . 1 *Rf*=0.49. $^1$H-NMR ($CDCl_3$):δ7.89-7.27(10H, m, Phx2), 5.89 (1H, s, benzylidenebenzylidene methine), 5.06 (1H, dd, *J*=6.7Hz, *J*=10.0Hz, H-2), 4.69 (1H, d, *J*=10.1Hz, H-1), 4.36 (1H, t, *J*=6.3Hz, H-4), 4.15 (1H, dd, *J=2.1Hz,* J=5.8Hz, H-3), 3.98 (1H, q, H-5), 1.38 (3H, d, *J*=6.3Hz, H-6).

Phenyl 2-*O*-benzyl-3,4-*O*-benzylidene-1-thio-β-L-fucopyranoside (60)

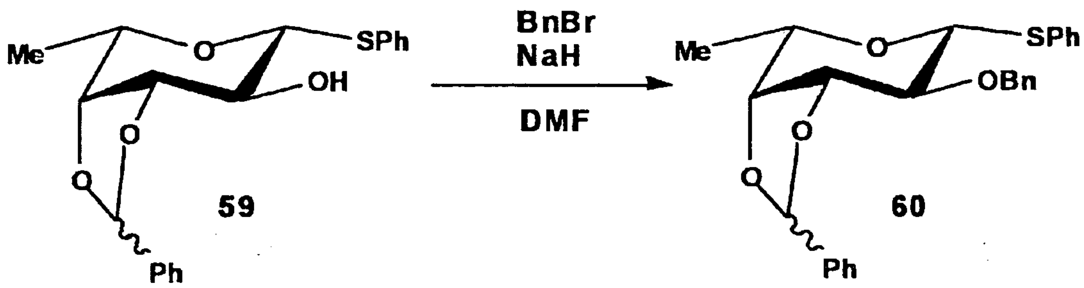


59 60

Compound 59 (1.1 g (a,b mixture), 3.0 mmol) was dissolved in DMF (20 mL). Thereafter, BnBr (760 µL, 6.1 mmol) was added to the solution, and NaH (8.8 mg, 0.61 mmol) was then added thereto while cooling on ice. The obtained mixture was stirred for 1.5 hours. After completion of the reaction, the reaction solution was extracted with ethyl acetate, and then dehydrated with $MgSO_4$, followed by vacuum concentration. The resultant was purified by silica gel column chromatography (hexane : ethyl acetate = 4 : 1), so as to obtain compound 60 (783 mg (a,b mixture), 1.74 mmol) at a yield of 58%.

Compound 60a: $^1$H-NMR ($CDCl_3$):δ7.59-7.22 (15H, m, Ph x 3), 5.99 (1H, s, benzylidene methine), 4.88, 4.78 (2H, each d, *J*=11.4Hz, benzyl methylene), 4.67 (1H, d, *J*=9.5Hz, H-1), 4.55 (1H, t, *J*=6.0Hz, H-4), 4.07 (1H, dd, *J*=9.5Hz, *J*=1.8Hz, H-3), 3.77 (1H, q, H-5), 3.64 (1H, dd, *J*=5.6Hz, *J*=9.8Hz, H-2), 1.41 (3H, d, *J*=6.7Hz, H-6).

Compound 60b: $^1$H-NMR ($CDCl_3$):δ7.58-7.22 (15H, m, Ph x 3), 5.89 (1H, s, benzylidene methine), 4.73, 4.56 (2H, each d, *J*=11.4Hz, benzyl methylene), 4.65 (1H, d, *J= 10.* 1 Hz, H-1), 4.37 (1H, t, *J*=5.8Hz, H-4), 4.11 (1H, dd, *J*=6.5Hz, *J*=2.5Hz, H-3), 3.91 (1H, q, H-5), 3.52 (1H, dd, *J*=5.6Hz, *J*=9.8Hz, H-2), 1.46 (3H, d, *J*=6.5Hz, H-6).

Phenyl 2,3-di-*O*-benzyl-1-thio-β-L-fucopyranoside (61) and phenyl 2,4-di-*O*-9-benzyl-1-thio-β-L-fucopyranoside (62)

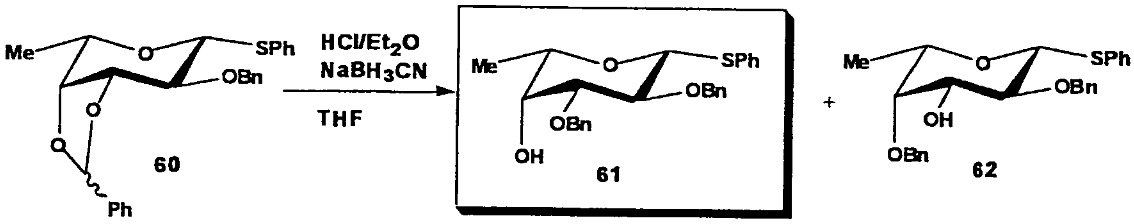


60 61 + 62

Compound 60 (90mg (60 a,b mixture), 0.021 mmol) was dissolved in THF (1.4 mL). Thereafter, MS3A (500 mg) was added to the solution, and $NaBH_3CN$/THF (110 mL, 0.17 mmol) was then added thereto under nitrogen stream. The obtained mixture was stirred for 2 hours. Thereafter, HCl/diethyl ether (5.0 mL) was added to the reaction solution. After completion of the reaction, the reaction solution was extracted with $CH_2Cl_2$, and then dehydrated with $MgSO_4$, followed by vacuum concentration. The resultant was purified by silica gel column chromatography (hexane : ethyl

acetate = 4:1), so as to obtain compound 61 (45 mg, 0.01 mmol, 50%) and compound 62 (25 mg, 0.006 mmol, 27%).

Compound 61: TLC hexane : ethyl acetate=4 : 1 *Rf*=0.89. $^1$H-NMR ($CDCl_3$):δ7.59-7.15 (20H, m, Ph x 4), 4.85, 4.75, 4.70, 4.61 (6H, each, d, benzyl methylene x 2), 4.6 (1H, d, H-1), 3 .81 (1H, t, H-4), 3.69 (1H, t, $J_{2-3}$ =9.24Hz, H-2), 3.59-3.12 (2H, m, H-3, H-5), 1.36 (3H, d, $J_{6-5}$=6.3Hz , H-6), 5.27 (1H, s, OH). $^{13}$C-NMR ($CDCl_3$) : δ87.5 (C-1), 82.8 (C-3), 77.5 (C-5), 76.5 (C-2), 74.2 (C-5), 16.9 (C-6).

Compound 62: $^1$H-NMR ($CDCl_3$): δ7.58-7.1 8 (20H, m, Ph x 4), 4.89, 4.76, 4.72, 4.61 (6H, each, d, benzyl methylene x 2), 4.56 (1H, d, $J_{1,2}$=B.SHz, H-1), 3.70-3.63 (2H, m, H-2, H-4), 3.59-3.52 (2H, m, H-3, H-5), 1.36 (3H, d, $J_{6-5}$=6.3, H-6), 5.26 (1H, s, OH). $^{13}$C-NMR ($CDCl_3$) :δ87.1 (C-1), 79.3 (C-3), 77.9 (C-5), 76.0 (C-2), 74.5 (C-5), 16.6 (C-6).

Phenyl 2-*O*-benzyl-1-thio-β-L-fucopyranoside (63)

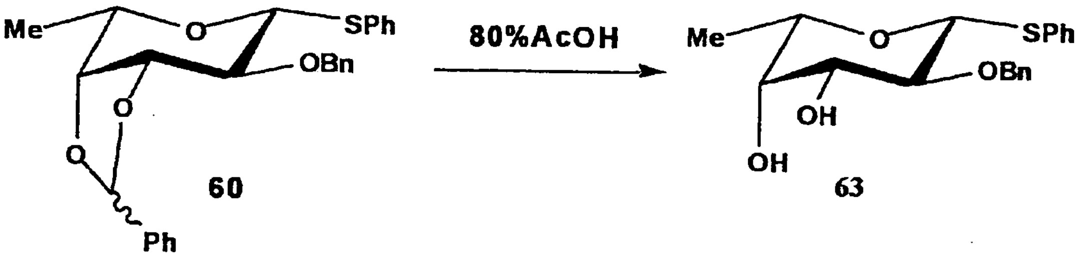


Compound 60 (2.7 g (mix), 6.1 mmol) was dissolved in acetic acid (80 mL), and thereafter, water (20 mL) was added dropwise to the solution. The obtained mixture was stirred at 45°C for 5 hours. After completion of the reaction, the reaction solution was subjected to vacuum concentration. The resultant was purified by silica gel column chromatography ($CH_2Cl_2$ : methanol = 100:1), so as to obtain compound 63 (1.9 g, 5.6 mmol) at a yield of 91%. TLC toluene : ethyl acetate =4 : 1 *Rf*=0.22.

Phenyl 2,3-di-*O*-benzyl-1-thio-β-L-fucopyranoside (61)

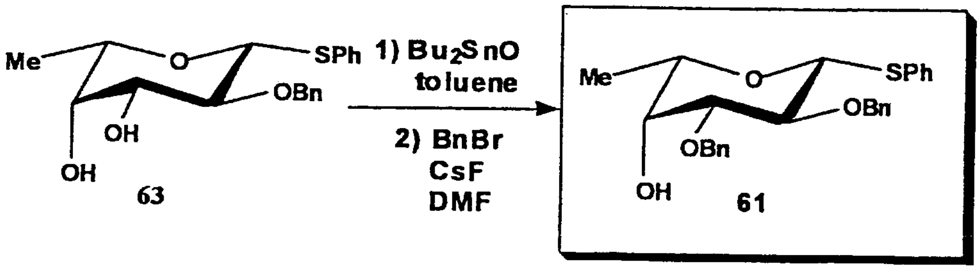


Compound 63 (1.9 g, 5.6 mmol) was dissolved in toluene (30 mL), and $Bu_2SnO$ (1.66 g, 6.6 mmol) was then added thereto. The obtained mixture was heated to reflux for 3 hours, and the reaction solution was then subjected to vacuum concentration. The obtained residue was dissolved in DMF (30 mL), and thereafter, CsF (1.1 g, 6.6 mmol) and BnBr (820 μL, 6.6 mmol) were added to the solution. The obtained mixture was stirred for 3 hours. After completion of the reaction, the reaction solution was extracted with ethyl acetate, washed with $NaHCO_3$, and then dehydrated with $MgSO_4$, followed by vacuum concentration. The resultant was purified by silica gel column chromatography (toluene : ethyl acetate = 10:1), so as to obtain compound 61 (1.9 g, 2.4 mmol) at a yield of 89%. TLC toluene : ethyl acetate =4: 1 *RF=-0.51.*

Phenyl 2,3-di-O-benzyl-4-O-succinoyl-1-thio-β-L-fucopyranoside (64)

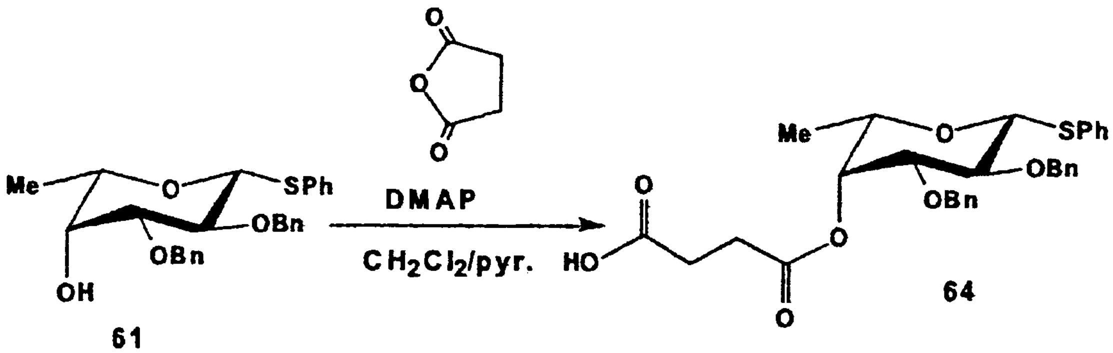


61 64

Compound 61 (492 mg, 1.13 mmol) was dissolved in pyridine (5.0 mL) and $CH_2Cl_2$ (5.0 mL). Thereafter, DMAP (278 mg, 2.25 mmol) and succinic anhydride (225 mg, 2.25 mmol) were added to the solution. The obtained mixture was stirred at room temperature for 12 hours. After completion of the reaction, the reaction solution was extracted with $CH_2Cl_2$, washed with HCl, and then dehydrated with $MgSO_4$, followed by vacuum concentration. The resultant was purified by silica gel column chromatography (toluene : ethyl acetate = 2:1), so as to obtain compound 64 (556 mg, 1.02 mmol) at a yield of 92%. TLC toluene : ethyl acetate=2:1 *Rf*=0.32. $^1$H-NMR ($CDCl_3$):δ7.59-7.25 (15H, m, Ph x 3), 5.38 (1H, d, H-4), 4.63 (1H, d, H-1), 4.78, 4.74, 4.71, 4.50 (4H, each, d, *J*=10.6Hz, benzyl methylene x 2), 3.70-3.31 (3H, m, H-2, H-3, H-5), 2.74 (4H, m, $COC\underline{H_2}C\underline{H_2}CO$), 1.24 (3H, d, $J_{6\text{-}5}$=6.3Hz , H-6). $^{13}$C-NMR ($CDCl_3$) : 177.5 (CO), 171.8 (CO), 87.4 (C-1), 70.2 (C-4), 16.8 (C-6), 81.1, 76.3, 71.8.

## PhS-β-Fuc

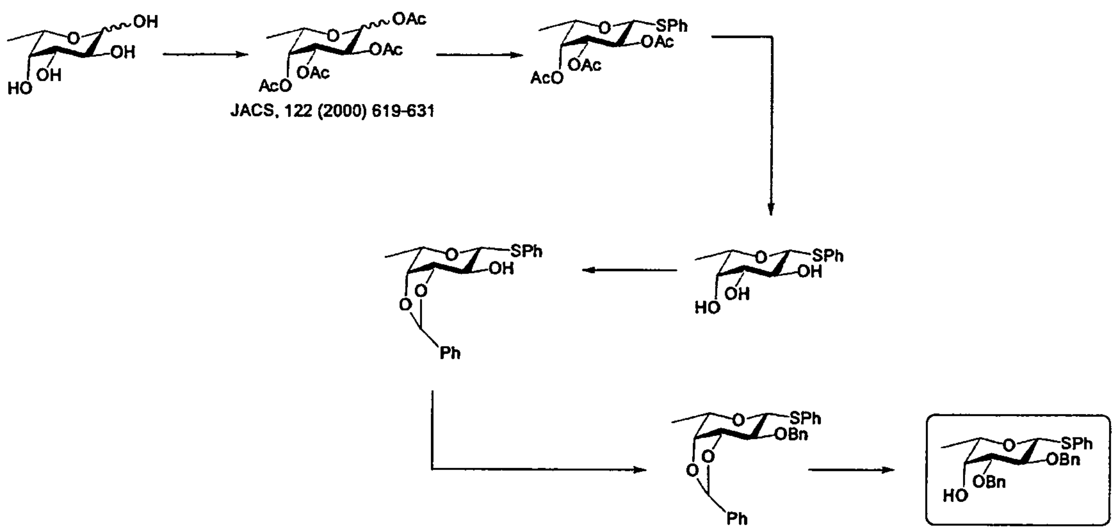


Lee E.; Bruzzi A.; O'Brien E.; O'Colla P. S. (1979) *Carbohydr. Res.* 71, 331-4.
Lopez R.; Fernandez-Mayoralas A. (1994) J. *Org. Chem.* 59, 737-45.

(4) Synthesis of galactosamine derivative

Phenyl 2-acetamide-3,4,6-tri-*O*-acetyl-2-deoxy-1-thio-β-D-galactopyranoside (2)

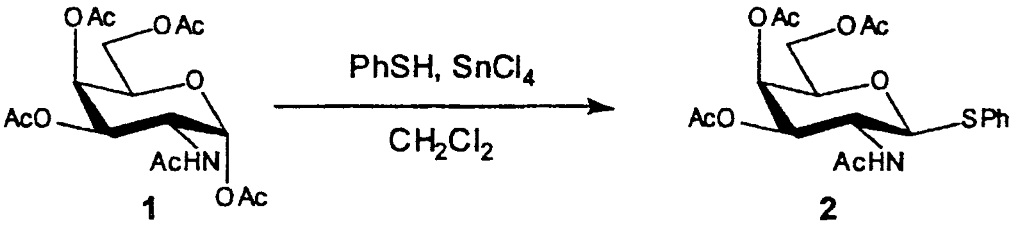


PhSH (0.2 mL, 2.6 mmol) and $SnCl_4$ (0.2 mL, 1.7 mmol) were added to a $CH_2Cl_2$ solution (5 mL) containing compound 1 (1.0 g, 2.6 mmol). The obtained mixture was refluxed at an external temperature of 50°C overnight. Thereafter, the temperature was returned to room temperature, and the reaction solution was diluted with ethyl acetate. Thereafter, an aqueous saturated sodium bicarbonate solution was added to the diluted solution. The mixture was extracted with ethyl acetate, washed with $H_2O$, and then dehydrated with $MgSO_4$. Thereafter, the solvent was distilled away. The obtained crystals were recrystallized with ethyl acetate-hexane, so as to obtain compound 2 (0.53 g, 46.9%). $^1H$ NMR ($CDCl_3$): δ 7.53-7.50 (m, 2H, aromatic-H), 7.30-7.28 (m, 3H, aromatic-H), 5.74 (br.d, 1H, *J*= 9.2Hz, NH), 5.38 (d, 1H, *J* = 3.3 Hz, H-4), 5.23 (dd, 1H, J = 3.3, 10.7 Hz, H-3), 4.94 (d, 1H, J = 10.5 Hz, H-1), 4.26-4.08 (m, 3H, H-2 and H-6), 3.95 (t, 1H, *J* = 6.5 Hz, H-5), 2.13, 2.03, 1.99, 1.97 (each 3H, s, $COCH_3$). $^{13}C$ NMR ($CDCl_3$): δ 87.06 (C-1), 74.39 (C-5), 71.07 (C-3), 66.96 (C-4), 61.85 (C-6), 49.74 (C-2), 23.36, 20.61 ($CH_3$).

Phenyl 2-azido-2-deoxy-1-thio-β-D-galactopyranoside (3)

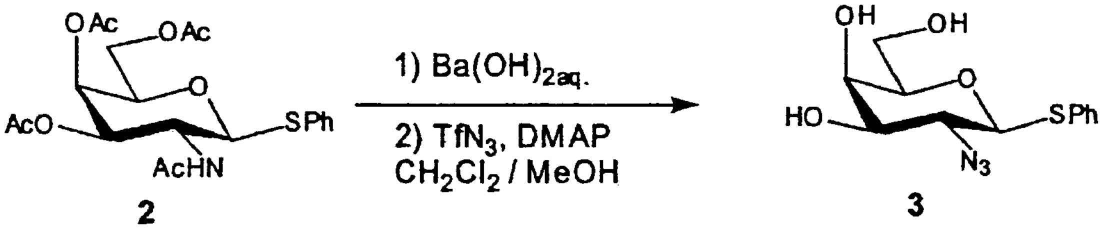


Compound 2 (1.0 g, 2.4 mmol) was added to an aqueous saturated barium hydroxide solution (150 mL). The obtained mixture was refluxed at an external temperature of 120°C for 24 hours. Thereafter, the bubbling of $CO_2$ gas was performed on the reaction solution, and the resultant was then filtrated with a glass filter. Thereafter, the solvent was distilled away from the filtrate. The residue was dissolved in methanol (50 mL), and thereafter, DMAP (0.32 g, 2.6 mmol) and 0.4M $TfN_3CH_2Cl_2$ solution (15 mL, 0.6 mmol) were added to the solution. The obtained mixture was stirred at room temperature under nitrogen stream for 2 days, and the solvent was distilled away. The residue was subjected to silica gel column chromatography (silica gel: 20 g), and compound 3 (0.92 g, quant. in 2 steps) was obtained from the ethyl acetate elution portion. $^1H$ NMR ($CD_3OD$):δ7.59-7.55 (m, 2H, aromatic-H), 7.33-7.26 (m, 3H, aromatic-H), 4.51 (d, 1H, J = 9.9 Hz, H-1), 3.85 (br.s, 1H, H-4), 3.77 (dd, 1H, J = 6.5, 11.3 Hz, H-6), 3.70 (dd, 1H, J = 5.0, 11.3 Hz, H-6), 3.54-3.49 (m, 3H, H-2, 3 and 5H). $^{13}C$ NMR ($CD_3OD$):δ88.12 (C-1), 80.64 (C-3), 75.22 (C-5), 69.72 (C-4), 64.52 (C-2), 62.74 (C-6).

Phenyl 2-azido-4,6-O-benzylidene-2-deoxy-1-thio-β-D-galactopyranoside (4)

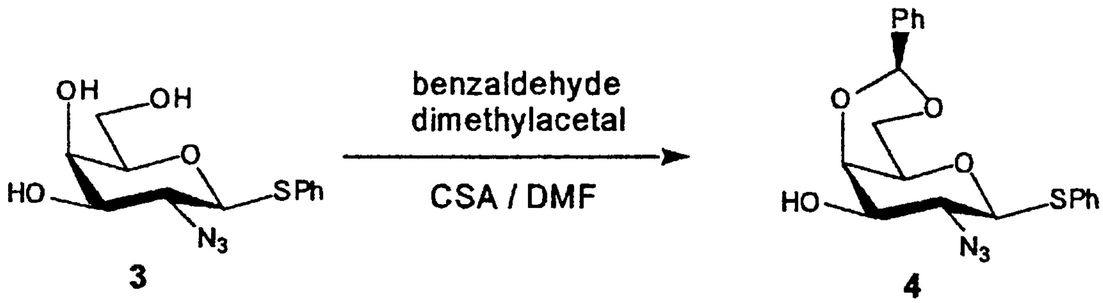


3

4

Benzaldehyde dimethyl acetal (40 µl, 0.27 mmol) and CSA (one microspatula) were added to a DMF solution (1 mL) containing compound 3 (37 mg, 0.12 mmol). Thereafter, the mixture was stirred at an external temperature of 60°C for 3 hours. Thereafter, the reaction solution was poured into an aqueous saturated sodium bicarbonate solution that had been cooled on ice. The mixture was extracted with ethyl acetate, washed with brine, and then dehydrated with $MgSO_4$. Thereafter, the solvent was distilled away. The residue was subjected to silica gel column chromatography (silica gel: 10 g), and as a result, compound 4 (47 mg, 97.6%) was obtained from the n-hexane : ethyl acetate = 1:1 elution portion. $^1$H NMR ($CDCl_3$):δ5.51 (s, 1H, C*H*Ph), 4.40 (d, 1H, *J* = 9.8 Hz, H-1), 4.37 (dd, 1H, *J* = 2.0, 12.5 Hz, H-6), 4.15 (d, 1H, *J* = 2.3 Hz, H-4), 4.00 (dd, 1H, *J* = 1.8, 12.6 Hz, H-6), 3.53 (t, 1 H, *J* = 9.3 Hz, H-2). $^{13}$C NMR ($CDCl_3$) : δ101.31 (CHPh), 85.07 (C-1), 74.41 (C-4), 73.08 (C-3 or C-5), 69.82 (C-3 or C-5), 69.15 (C-6), 62.03 (C-2).

Phenyl 2-azido-4,6-*O*-benzylidene-2-deoxy-3-O-*p*-methoxybenzyl-1-thio -β-D-galactopyranoside (5)

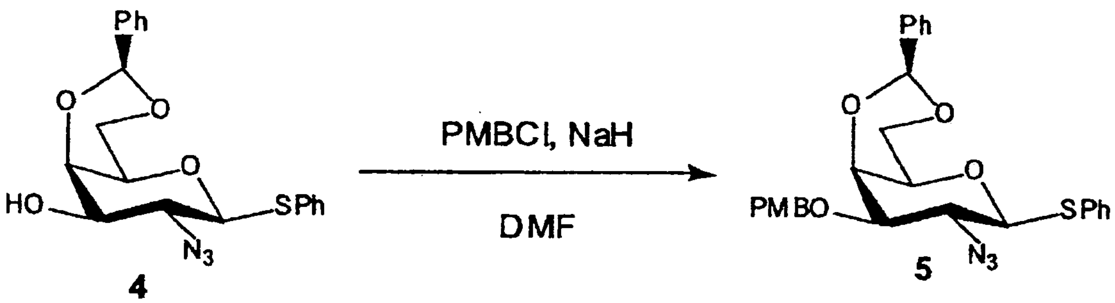


4

5

60% NaH (51 mg, 1.28 mmol) was added to a DMF solution (8 mL) containing compound 4 (0.38 g, 0.99 mmol). The obtained mixture was stirred at room temperature for 1 hour. Thereafter, PMBCl (150 µl, 1.1 mmol) was added to the reaction solution, and the obtained mixture was stirred at room temperature overnight. Thereafter, ice was added to the reaction solution. The reaction solution was extracted with ethyl acetate, washed with brine, and then dehydrated with $MgSO_4$. Thereafter, the solvent was distilled away. The residue was subjected to silica gel column chromatography (silica gel: 10 g). As a result, compound 5 (0.35 g, 70.8%) was obtained from the n-hexane : ethyl acetate = 2:1 elution portion thereof, and compound 4 (0.9 g, 23.1 %) was obtained from the n-hexane : ethyl acetate = 1:1 elution portion thereof. $^1$H NMR ($CDCl_3$) δ5.46 (s, 1H, CHPh), 4.64 (s, 2H, benzyl methylene), 4.39 (d, 1H, *J* = 9.9Hz, H-1), 4.35 (dd, 1 H, *J* = 1.5, 12.2 Hz, H-6), 4.07 (d, 1H, *J* = 3.3 Hz, H-4), 3.97 (dd, 1H, *J* = 1.5, 12.2 Hz, H-6), 3.80 (t, 1H, *J* = 9.9 Hz, H-2), 3.80 (s, 3H, $OCH_3$), 3.44 (dd, 1H, *J* = 3.3, 9.4 Hz, H-3), 3.37 (br.s, 1H, H-5). $^{13}$C NMR ($CDCl_3$) δ 101.06 (CHPh), 85.14 (C-1), 79.08 (C-3), 72.09 (C-4), 71.12 (benzyl methylene), 69.81 (C-5), 69.26 (C-6), 59.73 (C-2), 55.18 ($CH_3$).

Phenyl 2-azido-2-deoxy-3-O-*p*-methoxybenzyl-1-thio-β-D-galactopyranoside (6)

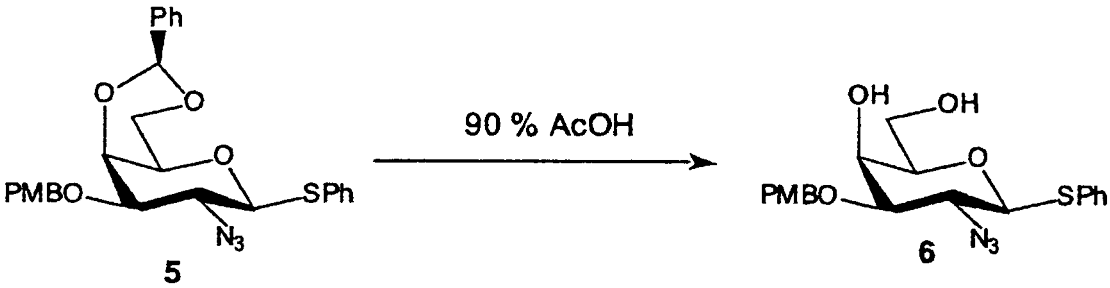


5 6

Compound 5 (0.17 g, 0.30 mmol) was dissolved in an aqueous 90% acetic acid solution. The obtained solution was stirred at an external temperature of 50°C for 4 hours, and the solvent was distilled away. The residue was subjected to silica gel column chromatography (silica gel: 10 g). As a result, compound 5 (0.13 g, 7.3 %) was obtained from the toluene : ethyl acetate = 9:1 elution portion thereof, and compound 6 (0.10 g, 84.9%) was obtained from the toluene : ethyl acetate = 1:1 elution portion thereof. $^1$H NMR ($CDCl_3$) δ4.63 (s, 2H, benzyl methylene), 4.40 (d, 1H, *J*= 10.1 Hz, H-1), 4.00 (d, 1H, *J*= 1.9 Hz, H-4), 3.95 (t, 1H, *J*= 5.8 Hz, H-6), 3.81 (s, 3H, $CH_3$), 3.62 (t, 1H, J = 9.9 Hz, H-2), 3.46 (t, 1H, J = 5.4 Hz, H-5), 3.41 (dd, 1H, *J* = 3.0, 9.5 Hz, H-3). $^{13}$C NMR ($CDCl_3$) δ86.19 (C-1), 80.55 (C-3), 78.15 (C-5), 71.77 (benzyl methylene), 66.03 (C-3), 62.63 (C-6), 60.92 (C-2).

Phenyl 2-azido-4,6-di-*O*-benzyl-2-deoxy-3-*O*-*p*-methoxybenzyl-1-thio -β-D-galactopyranoside (7)

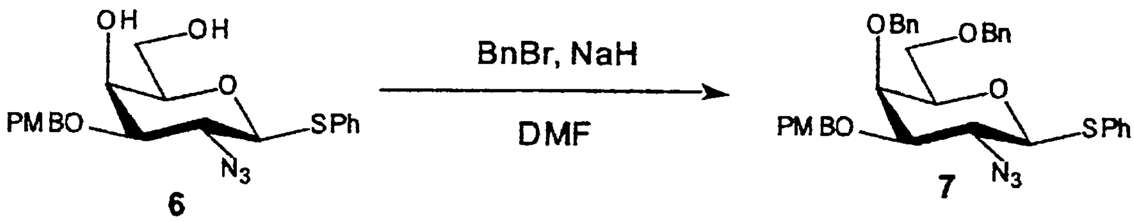


6 7

60 % NaH (0.09 g, 2.2 mmol) was added to a DMF solution (4 mL) containing compound 6 (0.19 g, 0.45 mmol). The obtained mixture was stirred at room temperature for 1 hour. Thereafter, BnBr (260 μl, 2.2 mmol) was added to the reaction solution, and the obtained mixture was stirred at room temperature overnight. Thereafter, ice was added to the reaction solution. The resultant was diluted with ethyl acetate, washed with $H_2O$ and brine, and then dehydrated with $MgSO_4$. Thereafter, the solvent was distilled away. The residue was subjected to silica gel column chromatography (silica gel: 10 g), and as a result, compound 7 (0.17 g, 63.4%) was obtained from the n-hexane : ethyl acetate = 4:1 elution portion thereof. $^1$H NMR ($CDCl_3$)δ4.87 (d, 1H, J = 11.4 Hz, benzyl methylene), 4.66-4.35 (m, 5H, benzyl methylene), 4.39 (d, 1H, *J*= 10.0 Hz, H-1), 3.90 (d, 1H, *J*= 2.2 Hz, H-4), 3.81 (t, 1H, *J*= 9.9 Hz, H-2), 3.77 (s, 3H, $CH_3$), 3.64-6.61 (m, 2H, H-6), 3.57-3.52 (m, 1H, H-5), 3.39 (dd, 1H, *J*= 2.7, 9.8 Hz, H-3). $^{13}$C NMR ($CDCl_3$)86.31 (C-1), δ2.05 (C-3), 77.32 (C-5), 74.32 73.50 and 71.99 (benzyl methylene), 72.13 (C-4), 68.48 (C-6), 61.40 (C-2), 55.19 ($CH_3$).

Phenyl 2-azido-4,6-di-*O*-benzyl-2-deoxy-1-thio-β-D-galactopyranoside (8)

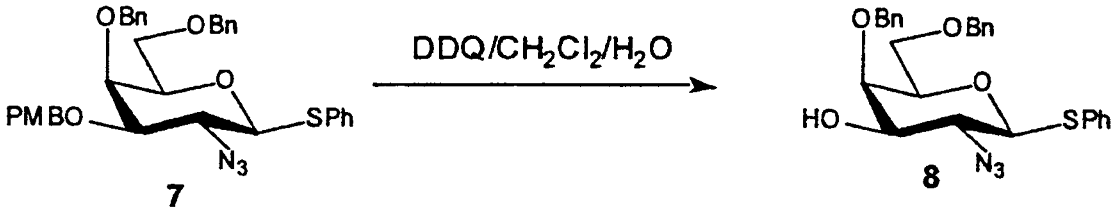


7

8

Compound 8 (0.16 g, 0.27 mmol) was dissolved in $CH_2Cl_2$-$H_2O$ (19 : 1; 2 mL), and thereafter, DDQ (0.07 g, 0.32 mmol) was added thereto under cooling on ice. The obtained mixture was stirred at room temperature for 2 hours. Thereafter, the reaction solution was filtrated with celite, and the solvent was distilled away. The residue was subjected to silica gel column chromatography (silica gel: 10g), and as a result, compound 8 (0.10 g, 80.7%) was obtained from the toluene : ethyl acetate = 9:1 elution portion thereof. $^1$H NMR ($CDCl_3$)δ4.69 (s, 2H, benzyl methylene), 4.55 (d, 1H, *J* = 11.8 Hz, benzyl methylene), 4.48 (d, 1H, *J*= 11.7 Hz, benzyl methylene), 4.42 (d, 1H, *J* = 9.2 Hz, H-1), 3.88 (d, 1H, *J*= 2.1 Hz, H-4), 3.73-3.66 (m, 3H), 3.58-3.55 (m, 2H), 2.24 (br.s, 1H, 3-OH). $^{13}$C NMR ($CDCl_3$)δ85.40 (C-1), 77.36 (C-3 or C-5), 75.25 (C-4), 74.30 (C-3 or C-5), 74.99 and 73.50 (benzyl methylene), 68.14 (C-6), 63.33 (C-2).

Phenyl 2-azido-3-*O*-benzyl-4,6-*O*-benzylidene-2-deoxy-1-thio-β-D-galactopyranoside (9)

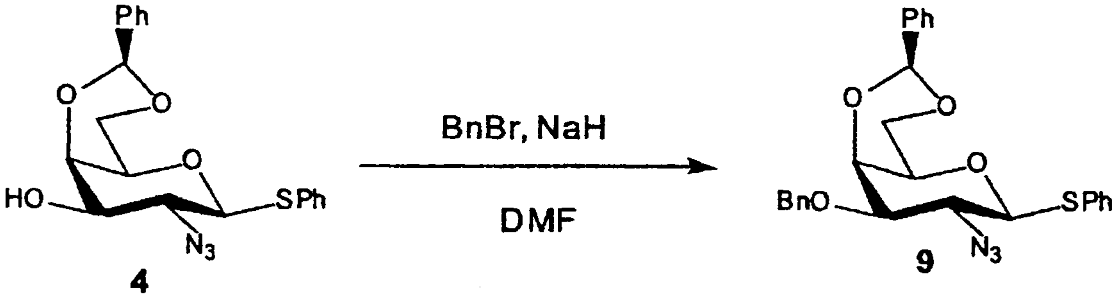


4

9

60% NaH (0.10 g, 2.4 mmol) was added to a DMF solution (10 mL) containing compound 4 (0.58 g, 1.50 mmol). The obtained mixture was stirred at room temperature for 1 hour. Thereafter, BnBr (0.26 μl, 2.1 mmol) was added to the reaction solution, and the obtained mixture was stirred at room temperature overnight. Thereafter, ice was added to the reaction solution. The obtained mixture was extracted with ethyl acetate, washed with brine, and then dehydrated with $MgSO_4$. Thereafter, the solvent was distilled away. The residue was subjected to silica gel column chromatography (silica gel: 20g), and as a result, compound 9 (0.55 g, 77.2%) was obtained from the n-hexane : ethyl acetate = 2:1 elution portion thereof. $^1$H NMR ($CDCl_3$) δ5.43 (s, 1H, C*H*Ph), 4.68 (s, 2H, benzyl methylene), 4.37 (d, 1H, J = 10.0Hz, H-1 ), 4.32 (dd, 1 H, J = 1.1, 12.5 Hz, H-6), 4.06 (d, 1H, *J* = 3.0 Hz, H-4), 3.93 (dd, 1H, J = 1.5, 12.5 Hz, H-6), 3.79 (t, 1 H, J = 9.8 Hz, H-2), 3.43 (dd, 1H, J = 3.1, 9.6 Hz, H-3), 3.33 (d, 1H, J = 0.8 Hz, H-5). $^{13}$C NMR ($CDC_3$) δ101.03 (CHPh), 85.10 (C-1), 79.46 (C-3), 71.99 (C-4), 71.48 (benzyl methylene), 69.74 (C-5), 69.24 (C-6), 59.73 (C-2)

**PhS-β-GalN3**

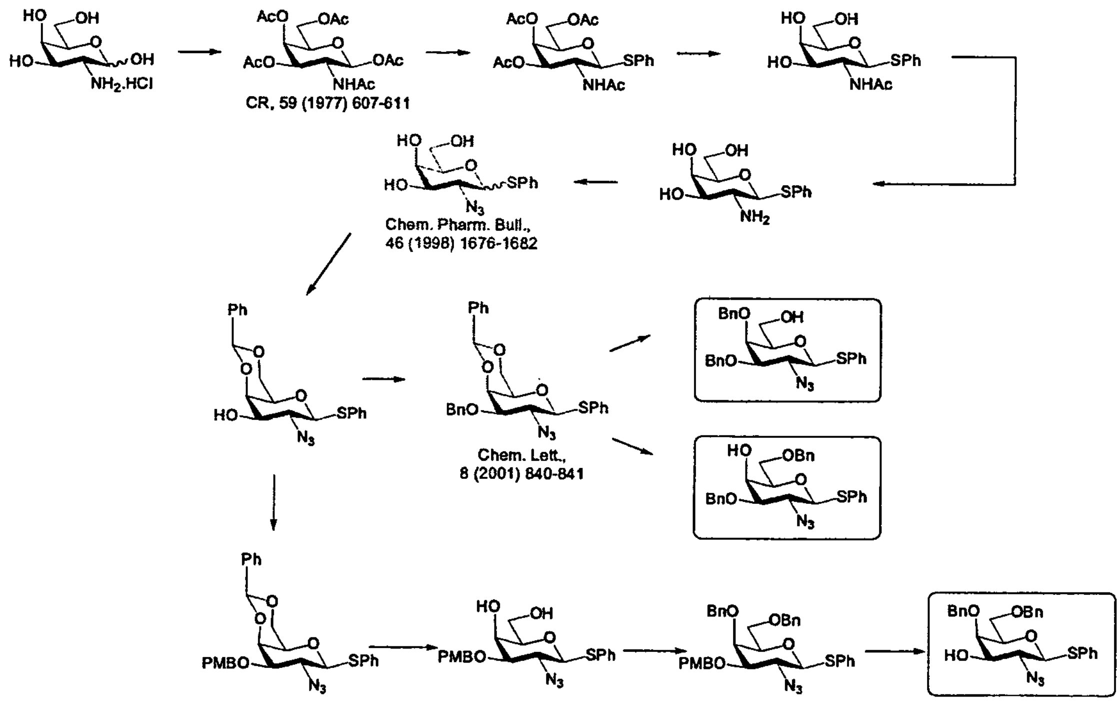


Horton D.; Rodemeyer G.; Saeki H. (1977) *Carbohydr. Res.* 59, 607-11.
Miyajima K.; Nekado T.; Ikeda K.; Achiwa K. (1998) *Chem. Pharm. Bull.* 46, 1676-1682.
Mukaiyama T.; Ikegai K.; Jona H.; Hashihayata T.; Takeuchi K. (2001) *Chem. Lett.* 840-841.

(5) Synthesis of glucose derivative

Phenyl 4,6-*O*-benzylidene-3-*O*-*p*-methoxybenzyl-1-thio-β-D-glucopyranoside (2)

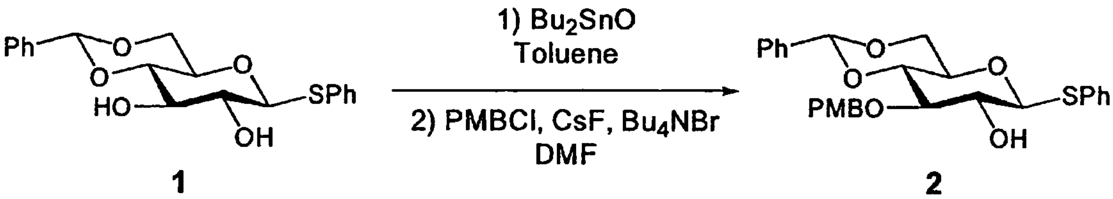


1 2

$Bu_2SnO$ (85 mg, 0.34 mmol) was added to a toluene solution (4 mL) containing compound 1 (120 mg, 0.33 mmol). The obtained mixture was refluxed in nitrogen atmosphere at an external temperature of 150°C for 3 hours. Thereafter, the solvent was distilled away. The residue was dissolved in DMF (3.3 mL), and thereafter, PMBCl (54 μl, 41 mmol) and CsF (64 mg, 42 mmol) were added to the solution. The obtained mixture was then stirred at room temperature overnight. Thereafter, the reaction solution was extracted with AcOEt, washed with brine, and then dehydrated with $Na_2\ SO_4$. Thereafter, the solvent was distilled away. The residue was subjected to silica gel column chromatography (silica gel: 11 g), and as a result, compound 2 (110 mg, 75%) was obtained from the toluene/AcOEt = 20/1 elution portion.

$^1$HNMR($CDCl_3$)7.53-6.82(m, 14H, *Ph, SPh, PMB),* 5.55(s, 1H, C*H*Ph), 4.87(d, 1H, benzyl methylene), 4.71(d, 1H, benzyl methylene), 4.61(1H, d, $J_{1,2}$=9.7Hz, H-1), 4.37(dd, 1H, $J_{5,6}$,=5.0Hz, $J_{6,6'}$=10.5Hz, H-6'), 3.79-3.75(m, 4H, $J_{6,6'}$=10.5Hz, H-6, $CH_3$), 3.67-3.60(m, 2H, H-3, H-4), 3.50-3.46(m, 2H, $J_{1,2}$=9.7Hz, H-2, $J_{5,6'}$=10.5Hz, H-5); $^{13}$CNMR($CDCl_3$)159.45, 137.30, 133.19, 131.47, 129.83, 129.05, 128.34, 128.29, 126.07, 113.95(aromatic-C), 101.29(CHPh), 88.45(C-1), 81.30,

81.17, 74.48(benzyl methylene), 72.25(C-2), 70.79(C-5), 68.67(C-6), 55.28($CH_3$).

Phenyl 3-*O*-*p*-methoxybenzyl-1-thio-β-D-glucopyranoside (3)

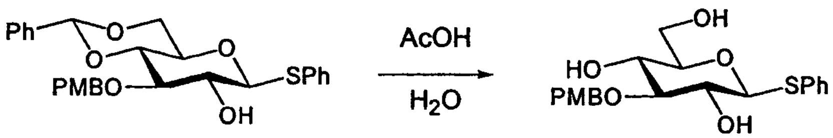


2 3

Compound **2** (78 mg, 0.16 mmol) was dissolved in an aqueous 90% AcOH solution. The obtained mixture was stirred at an external temperature of 50°C for 3 hours. Thereafter, the solvent was distilled away. The residue was subjected to silica gel column chromatography (silica gel: 10g), and as a result, compound 3 (56 mg, 88%) was obtained from the toluene : AcOEt = 1:1 elution portion thereof.
$^1$HNMR($CD_3OD$)7.53-6.86(m, 9H, *SPh, PMB),* 4.85(d, 1H, benzyl methylene), 4.68(d, 1H, benzyl methylene), 4.45(1H, d, $J_{1,2}$=9.7Hz, H-1), 3.85-3.62(m, 5H, H-6', H-6, $CH_3$), 3.33-3.26(m, 4H, H-2, H-3, H-4, H-5), 2.59(s, 2H, OH); $^{13}$CNMR ($CD_3OD$)132.71, 130.73,129.86, 128.31, 114.57(aromatic-C), 89.63(C-1), 87.56, 82.04, 75.96, 73.91, 71.21, 62.81(C-6), 55.67($CH_3$)

Phenyl 2,4,6-tri-*O*-benzyl-3-O-p-methoxybenzyl-1-thio-P-D-glucopyranoside (4)

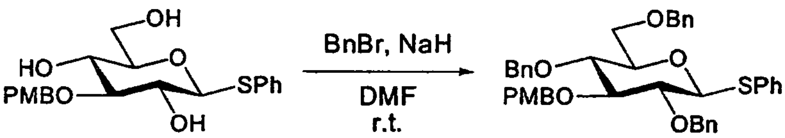


3 4

60%NaH (0.21 mg, 5.2 mmol) was added to a DMF solution (5 mL) containing compound 3 (0.63 mg, 1.6 mmol). The obtained mixture was stirred at room temperature for 1 hour. Thereafter, BnBr (0.7 mL, 5.7 mmol) was added to the reaction solution, and the obtained mixture was then stirred overnight. Thereafter, ice was added to the reaction solution. The obtained mixture was extracted with AcOEt, washed with brine, and then dehydrated with $MgSO_4$. Thereafter, the solvent was distilled away. The residue was subjected to silica gel column chromatography (silica gel: 40 g), and as a result, compound 4 (0.87 mg, 82%) was obtained from the AcOEt : Hex = 1 : 5 elution portion thereof.
$^1$HNMR($CDCl_3$)7.62-6.82(m, 24H, *Ph, SPh, PMB),* 4.92-4.52(m, 9H), 3.77(s, 3H, $CH_3$) 3.76-3.48(m, 5H); $^{13}$CNMR ($CDCl_3$) 159.19, 138.24, 138.06, 133.76, 131.86, 130.49, 129.43, 128.81, 128.36, 128.27, 128.07, 127.78, 127.71, 127.57, 127.48, 127.33, 126.99, 126.86, 113.80(aromatic-C), 87.35(C-1), 86.40, 80.79, 79.03, 76.82, 76.53, 75.45, 75.29, 74.92, 73.32(benzyl methylene), 68.97(C-6), 55.17($CH_3$)

Phenyl 2,4,6-tri-O-benzyl-1-thio-β-**D**-glucopyranoside (5)

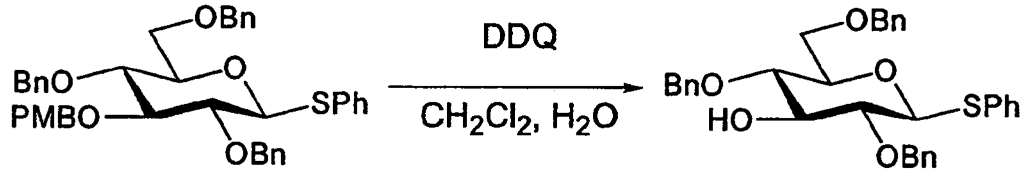


**4** **5**

Compound 4 (93 mg, 0.14 mmol) was dissolved in $CH_2Cl_2$-$H_2O$ (19 : 1; 1 mL). Thereafter, DDQ (34 mg, 0.15 mmol) was added to the solution under cooling on ice, and the obtained mixture was stirred at room temperature for 2 hours. The reaction solution was filtrated with celite, and the solvent was then distilled away. The residue was subjected to silica gel column chromatography (silica gel: 10g), and as a result, compound 5 (68 mg, 89%) was obtained from the toluene : AcOEt = 49 : 1 elution portion thereof.
$^1$HNMR($CDCl_3$)7.58-7.15(m, 20H, *Ph, SPh),* 4.94-4.50(m, 7H, H-1, benzyl methylene), 3.85-3.68(m, 3H, H-3, H-6, H-6'), 3.56-3.48(m, 2H, H-4, H-5), 3.36(t, 1H, $J_{2,3}$=$J_{3,4}$=9.0Hz, H-3); $^{13}$CNMR($CDCl_3$)138.20, 138.06, 133.80, 131.73, 131.61, 128.99, 128.86, 128.73, 128.54, 128.43, 128.28, 128.16, 127.98, 127.89, 127.78, 127.66, 127.53, 127.37(aromatic-C), 87.01(C-1), 80.58(C-2), 78.76, 78.58(C-3), 77.34, 75.04, 74.56, 73.37(benzyl methylene), 69.04(C-6)

**PhS-β-Glc**

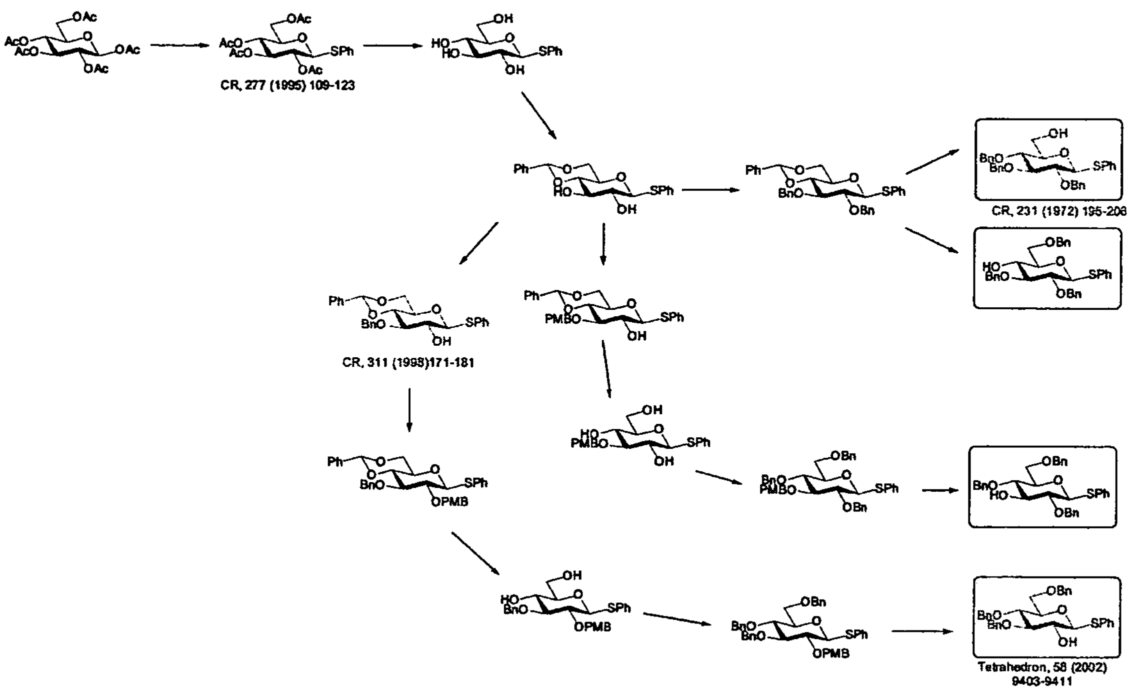


Pfaeffli P. J.; Hixson S. H.; Anderson L. (1972) *Carbohydr. Res.* 231, 195-206.
Motawia M. S.; Olsen C. E.; Enevoldsen K.; Marcussen J.; Moeller B. L. (1995) *Carbohydr. Res.* 277, 109-23.
Bousquet E.; Khitri M.; Lay L.; Nicotra F.; Panza L.; Russo G. (1998) *Carbohydr. Res.* 311, 171-181.
Ennis S. C.; Cumpstey I.; Fairbanks A. J.; Butters T. D.; Mackeen M.; Wormald M. R. (2002) *Tetrahedron* 58, 9403-9411.
Gordon D. M.; Danishefsky S. J. (1990) *Carbohydr. Res.* 206, 361-6
Micheel F.; Kreutzer U. (1969) Justus Liebig. Ann. Chem. 722, 228-31.
Mukaiyama T.; Chiba H.; Funasaka S. (2002) *Chem. Lett.* 392-393.

(6) Synthesis of glucosamine derivative

Phenyl 2-azido-4,6-*O*-benzylidene-2-deoxy-3-*O*-*p*-methoxybenzyl-1-thio -β-D-glucopyranoside (2)

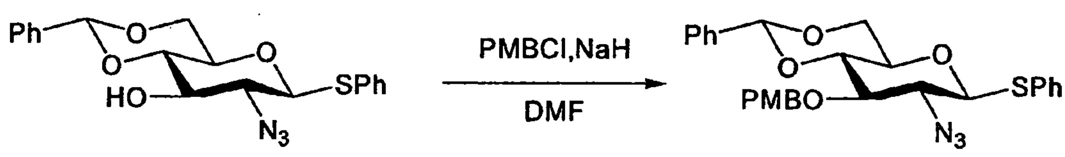


1 2

60%NaH (18 mg, 0.30 mmol) was added to a DMF solution (2.5 mL) containing compound 1 (90 mg, 0.23 mmol). The obtained mixture was stirred at room temperature for 1 hour. Thereafter, PMBCl (38μl, 0.28 mmol) was added to the reaction solution, and the obtained mixture was then stirred for 1 hour. Thereafter, ice was added to the reaction solution. The obtained mixture was extracted with ethyl acetate, washed with brine, and then dehydrated with $Na_2SO_4$. Thereafter, the solvent was distilled away. The residue was subjected to silica gel column chromatography (silica gel: 70 g), and as a result, compound 2 (98 mg, 83%) was obtained from the toluene elution portion thereof.
Compound 2 : $^1$H NMR ($CDCl_3$)δ7.56-7.25 (m, 14H, *Ph*, *SPh*, *PMB*), 5.56 (s, 1H, C*H*Ph), 4.83 (d, 1H, benzyl methylene), 4.72 (d, 1H, benzyl methylene), 4.47 (1H, d, $J_{1,2}$=10.2Hz, H-1), 4.38 (dd, 1H, $J_{5,6'}$=5.0Hz, $J_{6,6'}$=10.5Hz, H-6'), 3.80-3.76 (m, 4H, $J_{6,6'}$=10.5Hz, H-6, $CH_3$), 3.65-3.59 (m, 2H, H-3, H-4), 3.45 (m, 1H, H-5), 3.34 (dd, 1H, $J_{1,2}$=10.2Hz, $J_{2,3}$=8.7Hz, H-2). $^{13}$CNMR ($CDCl_3$)δ159.54, 137.13, 133.97, 130.63, 130.06, 129.70, 129.13, 128.75, 128.32, 126.00, 113.89 (aromatic-C), 101.30 (CHPh), 86.61 (C-1), 81.34 (C-3), 80.58 (C-4), 74.85 (benzyl methylene), 70.55 (C-5), 68.53 (C-6), 64.67 (C-2), 55.28 ($CH_3$).

Phenyl 2-azido-2-deoxy-3-*O*-*p*-methoxybenzyl-1-thio-β-D-glucopyranoside (3)

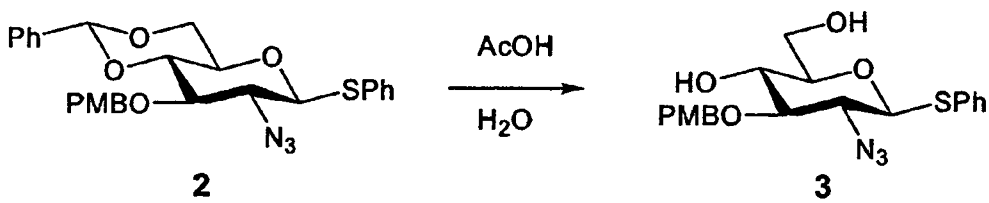


2 3

Compound 2 (0.21 g, 0.42 mmol) was dissolved in an aqueous 90% AcOH solution. The obtained solution was stirred at an external temperature of 50°C for 3 hours. Thereafter, the solvent was distilled away. The residue was subjected to silica gel column chromatography (silica gel: 18 g), and as a result, compound 3 (0.12 g, 67%) was obtained from the toluene : ethyl acetate = 1:1 elution portion thereof.
Compound 3 : $^1$H NMR ($CDCl_3$) δ7.53-6.86 (m, 9H, *SPh, PMB),* 4.85 (d, 1H, benzyl methylene), 4.68 (d, 1H, benzyl methylene), 4.45 (1H, d, $J_{1,2}$=9.7Hz, H-1), 3.85-3.62 (m, 5H, H-6', H-6, $CH_3$), 3.51 (t, 1H, H-3), 3.33-3.26 (m, 3H, H-2, H-4, H-5), 2.59 (s, 2H, OH). $^{13}$C NMR ($CDCl_3$) δ159.54, 133.16, 131.26, 129.82, 129.80, 129.07, 128.40, 114.08 (aromatic-C), 86.34 (C-1), 84.26 (C-5), 79.40 (C-4), 75.05 (benzyl methylene), 70.08 (C-3), 64.85 (C-2), 62.25 (C-6), 55.22 ($CH_3$).

Phenyl 2-azido-4,6-di-*O*-benzyl-2-deoxy-3-*O*-*p*-methoxybenzyl-1-thio -β-D-glucopyranoside (4)

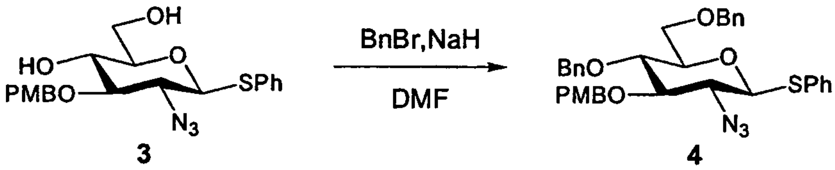


3 4

60% NaH (34 mg, 0.56 mmol) was added to a DMF solution (2.2 mL) containing compound 3 (91 mg, 0.22 mmol). The obtained mixture was stirred at room temperature for 1 hour. Thereafter, BnBr (61 μl, 0.52 mmol) was added to the reaction solution, and the obtained mixture was then stirred for 3 hours. Thereafter, ice was added to the reaction solution. The obtained mixture was extracted with ethyl acetate, washed with brine, and then dehydrated with $Na_2SO_4$. Thereafter, the solvent was distilled away. The residue was subjected to silica gel column chromatography (silica gel: 12g), and as a result, compound 4 (100 mg, 81%) was obtained from the toluene elution portion thereof.

Compound 4 : $^1H$ NMR ($CDCl_3$) δ7.60-6.82 (m, 19H, *Ph, SPh, PMB),* 4.81-4.51 (m, 6H, benzyl methylene), 4.40 (1H, d, $J_{1,2}$=10.1Hz, H-1), 3.77-3.71 (m, 5H, H-6, H-6', $CH_3$), 3.58 (m, 1H, $J_{2,3}$=$J_{3,4}$=9.4Hz, H-3), 3.51-3.44 (m, 2H, $J_{3,4}$=9.4Hz, H-4, H-5), 3.32 (dd, 1H, $J_{1,2}$=10. 1Hz, $J_{2,3}$=9.4Hz, H-2). $^{13}C$ NMR ($CDCl_3$) δ159.55, 138.30, 138.02, 133.70, 131.27, 129.96, 129.87, 129.02, 128.51, 128.41, 128.38, 127.90, 127.82, 127.64, 127.60, 114.00 (aromatic-C), 85.95 (C-1), 84.80 (C-4), 79.44 (C-5), 77.37 (C-3), 75.57, 75.03, 73.48 (benzyl methylene), 68.84 (C-2), 65.14 (C-6), 55.30 ($CH_3$).

Phenyl 2-azido-4,6-di-*O*-benzyl-2-deoxy-1-thio-(3-D-glucopyranoside (5)

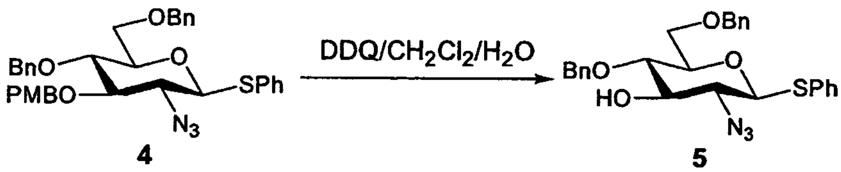


4 5

Compound 4 (0.10 g, 0.12 mmol) was dissolved in $CH_2Cl_2$-$H_2O$ (19:1; 1.8 mL), and thereafter, DDQ (0.05 mg, 0.20 mmol) was added to the solution under cooling on ice. The obtained mixture was stirred at room temperature for 4 hours. Thereafter, the reaction solution was filtrated with celite, and the solvent was then distilled away. The residue was subjected to silica gel column chromatography (silica gel: 10 g), and as a result, compound 5 (0.05 g, 58 %) was obtained from the toluene elution portion thereof.

Compound 5 : $^1H$ NMR ($CDCl_3$)δ7.61-7.22 (m, 15H, *Ph, SPh),* 4.71-4.55 (d, 4H, benzyl methylene), 4.44 (1H, d, $J_{1,2}$=10.1Hz, H-1), 3.80 (dd, 1H, $J_{5,6}$=2.0Hz, $J_{6,6'}$=11.0Hz, H-6'), 3.76 (dd, 1H, $J_{5,6}$=4.0Hz, $J_{6,6'}$=11.0Hz, H-6), 3.59 (t, 1H, H-3), 3.51 (t, 1H, H-4), 3.45 (m, 1H, H-5), 3.28 (dd, 1H, $J_{1,2}$=10.1Hz, H-2). $^{13}C$ NMR ($CDCl_3$)δ138.15, 137.98, 133.39, 131.49, 128.40, 128.29, 128.12, 128.00, 127.65 (aromatic-C), 86.10 (C-1), 79.11 (C-5), 77.34 (C-3), 77.26 (C-4), 73.53 (benzyl methylene), 68.81 (C-6), 65.00 (C-2).

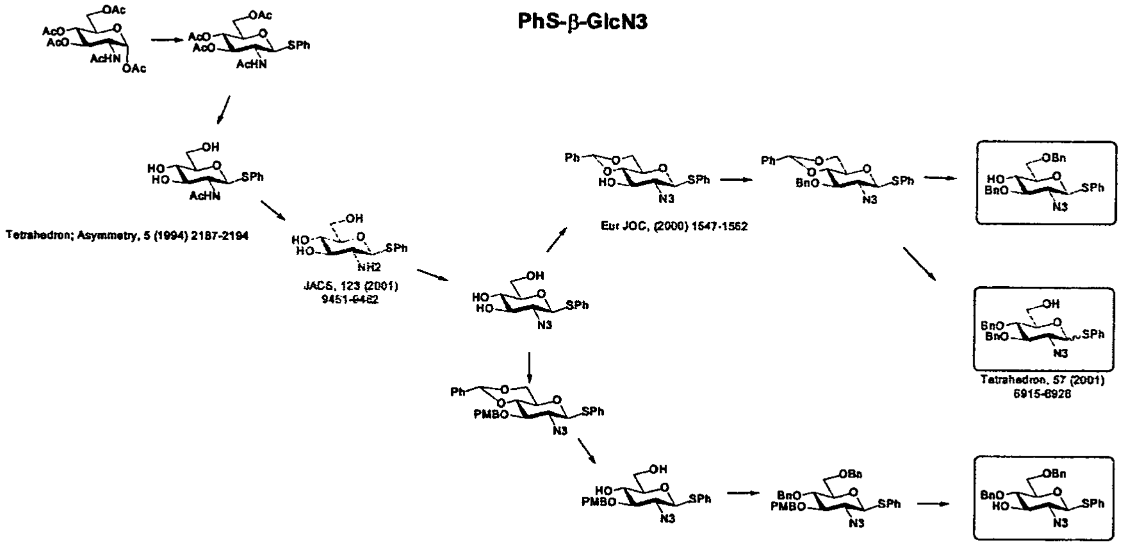


Benakli K.; Zha C.; Kerns R. J. (2001) *J. Am. Chem. Soc.* 123, 9461-9462.
Buskas T.; Garegg P. J.; Konradsson P.; Maloisel J.-L. (1994) *Tetrahedron: Asymmetry 5,* 2187-94.
Martin-Lomas M.; Flores-Mosquera M.; Chiara J. L. (2000) *Europ. J. Org. Chem.* 1547-1562.
Takahashi S.; Kuzuhara H.; Nakajima M. (2001) *Tetrahedron* 57, 6915-6926.
Vos Jan N.; Westerduin P.; Van Boeckel C. A. A. (1991) *Bioorg. Med. Chem. Lett. 1,* 143-6.

(7) Phenyl 2,3-di-*O*-benzyl-1-thio-β-D-glucopyranoside (70)

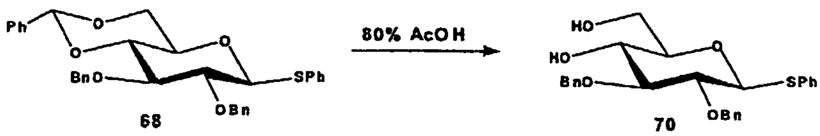


68 70

Compound 68 (1.9 g, 3.3 mmol) was dissolved in acetic acid (120 mL), and thereafter, water (24 mL) was added dropwise to the solution. The obtained mixture was stirred at 45°C for 4 hours. After completion of the reaction, the reaction solution was subjected to vacuum concentration, and the resultant was purified by silica gel column chromatography ($CH_2Cl_2$: methanol = 200 : 1), so as to obtain compound 70 (1.3 g, 2.7 mmol) at a yield of 81%. TLC toluene : ethyl acetate=1:1 *Rf*=0.20. $^1$H NMR ($CDCl_3$):δ7.52-7.26 (15H, m, Ph x 3), 4.97-4.69 (5H, m, H-1, benzyl methylene x 2), 3.91 (1H, dd, *J*=2.9Hz, *J*=7.8Hz, H-6a), 3.76 (1H, t, *J*=6.7Hz, H-4), 3.59 (1H, t, H-6b), 3.53 (1H, t, *J*=6.7Hz, H-3), 3.48 (1H, t, *J*=8.1Hz, H-2), 3.34 (1H, q, H-5).

Phenyl 2,3-di-*O*-benzyl-6-*O*-trityl-1-thio-β-D-glucopyranoside (71)

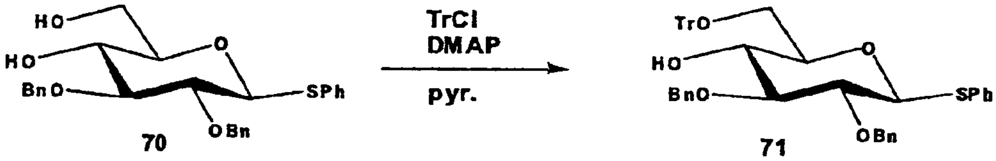


70 71

Compound 70 (696 mg, 1.5 mmol) was dissolved in pyridine (6.0 mL), and thereafter, TrCl (858 mg, 3.1 mmol) and DMAP (188 mg, 1.5 mmol) were added to the solution. The obtained mixture was stirred at 55°C for 12 hours. After completion of the reaction, methanol was added to the reaction solution, and the mixture was then subjected to vacuum concentration. The resultant was purified by silica gel column chromatography (hexane : ethyl acetate = 6 : 1), so as to obtain compound 71 (1.24 g, 2.2 mmol) at a yield of 72%. TLC hexane : ethyl acetate=3 : 1 *Rf*=0.49. $^1$H NMR ($CDCl_3$): δ7.66-7.21 (30H, m, Ph x 6), 4.98, 4.88, 4.76, 4.74 (4H, each d, *J*=10.8Hz, benzyl methylene x 2), 4.68 (1H, d, *J*=7.7Hz , H-1 ), 3.65 (1H, t, ), 3.48 (1H, t, *J*=7.7Hz, H-2), 3.41 (1H, dd, *J*=3.4Hz, H-6a), 3.41-3.18 (4H, m, -3, H-4, H-5, H-6b).

Phenyl 4-*O*-chloroacetyl-2,3-di-*O*-benzyl-1-thio-β-D-glucopyranoside (72)

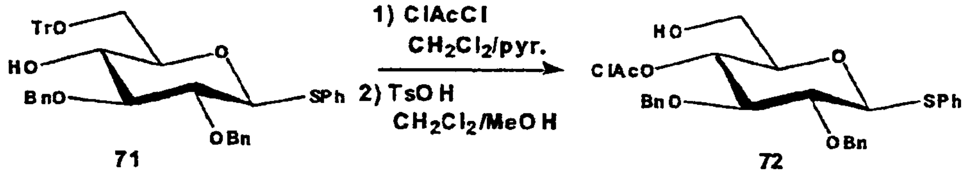


71 72

Compound 71 (550 mg, 0.97 mmol) was dissolved in $CH_2Cl_2$ (7.2 mL) and pyridine (0.4 mL), and thereafter, chloroacetyl chloride (154 mL, 1.94 mmol) was added to the solution under cooling on ice. The obtained mixture was stirred for 12 hours. Thereafter, methanol (3.0 mL) was added to the reaction solution, so as to terminate the reaction. The reaction solution was extracted with $CH_2Cl_2$, washed with HCl and $NaHCO_3$, and then dehydrated with $MgSO_4$, followed by vacuum concentration. The obtained residue was dissolved in $CH_2Cl_2$ (5.0 mL) and methanol (2.5 mL), and thereafter, TsOH (33 mg, 0.17 mmol) was added thereto. The obtained mixture was stirred at room temperature for 3 hours. After completion of the reaction, the pH of the reaction solution was returned to neutral by addition of triethylamine, and it was then subjected to vacuum concentration. The resultant was purified by silica gel column chromatography (toluene : ethyl acetate = 6:1), so as to obtain compound 72 (440 mg, 0.78 mmol) at a yield of 88% (2 steps). TLC toluene : ethyl acetate=4 : 1 Rf=0.51. $^1$H NMR ($CDCl_3$): δ7.55-7.22 (15H, m, Ph x 3), 5.00 (1H, t, *J=9.7Hz,* H-4), 4.68 (1H, d, $J_{1,2}$=8.0Hz, H-1), 4.87, 4.83, 4.75, 4.65 (4H, each d, *J*=11.2Hz, benzyl methylene x 2), 3.79-3.58 (5H, m, H-3, H-6a, H-6b, $-OCH_2Cl$), 3.53 (1H, t, H-2), 3.42 (1H, q, H-5).

Methyl(phenyl 4-*O*-chloroacetyl-2,3-di-*O*-benzyl-1-thio-β-D-glucopyranoside) uronate (73)

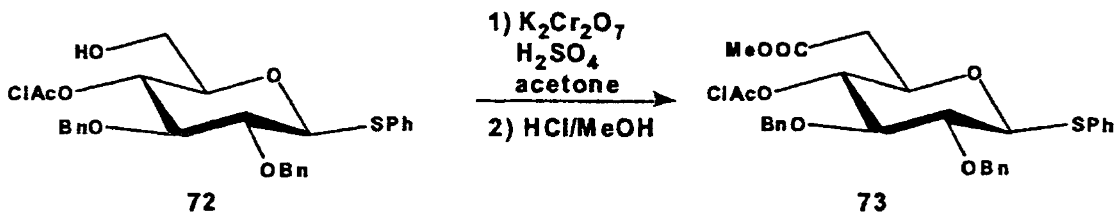


72 73

Compound 72 (390 mg, 0.74 mmol) was dissolved in acetone (10 mL), and thereafter, $K_2Cr_2O_7$ (409 mg, 1.39 mmol) and 3.5M $H_2SO_4$ (1.6 mL) were added to the solution. The obtained mixture was stirred at 55°C for 1 hour. Thereafter, the reaction solution was extracted with $CH_2Cl_2$ and then dehydrated with $MgSO_4$, followed by vacuum concentration. The residue was dissolved in HCl-methanol (20 mL), and the obtained solution was then stirred at room temperature for 2 hours. After completion of the reaction, the reaction solution was extracted with $CH_2Cl_2$, washed with $NaHCO_3$, and then dehydrated with $MgSO_4$, followed by vacuum concentration. The resultant was purified by silica gel column chromatography (toluene : ethyl acetate = 7 : 1), so as to obtain compound 73 (474 mg, 0.49 mmol) at a yield of 66%. TLC toluene : ethyl acetate=4 : 1 *Rf*=0.80. $^1$H NMR ($CDCl_3$): δ7.61-7.22 (15H, m, Ph x 3), 5.18 (1H, t, *J*=9.6Hz, H-4), 4.70 1H, d, H-1), 4.90, 4.83, 4.72, 4.64 (4H, each d, *J*=11.4Hz, benzyl methylene x 2), 3.93 (1H, d, *J*=9.8Hz, -OC$\underline{H}_2$Cl), 3.85-3.71 (3H, m, H-4, H-5, -OC$\underline{H}_2$Cl), 3.74 (3H, s, OMe), 3.68 (1H, t, *J*=9.6Hz, H-3), 3.58 (1H, t, *J*=9.7Hz, H-2).

Methyl(phenyl 2,3-di-*O*-benzyl-1-thio-β-D-glucopyranoside) uronate (74)

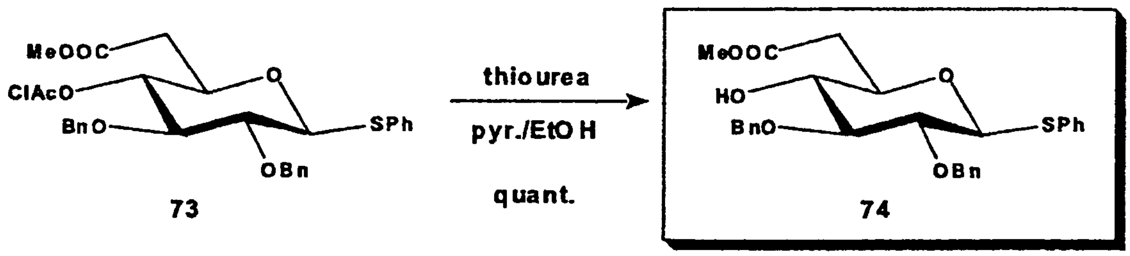


73 74

Compound 73 (500 mg, 0.92 mmol) was dissolved in pyridine (10.3 mL) and ethanol (1.7 mL), and thereafter, thiourea (70.2 mg, 9.2 mmol) was added to the solution. The obtained mixture was stirred at 80°C for 1 hour. After completion of the reaction, the reaction solution was extracted with $CH_2Cl_2$, washed with HCl and $NaHCO_3$, and then dehydrated with $MgSO_4$, followed by vacuum concentration. The resultant was purified by silica gel column chromatography (toluene : ethyl acetate = 3 : 1), so as to quantitatively obtain compound 74 (420 mg, 0.92 mmol). TLC toluene : ethyl acetate=4 : 1 *Rf*=0.58. $^1$H NMR ($CDCl_3$):δ7.58-7.24 (15H, m, Ph x 3), 4.70 (1H, d, H-1), 4.89, 4.83, 4.72, 4.66 (4H, each d, *J*=11.2Hz, benzyl methylene x 2), 3.87 (1H, H-4), 3.80 (3H, s, OMe), 3.57 (1H, t, *J=8.7Hz,* H-3), 3.51 (1H, t, *J*=9.3Hz, H-2), 3.02 (1H, d, H-5). $^{13}$C NMR ($CDCl_3$) :δ169.5 (C-6), 88.4 (C-1), 85.2 (C-3), 79.5 (C-2), 77.2 (C-5), 71.8 (C-4), 52.8 (O$\underline{C}H_3$).

**PhS-β-GlcA**

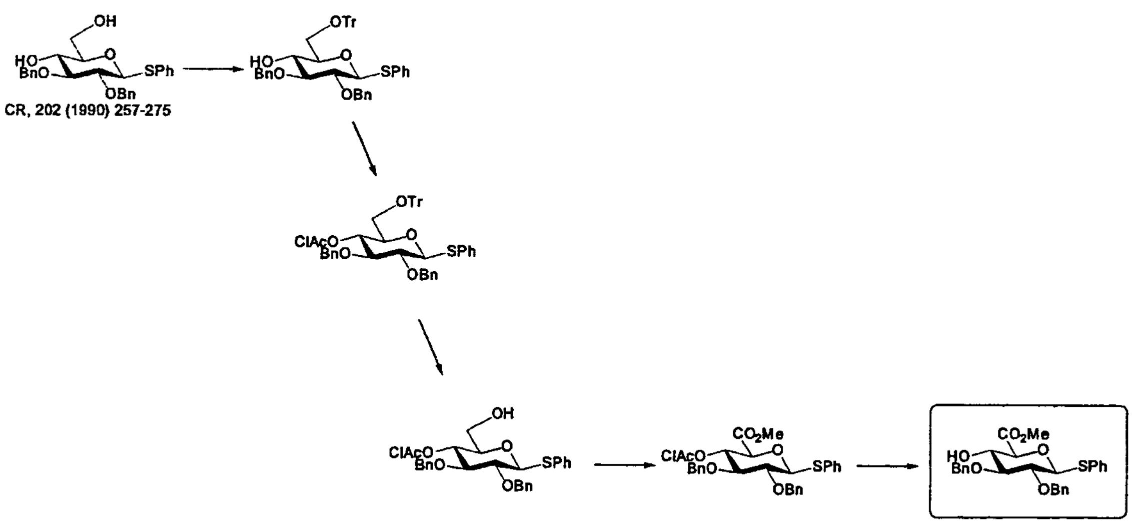

Trumtel M.; Tavecchia P.; Veyrieres A,; Sinay P. (1990) *Carbohydr. Res.* 202, 257-75.

Example 2: Diffusive mixing solid-phase reaction

The present invention is characterized in that a resin is allowed to swell with a minimum necessary amount of liquid, so as to carry out a reaction. With regard to the amount of a liquid necessary for swelling a resin, the aforementioned Table 1 or the like was referred. In addition, the amount of a solvent, which is added dropwise to a certain amount of resin that has actually been weighed, so as to decrease the friction among resin beads, so that the "liquefaction" phenomenon takes place, was confirmed and determined.

(1) Introduction of carboxylate compound into amino resin (amidation reaction)

Synthesis of mannosyl donor binding to resin by diffusive mixing solid-phase reaction method

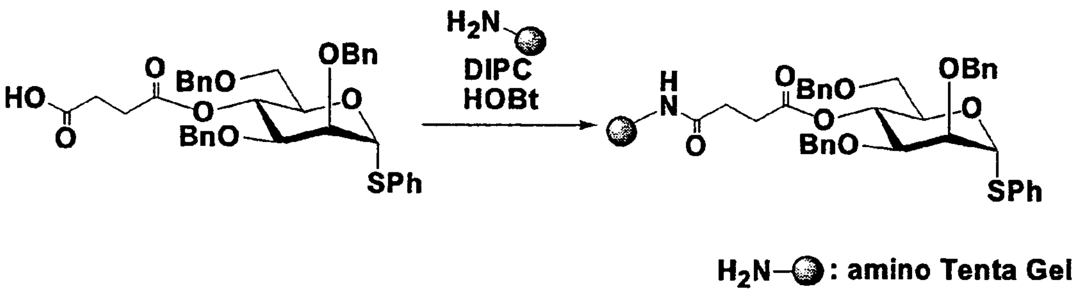


$CH_2Cl_2$ (5.0 ml), in which phenyl 2,3,6-tri-*O*-benzyl-4-*O*-succinoyl-1-thio-β-D-mannopyranoside (295 mg, 0.46 mmol), DIPC (108 μL, 0.69 mmol), and HOBt (hydroxybenzotriazole) (74.5 mg, 0.55 mmol) had been dissolved, was added to NovaSyn amino TG resin (511 mg, 15.0 μmol loading). The obtained mixture was left at room temperature for 14 hours. After completion of the reaction, the resin was washed with $CH_2Cl_2$, methanol, DMF, and $CH_2Cl_2$, and the resultant was then subjected to vacuum drying. The above process was repeated twice, so as to dry the resin under reduced pressure. Thereafter, $Ac_2O$ (3.0 mL) and pyridine (3.0 mL) were added to the resin, and the obtained mixture was then left at room temperature for 12 hours. Thereafter, the resultant was washed with $CH_2Cl_2$, methanol, and $CH_2Cl_2$, followed by vacuum drying for capping, so as to obtain a resin (530 mg). FT-IR (ATR) : $cm^{-1}$ 1739.4 (OC=O), 1671.9 (NHC=O).

The yield was determined by carrying out a cleavage reaction. The resin (49.0 mg) was cleaved out, and it was then swollen with $CH_2Cl_2$ (0.5 mL) and methanol (0.5 mL). Thereafter, 0.5M sodium methoxide-methanol (0.1 mL) was added thereto, and the obtained mixture was then left at room temperature for 1 hour. Thereafter, the resultant was neutralized with Amberlite IR 120B$^+$, and thereafter, the filtrate obtained by suction filtration was subjected to vacuum concentration, so as to obtain phenyl 1-thio-2,3,6-tri-*O*-benzyl-β-D-mannopyranoside (4.8 mg, 7.7 μmol, 62%).

Synthesis of fucosyl donor binding to resin by diffusive mixing solid-phase reaction method

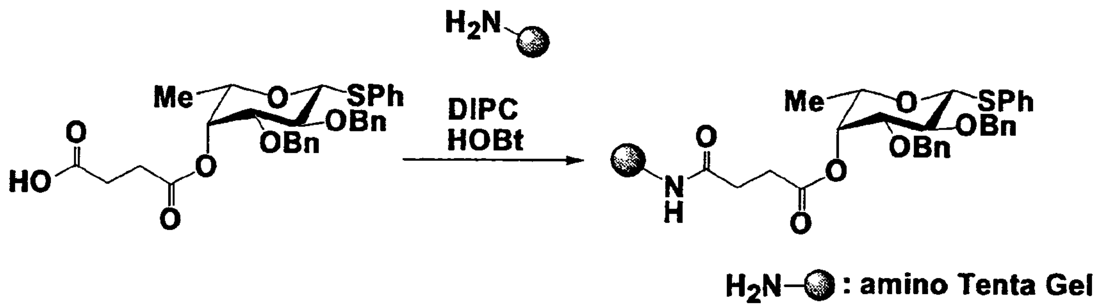


DMF (6.0 mL), in which phenyl 2,3-di-O-benzyl-4-*O*-succinoyl-1-thio-β-L-fucopyranoside (316 mg, 0.59 mmol), DIPC (138 μL, 0.88 mmol), and HOBt (96 mg, 0.71 mmol) had been dissolved, was added to NovaSyn amino TG resin (982 mg, 0.29 mmol loading), and the obtained mixture was then left at room temperature for 48 hours. After completion of the reaction, the resin was washed with $CH_2Cl_2$, methanol, and $CH_2Cl_2$, and the resultant was then subjected to vacuum drying. $Ac_2O$ (3.0 mL) and pyridine (3.0 mL) were added thereto, and the obtained mixture was left at room temperature for 12 hours. Thereafter, the resultant was washed with $CH_2Cl_2$, methanol, and $CH_2Cl_2$, followed by vacuum drying for capping, so as to obtain a resin (1.05g).
FT-IR (ATR) : $cm^{-1}$ 1739.4 (OC=O), 1671.9 (NHC=O).
The yield was determined by carrying out a cleavage reaction. The resin (30.0 mg) was swollen with $CH_2Cl_2$ (0.5 mL) and methanol (0.5 mL). Thereafter, 0.5M sodium methoxide-methanol (0.1 mL) was added thereto, and the obtained mixture was then left at room temperature for 2 hour. Thereafter, the resultant was neutralized with Amberlite IR 120B$^+$, and thereafter, the filtrate obtained by suction filtration was subjected to vacuum concentration, so as to obtain phenyl 2,3-di-O-benzyl-1-thio-β-L-fucopyranoside (3.0 mg, 6.9 μmol, 85%)

(2) Conversion reaction of thioglycoside form to fluoride form on solid-phase by diffusive mixing solid-phase reaction method

L-fucopyranosyl fluoride binding to resin

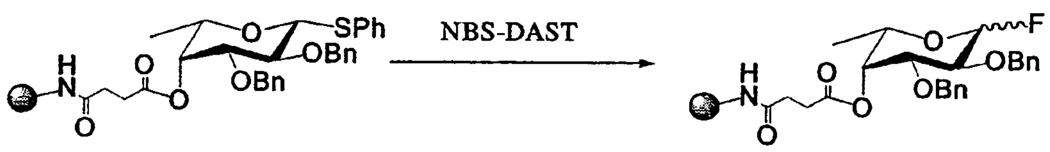


DAST (26 mL, 0.19 mmol) and NBS (35 mg, 0.19 mmol) were dissolved in $C_2H_4Cl_2$ (1.5mL). The obtained solution was added at -15°C to phenylthio-L-fucopyranoside (300 mg, 35 mg: fuc) to which a resin bound, and the obtained mixture was then left for 1 hour. Thereafter, the temperature of the reaction solution was increased to 0°C over 1 hour, and it was then left at 0°C for 8 hours. A portion of the resin was revovered, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$ , followed by vacuum drying.
FT-IR (ATR) : $cm^{-1}$ 1737.5 (OC=O), 1673.9 (NHC=O), 1041.4 (C-F).

(3) Glycosylation reaction by activation of glycosyl fluoride on solid-phase 2,3-di-*O*-benzyl-L-fucopyranosyl-(1→6)-phenyl-2,3,4-tri-*O*-benzyl-1-thio-α-D-galactop yranoside binding to resin

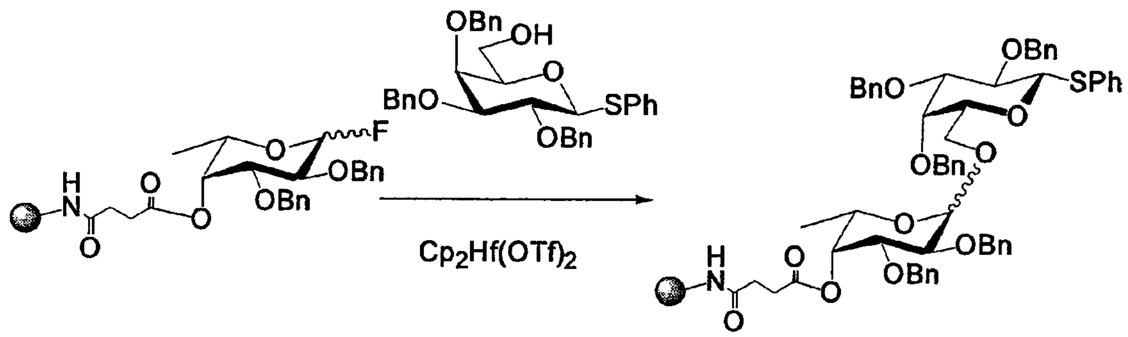


Phenyl 2,3,4-tri-*O*-benzyl-1-thio-β-D-galactopyranoside (24.0 mg, 44 μmol) was dissolved in EtCN (0.25 mL), and thereafter, MS4A (20 mg) was added to the solution. The obtained mixture was stirred in nitrogen atmosphere at room temperature for 3 hours. Separately, AgOTf (33.8 mg, 0.132 mmol) was dissolved in $CH_2Cl_2$ (0.25 mL), and thereafter, MS4A (20 mg) was added to the solution. The obtained mixture was stirred in nitrogen atmosphere at room temperature for 3 hours. Thereafter, $Cp_2HfCl_2$ (25 mg, 0.066 mmol) was added to each of the reaction solutions, and the obtained mixture was stirred for 5 minutes. The reaction solution was added at -15°C to L-fucopyranosyl fluoride (100 mg, 9.8 mg: Fuc) binding to a resin, and the reaction temperature was gradually increased to 10°C. For monitoring progress of the reaction, a portion of the resin was recovered, and it was then measured by IR. Thereafter, the portion was added to $CH_2Cl_2$ (0.5 mL) and methanol (0.5 mL), and 0.5M NaOMe (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1737.5 (OC=O), 1673.9 (NHC=O).
MALDI-TOFMS: Calcd for $C_{33}H_{36}O_9$: *m*/*z* 868.3 Found: *m*/*z* 891 $[M+Na]^+$.

The reaction was carried out while the reaction temperature was gradually changed from -15°C to 10°C, and the temperature rising rate was analyzed. The results are shown in Table 2 and Figures 6 to 8.

Table 2

| Temperature rising rate (°C/h) | Product/eliminated product |
|---|---|
| 8.3 | 15 |
| 5.6 | 100 |
| 4.2 | 100 |

As a result, it was revealed from the results obtained by the analysis of MALDI-TOFMS (Figures 6 to 8) that a little amount of eliminated product (Fw: 316) existed at 8.3°C/h, but that when the temperature rising rate was decreased, the glycosylation reaction preferentially progressed. In a glycosylation reaction by activation of glycosyl fluoride, such a temperature rising rate can be utilized.

(4) Glycosylation reaction by activation of thioglycoside on solid-phase 2,3-di-*O*-benzyl-L-fucopyranosyl-(1→6)-2,3,4-tri-*O*-benzyl-α-D-galactopyranosyl fluoride binding to resin

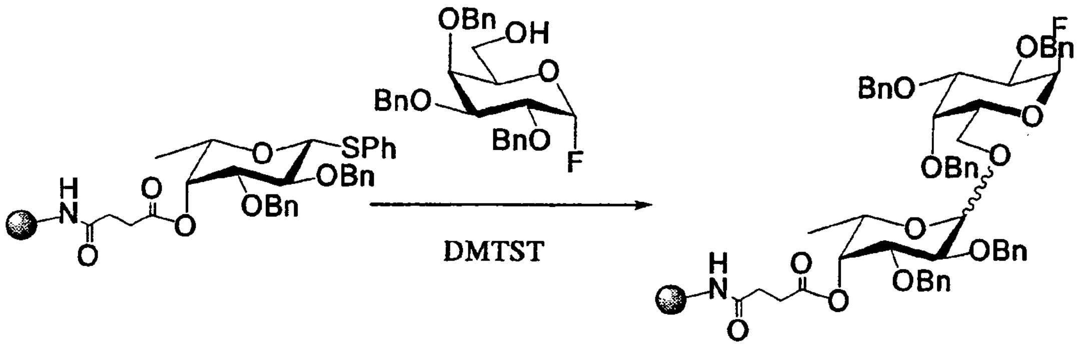


2,3,4-tri-O-benzyl-α-D-galactopyranosyl fluoride (23.0 mg, 51.6 μmol) was dissolved in $CH_2Cl_2$ (0.25 mL) and EtCN (0.25 mL), and thereafter, MS4A (20 mg) was added to the solution. The obtained mixture was stirred in nitrogen atmosphere at room temperature for 3 hours. Thereafter, MeSSMe (6.9 μL, 77.2 μmol) and MeOTf (8.7 μL, 77.2 μmol) were added at 0°C to the reaction solution, and the obtained mixture was then stirred for 10 minutes. This solution was added at -15°C to L-fucopyranoside (88 mg, 14 mg: Fuc) binding to a resin, and the obtained mixture was gradually heated up to 10°C. For monitoring the progress of the reaction, a portion of the resin was recovered, and it was then measured by IR. Thereafter, the portion was added to $CH_2Cl_2$ (0.5 mL) and methanol (0.5 mL), and 0.5M NaOMe (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1735.6 (OC=O), 1671.9 (NHC=O), 1029.8 (C-F), 636.9.
MALDI-TOFMS: Calcd for $C_{47}H_{51}FO_9$: *mlz* 778.9 Found: *mlz* 802.5 $[M+Na]^+$.

Previously, when a glycosylation reaction by activation of thioglycoside had been carried out on a solid-phase, DMTST had been added at an equivalence of 8.0 to a donor (1.0). However, when the same reaction is carried out in a liquid-phase, it is sufficient to add DMTST at an equivalence of 2.0 thereto. Thus, this time, it was assumed that the reaction progresses even when DMTST is added at an equivalence of 8.0 or less. Hence, in this example, taking into consideration the steric influence of the resin, the equivalence of DMTSM with respect to a donor was set at 3.0, and a glycosylation reaction was then analyzed. The reaction was carried out while the reaction temperature was gradually decreased from -15°C to 10°C, and the temperature rising rate was analyzed (Table 3 and Figures 9 to 11). From the results obtained by the analysis of mass spectrometry data (Figures 9 to 11), it was revealed that in the activation of thioglycoside by DMTST, even when the temperature rising rate is large, side reaction(s) do not progress, and that the amount of a generated product depends on the reaction time. When DMTST was used at an equivalence of 3.0, although the reaction time was insufficient in the present experiment, it was shown that at least thioglycoside acting as a sugar donor was activated selectively in the presence of glycosyl fluoride acting as a sugar receptor. In addition, it was clearly shown that it can be used for an orthogonal glycosylation reaction. The problem regarding reaction time can be solved by addition of a more excess amount of DMTST or by using a stronger promoter (for example, NIS-TfOH).

Table 3

| Temperature rising rate (°C/h) | Product/raw material |
|---|---|
| 5.6 | 1.1 |
| 4.2 | 2.0 |
| 2.1 | 4.4 |

(5) Order of introduction of reactants in glycosylation reaction based on diffusive mixing solid-phase reaction method

(Method 1) Method for swelling resin, to which donor has bound, with mixed solution consisting of receptor and promoter

2,3-di-*O*-benzyl-L-fucopyranosyl-(1→6)-2,3,4-tri-*O*-benzyl-α-D-galactopyranosyl fluoride binding to resin

2,3,4-tri-*O*-benzyl-α-D-galactopyranosyl fluoride (16.8 mg, 0.04 mmol) was dissolved in $C_2H_4Cl_2$ (0.2 mL) and EtCN (0.2 mL), and thereafter, MS4A (20 mg) was added to the solution. The obtained mixture was stirred in a nitrogen

atmosphere at room temperature for 3 hours. Thereafter, MeSSMe (13.4 μL, 0.15 mmol) and MeOTf (16.9 μL, 0.15 mmol) were added at 0°C to the reaction solution, and the obtained mixture was stirred for 10 minutes. This solution portion was added at 0°C to phenylthio-L-fucopyranoside (79 mg, 10 mg: Fuc) binding to a resin, and the obtained mixture was left for 12 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1735.6 (OC=O), 1671.9 (NHC=O), 1029.8 (C-F), 636.9.
MALDI-TOFMS: Calcd for $C_{47}H_{51}FO_9$: *mlz* 778.9 Found: *mlz* 802.5 $[M+Na]^+$.

The results obtained by following up this glycosylation reaction are as shown in Figure 12. When DMTST was used at an equivalence of 8, the reaction was completed for 6 hours. The yield was also satisfactory.

(Method 2) Method of swelling resin, to which donor has bound, with receptor solution and then adding promoter thereto

2,3-di-*O*-benzyl-L-fucopyranosyl-(1→6)-2,3,4-tri-*O*-benzyl-α-D-galactopyranosyl fluoride binding to resin

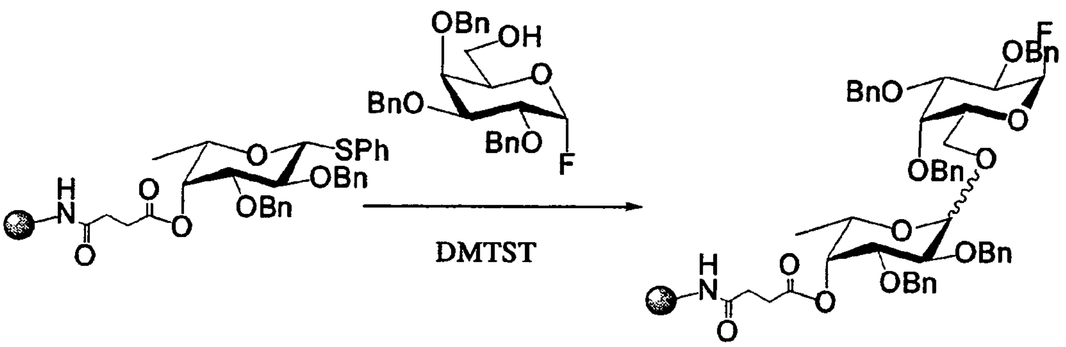


2,3,4-tri-*O*-benzyl-α-D-galactopyranosyl fluoride (77.0 mg, 0.14 mmol) was dissolved in $C_2H_4Cl_2$ (0.5 mL) and EtCN (0.5 mL), and thereafter, MS4A (200 mg) was added to the solution. The obtained mixture was stirred in nitrogen atmosphere at room temperature for 3 hours. Thereafter, phenylthio-L-fucopyranoside (340 mg, 46 mg: Fuc) obtained by binding a solution portion (supernatant) to a resin was added to a suspension that had been swollen with $CH_2Cl_2$ (0.5 mL) and EtCN (0.5 mL). Thereafter, MeSSMe (61.7 μL, 0.56 mmol) and MeOTf (77.7 μL, 0.56 mmol) were added at 0°C to the obtained solution, and the obtained mixture was left for 12 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M NaOMe (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.

The results obtained by following up this glycosylation reaction are as shown in Figure 13. The difference between this method and method 1 is only the point that a promoter was simultaneously added, or it was added separately. The reaction required 12 hours.

It can be understood that the difference in the reaction time between method 1 and method 2 corresponds to the time required for the second reactant and reactants following it to diffuse from the additive point to the entire reaction system.

From these experiment results, taking into consideration the characteristics of a solid-phase reaction progressing in a microchannel (a pore existing in the resin), it can be said that it is adequate that all reactants be added to the resin in the form of a single solution. In addition, in a case where the mixing of only reactants should be prevented, they may also be added separately.

As a result of the analysis of such two methods, it was clarified that both methods 1 and 2 are effective. Accordingly, when reactants are reacted with one another if they have previously been mixed, method 1 involving successive addition of reactants can be applied. When it is possible to mix reactants, method 2 can be applied.

(6) Comparison between the solid-phase reaction method of the present invention involving diffusion and the conventional solid-phase reaction method

In order to confirm the effectiveness of the diffusion solid-phase reaction method of the present invention, this method was compared with the conventional method using external force such as stirring, which does not involve diffusion.
For comparison purpose, all the same conditions were applied in all reaction methods, except for a concentration condition and the presence or absence of stirring. In addition, the reaction was terminated before completion of the reaction, and both cases were compared in terms of yield. In the method of the present invention (diffusion solid-phase reaction method), a solvent was used at a minimum necessary amount for the swelling of a resin, and the reaction solution was left at rest without performing stirring or the like. On the other hand, in the conventional method (solid-phase reaction method involving stirring), since if the same amount of solvent had been used, it would have been impossible to perform stirring, the amounts of the used reagents were set at identical, and the amount of a solution was set at double.

*Octyl 2,3-Di-O-benzyl-α,β-L-fucopyranoside*

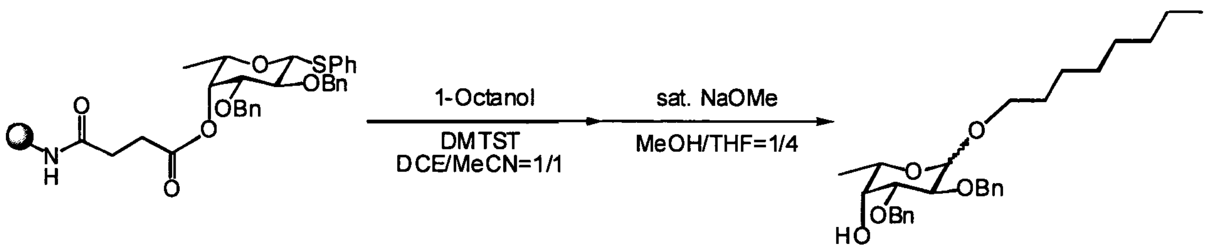


MeOTf (11.6 μL, 101 μmol) was added to MeSSMe (9.2 μL, 101 μmol) in nitrogen atmosphere, and the obtained mixture was stirred at room temperature for 5 minutes. Thereafter, DCE (300 μL) and MeCN (300 μL) were added to the reaction solution, so as to prepare a 0.169 M-DMTST stock solution. Subsequently, Acceptor: 1-Octanol (10.4 μL, 65.4 μmol) was added to this solution, so as to prepare a 0.109 M-Acceptor/0.169 M-DMTST stock solution.

[Diffusion solid-phase reaction method]

A 0.109 M-Acceptor/0.169 M-DMTST stock solution (200 μL, Acceptor: 21.8 μmol, DMTST: 33.8 μmol) was added to Resin-bound-Phenyl-1-thio-L-fucopyranoside (40.5 mg, 11.2 μmol) in nitrogen atmosphere at -30°C. Thereafter, the obtained mixture was subjected to a swelling reaction for 15 minutes. After completion of the reaction, the resin was washed with DCM, DMF, and DCM. Subsequently, the resultant resin was left in sat. NaOMe-MeOH/THF=1/4 (200 μL) at room temperature for 1 hour, so as to cleave it out of the solid-phase. The obtained resin was washed with DCM, and it was then subjected to vacuum drying. The obtained residue was observed by $^1$H NMR.

[Solid-phase reaction method involving stirring (conventional method)]

A 0.109 M-Acceptor/0.169 M-DMTST stock solution was 2 times diluted with a DCE/MeCN=1/1 solution, so as to prepare a 0.055 M-Acceptor 0.085 M-DMTST stock solution. This solution (400 μL, Acceptor: 22 μmol, DMTST: 34 μmol) was added to Resin-bound-Phenyl-1-thio-L-fucopyranoside (40.4 mg, 11.2 μmol) at -30°C in nitrogen atmosphere, and the obtained mixture was stirred for 15 minutes. The following operations were the same as those of the aforementioned diffusion solid-phase reaction method.

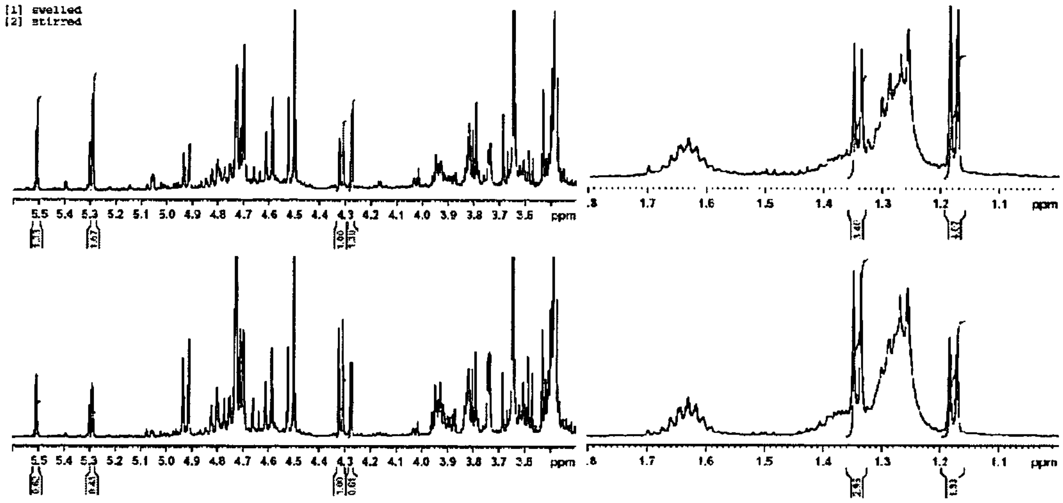


According to $^{1}H$ NMR, the doublet around 4.32 pm and 1.35 ppm was found to be a main product, octyl β-fucopyranoside, and the doublet around 4.28 ppm and 1.18 ppm was found to be a by-product, a fucose-dimer. The fucose-dimer was a hydrolysate generated due to water mixed during a post-reaction treatment. In addition, phenyl β-fucopyranoside used as a raw material completely disappeared. Thus, since products derived from raw materials are the octyl β-fucopyranoside and the fucose-dimer, the degree of progress of the reaction can be calculated based on the ratio of the octyl β-fucopyranoside as a main product to the products derived from raw materials.
The diffusion solid-phase reaction method of the present invention:

$$1.00/(1.00 + 0.61) = 62.1\%$$

The solid-phase reaction method involving stirring (conventional method):

$$1.00/(1.00 + 1.30) = 43.5\%$$

From these results, it was found that the diffusion solid-phase reaction method of the present invention is clearly more advantageous than the conventional method. That is, the results showed that the reaction rate of the method of the present invention is 143% higher than that of the conventional method. This means that when the reaction is carried out using the same amount of reagent, if the reaction is carried out with a minimum necessary amount of reagent by leaving a reaction product at rest without conducting stirring, the reaction can be extremely efficiently carried out. In addition, when the same concentration is applied, the conventional method requires 2 times higher amount of reagent than that of the method of the present invention, and thus it is not efficient. Moreover, since the method of the present invention does not need stirring, special devices are not necessary, except for a reactor used for leaving a resin as a solid-phase at rest.
From these results, it was shown that the diffusion solid-phase reaction method of the present invention is extremely efficient.

Example 3: Synthesis of sugar chain by diffusive mixing solid-phase reaction method based on orthogonal glycosylation method

(1) Synthesis of disaccharide by activation of thioglycoside

2,3-di-*O*-benzyl-L-fucopyranosyl-(1→2)-3,4,6-tri-*O*-benzyl-α-D-galactopyranosyl fluoride binding to resin

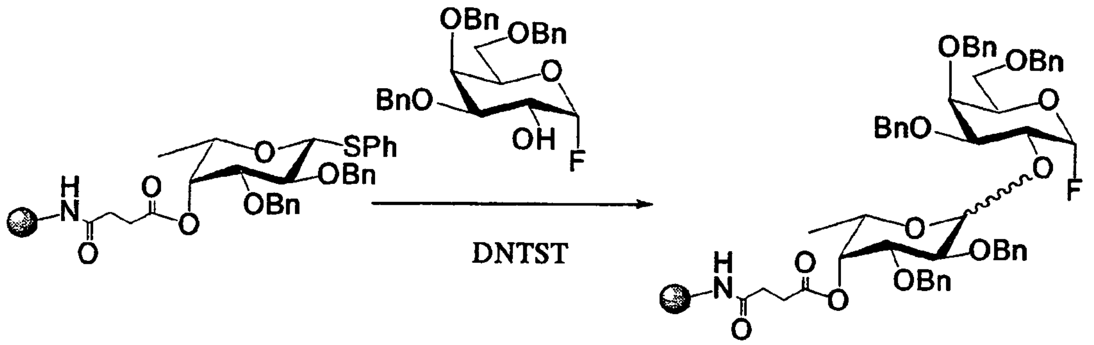


3,4,6-tri-*O*-benzyl-α-D-galactopyranosyl fluoride (35.3 mg, 0.08 mmol) was dissolved in $C_2H_4Cl_2$ (0.45 mL) and EtCN (0.45 mL), and thereafter, MS4A (20 mg) was added to the solution. The obtained mixture was stirred in nitrogen atmosphere at room temperature for 3 hours. Thereafter, MeSSMe (27.0 μL, 0.30 mmol) and MeOTf (34.0 μL, 0.30 mmol) were added at 0°C to the reaction solution, and the obtained mixture was then stirred for 10 minutes. This solution portion was added at 0°C to phenylthio-L-fucopyranoside (169 mg, 20 mg: Fuc) binding to a resin, and the obtained mixture was left for 12 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1735.6 (OC=O), 1671.9 (NHC=O), 1029.8 (C-F), 636.4.
MALDI-TOFMS: Calcd for $C_{47}H_{51}FO_9$: *m*/*z* 778.9 Found: *m*/*z* 802.5 $[M+Na]^+$.
(Figure 14)

(2) Synthesis of disaccharide by activation of thioglycoside

2,3-di-*O*-benzyl-L-fucopyranosyl-(1→3)-2,4,6-tri-*O*-benzyl-α-D-galactopyranosyl fluoride binding to resin

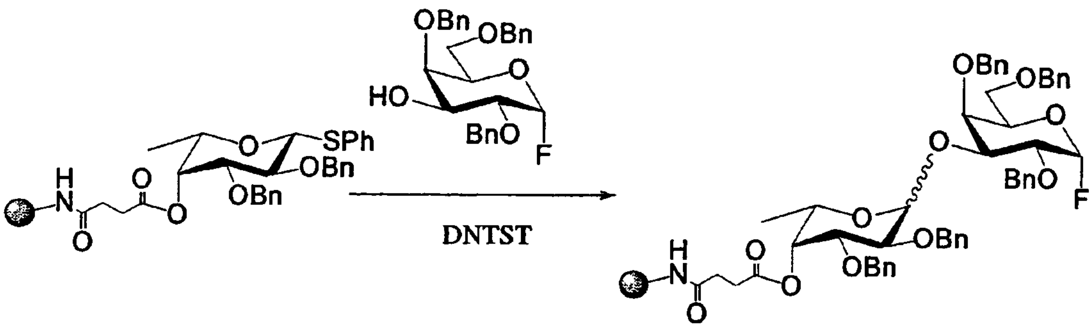


2,4,6-tri-*O*-benzyl-α-D-galactopyranosyl fluoride (35.0 mg, 0.08 mmol) was dissolved in $CH_2Cl_2$ (0.45 mL) and EtCN (0.45 mL), and thereafter, MS4A (20 mg) was added to the solution. The obtained mixture was stirred in nitrogen atmosphere at room temperature for 3 hours. Thereafter, MeSSMe (27.0 μL, 0.30 mmol) and MeOTf (34.0 μL, 0.30 mmol) were added at 0°C to the reaction solution, and the obtained mixture was then stirred for 10 minutes. This solution portion was added at 0°C to phenylthio-L-fucopyranoside (169 mg, 20 mg: Fuc) binding to a resin, and the obtained mixture was left for 12 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M NaOMe (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1735.6 (OC=O), 1671.9 (NHC=O), 1029.8 (C-F), 636.4.

MALDI-TOFMS: Calcd for $C_{47}H_{51}FO_9$: m/z 778.9 Found: *m/z* 802.5 $[M+Na]^+$. (Figure 15)

(3) Synthesis of disaccharide by activation of thioglycoside

2,3-di-*O*-benzyl-L-fucopyranosyl-(1→4)-2,3,6-tri-*O*-benzyl-α-D-galactopyranosyl fluoride binding to resin

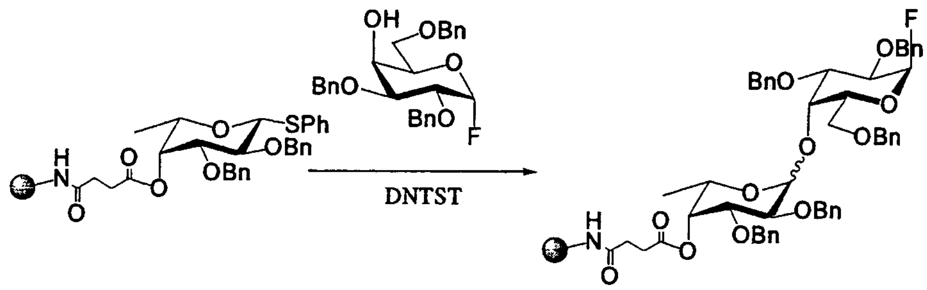


2,3,6-tri-*O*-benzyl-α-D-galactopyranosyl fluoride (34.8 mg, 0.08 mmol) was dissolved in $CH_2Cl_2$ (0.45 mL) and EtCN (0.45 mL), and thereafter, MS4A (20 mg) was added to the solution. The obtained mixture was stirred in nitrogen atmosphere at room temperature for 3 hours. Thereafter, MeSSMe (27.0 μL, 0.30 mmol) and MeOTf (34.0 μL, 0.30 mmol) were added at 0°C to the reaction solution, and the obtained mixture was then stirred for 10 minutes. This solution portion was added at 0°C to phenylthio-L-fucopyranoside (169 mg, 20 mg: Fuc) binding to a resin, and the obtained mixture was left for 12 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR (ATR) : $cm^{-1}$ 1735.6 (OC=O), 1671.9 (NHC=O), 1029.8 (C-F), 636.3.
MALDI-TOFMS: Calcd for $C_{47}H_{51}FO_9$: *m/z* 778.9 Found: *m/z* 802.5 $[M+Na]^+$.
(Figure 16)

(4) Synthesis of disaccharide by activation of thioglycoside

2,3-di-*O*-benzyl-L-fucopyranosyl-(1→6)-2,3,4-tri-*O*-benzyl-α-D-galactopyranosyl fluoride binding to resin

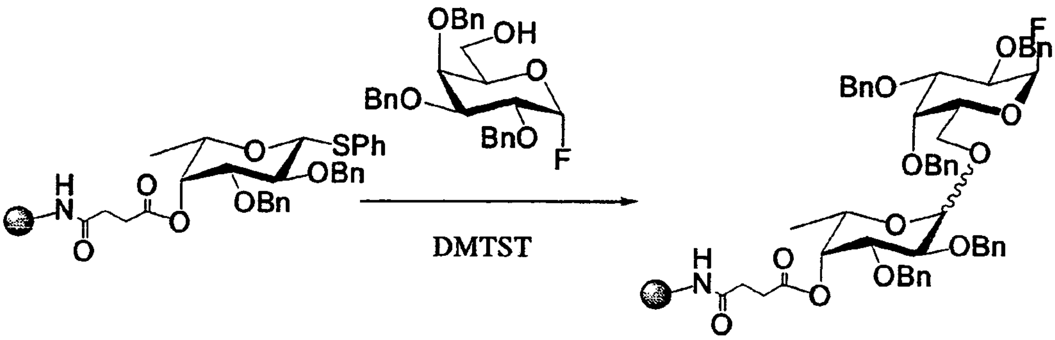


2,3,4-tri-*O*-benzyl-α-D-galactopyranosyl fluoride (34.6 mg, 0.08 mmol) was dissolved in $CH_2Cl_2$ (0.45 mL) and EtCN (0.45 mL), and thereafter, MS4A (20 mg) was added to the solution. The obtained mixture was stirred in nitrogen atmosphere at room temperature for 3 hours. Thereafter, MeSSMe (27.0 μL, 0.30 mmol) and MeOTf (34.0 μL, 0.30 mmol) were added at 0°C to the reaction solution, and the obtained mixture was then stirred for 10 minutes. This solution

portion was added at 0°C to phenylthio-L-fucopyranoside (169 mg, 20 mg: Fuc) binding to a resin, and the obtained mixture was then left for 12 hours. For monitoring the progress of the reaction, a portion of the resin was cleaved out, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.
FT-IR(ATR): $cm^{-1}$ 1735.6 (OC=O), 1671.9 (NHC=O), 1029.8 (C-F), 636.9.
MALDI-TOFMS: Calcd for $C_{47}H_{51}FO_9$: *m/z* 778.9 Found: *m/z* 802.5 $[M+Na]^+$.
(Figure 17)

(5) Synthesis of trisaccharide derivative on solid-phase

Phenyl 2,3,6-tri-O-benzyl-1-thio-β-D-glucopyranoside (41.0 mg, 0.074 mmol) was dissolved in EtCN (0.45 mL), and thereafter, MS4A (20 mg) was added to the solution. The obtained mixture was stirred under nitrogen stream at room temperature for 3 hours (solution A). Separately, AgOTf (57.0 mg, 0.222 mmol) and MS4A (20 mg) were added to $CH_2Cl_2$ (0.45 mL), and the obtained mixture was stirred under nitrogen stream at room temperature for 3 hours. Thereafter, $Cp_2HfCl_2$ (42.0 mg, 0.111 mmol) was added at 0°C to the reaction solution, and the obtained mixture was stirred for 5 minutes (solution B). The thus prepared solutions A and B were mixed with each other. The obtained mixed solution was added to disaccharide (169 mg, 0.037 mmol) binding to a resin under nitrogen stream at -15°C, and the temperature of the obtained mixture was increased to 10°C over 6 hours. Thereafter, the mixture was left for 3 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M sodium methoxide (10 μL) was then added dropwise to the mixture. The obtained mixture was left at room temperature for 1 hour, so as to cleave it out of the solid-phase. It was then observed by MALDI-TOFMS. After completion of the reaction, the resin was washed with $CH_2Cl_2$, EtCN, and $CH_2Cl_2$, followed by vacuum drying.

The same operations were performed on the previously synthesized 4 types of compounds. The schemes and the charts of MALDI-TOFMS thereof are shown below and in Figures 18 to 21.

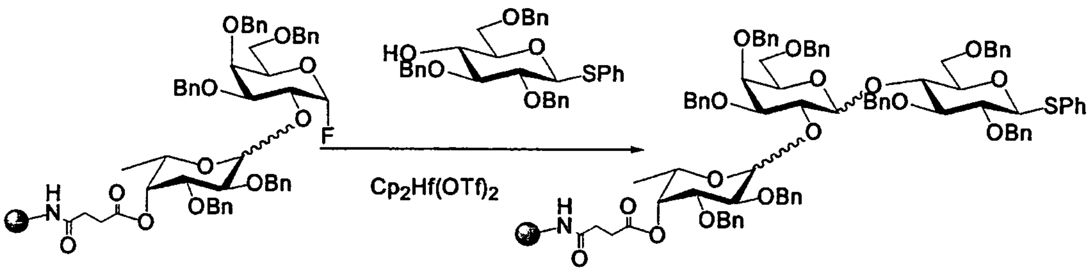


MALDI-TOFMS: Calcd for $C_{80}H_{84}O_{14}S$: *m/z* 1301.6 Found: *m/z* 1325.6 $[M+Na]^+$. (Figure 18)

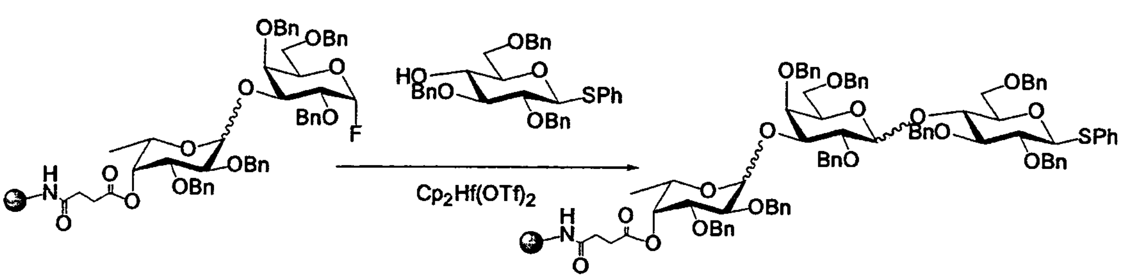


MALDI-TOFMS: Calcd for $C_{80}H_{84}O_{14}S$: *m/z* 1301.6 Found: *m/z* 1325.6 $[M+Na]^+$. (Figure 19)

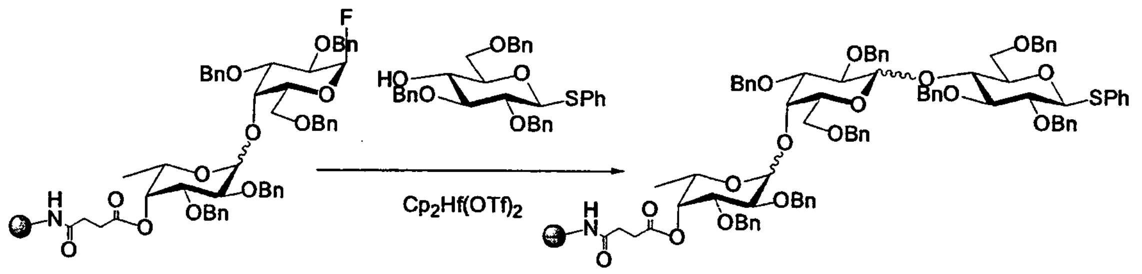


MALDI-TOFMS: Calcd for $C_{80}H_{84}O_{14}S$: *m*/*z* 1301.6 Found: *m*/*z* 1326.3 $[M+Na]^+$. (Figure 20)

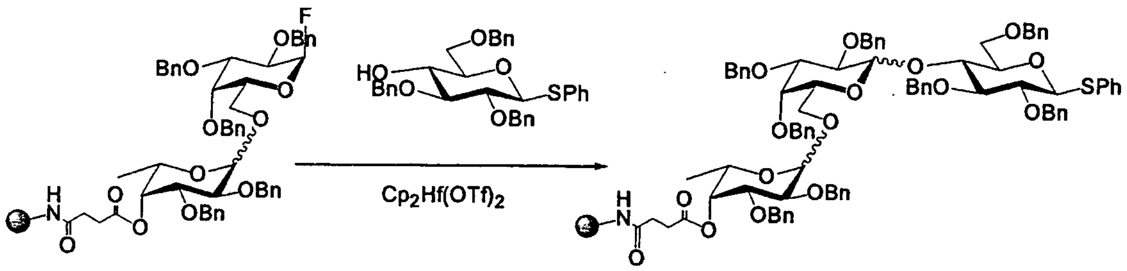


MALDI-TOFMS: Calcd for $C_{80}H_{84}O_{14}S$: *m*/*z* 1301.6 Found: *m*/*z* 1325.6 $[M+Na]^+$. (Figure 21)

In the charts of MALDE-TOEMS in Figures 18 to 21, since a pseudo parent peak derived from a compound of interest was confirmed in each of the 4 types of reactions, it was clear that a glycosylation reaction was promoted. When a reaction temperature gradient is kept constant and the finally reached temperature is set at 10°C higher than as usual, the reaction can be completed.

1-octanol (92.8 μL, 0.30 mmol) was dissolved in $CH_2Cl_2$ (0.45 mL) and EtCN (0.45 mL), and thereafter, MS4A (20 mg) was added to the solution. The obtained mixture was stirred under nitrogen stream at room temperature for 3 hours. Thereafter, MeSSMe (26.6 μL, 0.30 mmol) and MeOTf (33.5 μL, 0.30 mmol) were added at 0°C to the reaction solution, and the obtained mixture was then stirred for 10 minutes. This solution was added at -15°C to trisaccharide (169 mg, 20 mg: Fuc) binding to a resin, and the temperature of the obtained mixture was increased to 20°C over 6.5 hours. Thereafter, the mixture was left for 8 hours. For monitoring the progress of the reaction, a portion of the resin was recovered, and it was then measured by IR. Thereafter, the portion was added to $CH_2Cl_2$ (0.5 mL) and methanol (0.5 mL), and 0.5M NaOMe (10 μL) was then added dropwise to the mixture. The obtained mixture was left at room temperature for 1 hour, so as to cleave it out of the solid-phase. It was then observed by TLC and MALDI-TOFMS. After completion of the reaction, the resin was washed with $CH_2Cl_2$, methanol, and $CH_2Cl_2$, followed by vacuum drying.

The same operations were performed on the previously synthesized 4 types of trisaccharide derivatives. The schemes and the charts of MALDI-TOFMS thereof are shown below.

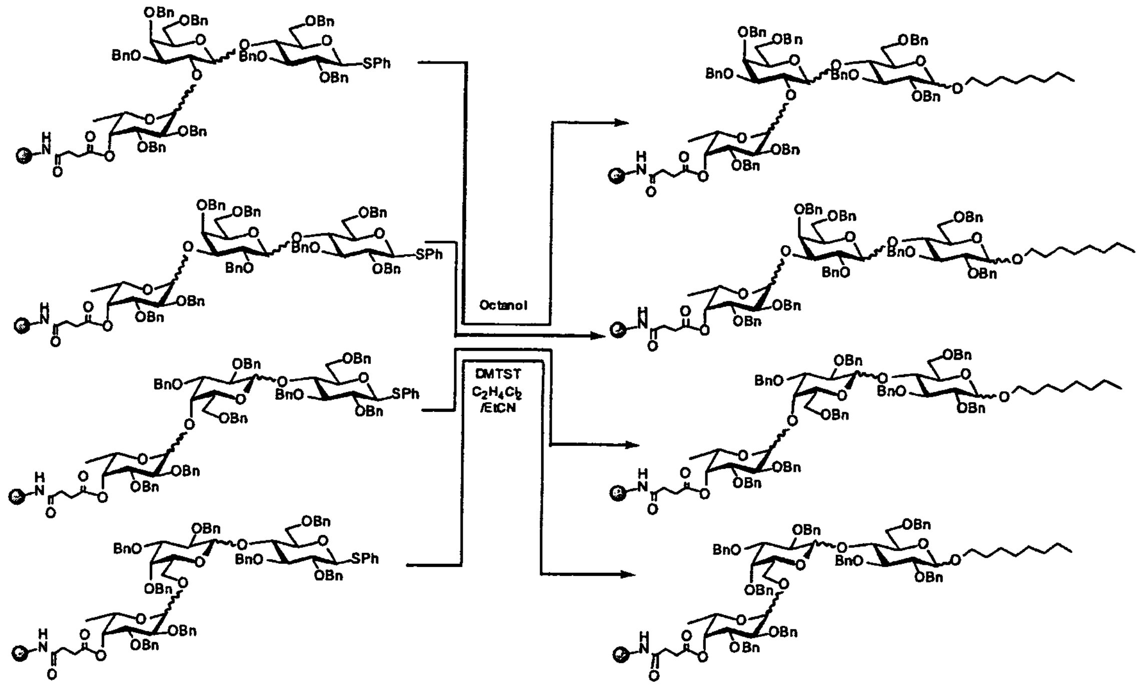


MALDI-TOFMS: Calcd for $C_{82}H_{95}O_{15}$: m/z 1320.7 Found: m/z 1343.8 $[M+Na]^+$.
(Figure 22)
MALDI-TOFMS: Calcd for $C_{82}H_{95}O_{15}$: m/z 1320.7 Found: m/z 1343.8 $[M+Na]^+$.
(Figure 23)
MALDI-TOFMS: Calcd for $C_{82}H_{95}O_{15}$: m/z 1320.7 Found: m/z 1343.8 $[M+Na]^+$.
(Figure 24)
MALDI-TOFMS: Calcd for $C_{82}H_{95}O_{15}$: m/z 1320.7 Found: m/z 1343.8 $[M+Na]^+$.
(Figure 25)

In the charts of MALDE-TOEMS in Figures 22 to 25, since a peak (FW: 1301) derived from a compound of interest was confirmed in each of the 4 types of reactions, it was clear that a glycosylation reaction was promoted. However, since many peaks that seem to be by-products are observed around 1367, 912, 694, and 480, further optimization of conditions is required.

Example 4: Synthesis of sugar chain library by diffusive mixing solid-phase reaction method based on orthogonal glycosylation method

Taking into consideration the results in Examples 1 to 3, it is possible to synthesize a sugar chain library.

That is to say, the 9 types of main monosaccharides (Example 1), which are suitably protected and constitute a living body, are essential for achieving the chemical synthesis of a sugar chain library. Moreover, steric control in glycosylation reactions for the synthesis of a sugar chain is an extremely difficult problem, and thus it is impossible to control all glycosylation reactions in the synthesis of such a library. Accordingly, stereo-nonselective methodology is suitable for the construction of a sugar chain library. Thus, the position 2 of a protected monosaccharide has preferably a substituent that is not involved in groups adjacent thereto. In the present example, the thus designed protected monosaccharide is synthesized in the form of a stable thioglycoside, and thereafter, it can be converted to glycosyl fluoride, as necessary.

Such monosaccharide derivatives are used, and they are allowed to react in any given combination, so as to synthesize a sugar chain library. In order to synthesize such a library, a suitable solid-phase synthesis method is required. The solid-phase synthesis method utilizing the diffusive mixing solid-phase reaction method of the present invention is one of such methods (Example 2). According to the present method, a reactant is dissolved in a solvent with a minimum necessary amount for swelling a resin as a solid-phase reaction site, and the resin is then swollen with the thus obtained solution, so as to rapidly introduce the reactant into a pore as a reaction site. In addition, since the reaction progresses based on diffusion, this method does not need devices used for physical means such as stirring.

The orthogonal glycosylation method in a solid-phase is a method for achieving the solid-phase synthesis of a sugar chain in a shortest process. In Example 3, a solid-phase reaction method by diffusion, the effectiveness of which had been demonstrated in the aforementioned Example 2, was used to carry out such an orthogonal glycosylation reaction, thereby obtaining favorable results.

In particular, in Examples 2 and 3, the diffusive mixing solid-phase reaction method and the solid-phase orthogonal glycosylation method were not only demonstrated, but also the library of (α/β)Fuc(1 -2/3/4/6)(α/β)Gal(1-4)(α/β)Glc-Octyl (2 x 4 x 2 x 2 = 32) was synthesized. From these results, it was demonstrated that according to the method of the present invention, a larger library can be synthesized.

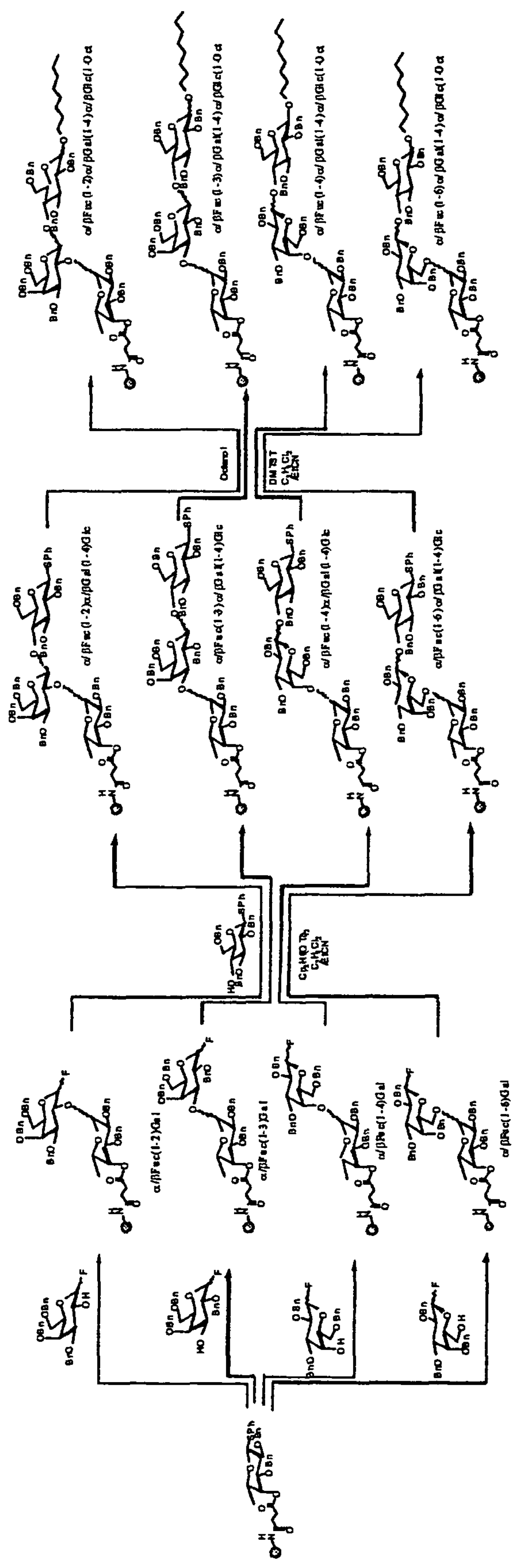

INDUSTRIAL APPLICABILITY

The present invention provides a method for efficiently chemically synthesizing biomolecules including a nucleotide, a peptide, and a sugar chain as representative examples. The method of solid-phase synthesis of sugar chain (s) of the present invention that is able to efficiently carry out the solid-phase synthesis of sugar chain(s) by controlling the temperature rising rate, thereby changing the temperature in a reaction system, and a diffusion solid-phase reaction method for efficiently carrying out a solid-phase reaction with a small amount of solvent, are both useful for the aforementioned chemical synthesis of biomolecules. These methods are particularly useful for combinatorial chemistry or the like. Moreover, the methods are also particularly useful for the synthesis of sugar chain(s), regarding which it has been conventionally difficult to control the synthesis and there have been no commercially available synthesizers.

The present application claims priority from Japanese Patent Application No. 2003-187878, filed on June 30, 2003; the disclosure of which is hereby incorporated by reference. In addition, all publications cited herein are also incorporated herein by reference in their entirety.

## Claims

**1.** A method of solid-phase synthesis of sugar chain(s) for synthesizing multiple types of sugar chains in at least one sugar chain synthesis reaction system comprising multiple types of monosaccharide units, which is **characterized in that** it comprises changing the temperature in the sugar chain synthesis reaction system depending on the temperature rising rate that has been determined based on a decrease in side reaction(s) in the reaction system as an indicator.

**2.** The method of solid-phase synthesis of sugar chain(s) according to claim 1, which comprises the following steps:

(a) a step of allowing a monosaccharide unit represented by the following formula (2):

$$HO\text{-}A^2\text{-}Y \qquad (2)$$

(wherein $A^2$ represents a monosaccharide skeleton wherein a hydroxyl group that is not involved in the reaction is protected by a protecting group; and Y represents a leaving group that is stable under conditions for activating a leaving group X),
to react with a monosaccharide unit represented by the following formula (1):

$$P\text{-}L\text{-}O\text{-}A^1\text{-}X \qquad (1)$$

(wherein P represents a solid-phase; L represents a linker, O represents an oxygen atom at the nonreducing terminus of the monosaccharide; $A^1$ represents a monosaccharide skeleton wherein a hydroxyl group that is not involved in the reaction is protected by a protecting group; and X represents a leaving group that is stable under conditions for activating the leaving group Y) under conditions for activating the leaving group X, so as to obtain sugars represented by the following formula (3):

$$P\text{-}L\text{-}O\text{-}A^1\text{-}O\text{-}A^2\text{-}Y \qquad (3);$$

and
(b) a step of allowing a monosaccharide unit represented by the following formula (4):

$$HO\text{-}A^3\text{-}X' \qquad (4)$$

(wherein $A^3$ represents a monosaccharide skeleton wherein a hydroxyl group that is not involved in the reaction is protected by a protecting group; X' represents a leaving group that is stable under conditions for activating the leaving group Y or a protecting group of a hydroxyl group at position 1),
to react with the sugars represented by formula (3) obtained in the above-described step (a) under conditions for activating the leaving group Y, so as to obtain sugars represented by the following formula (5):

$$P\text{-}L\text{-}O\text{-}A^1\text{-}O\text{-}A^2\text{-}O\text{-}A^3\text{-}X' \qquad (5).$$

**3.** The method of solid-phase synthesis of sugar chain(s) according to claim 2, wherein, in step (b), the monosaccharide

unit represented by formula (4), wherein X' represents a protecting group of a hydroxyl group at position 1, is used to synthesize a sugar chain wherein 3 sugars are connected with one another.

**4.** The method of solid-phase synthesis of sugar chain(s) according to claim 2, which comprises repeatedly performing steps (a) and (b) on the sugars represented by formula (5) obtained in step (b).

**5.** The method of solid-phase synthesis of sugar chain(s) according to any one of claims 2 to 4, wherein the monosaccharide units represented by formulas (1), (2), and (4) are allowed to simultaneously react in a single reaction system, so as to carry out the reaction.

**6.** The method of solid-phase synthesis of sugar chain(s) according to any one of claims 2 to 5, wherein the leaving group X is one of a phenylthio group or a fluorine group, and the leaving group Y is the other of a phenylthio group or a fluorine group.

**7.** The method of solid-phase synthesis of sugar chain(s) according to claim 6, wherein the phenylthio group is activated with N-iodosuccinimide-trifluoromethanesulfonic acid (NIS-TfOH) or dimethyl(methylthio)sulfonium triflate (DMTST), and wherein the fluorine group is activated with $Sn(ClO_4)_2$, $Sn(OTf)_2$, $C_{p2}Hf(ClO_4)_2$, or $Cp_2Hf(OTf)_2$.

**8.** The method of solid-phase synthesis of sugar chain(s) according to any one of claims 2 to 7, wherein the protecting group is a benzyl group.

**9.** The method of solid-phase synthesis of sugar chain(s) according to any one of claims 2 to 8, wherein monosaccharide skeletons represented by $A^1$, $A^2$, and $A^3$ are the monosaccharide skeletons of 3 types of sugars selected from among monosaccharides existing in a living body.

**10.** The method of solid-phase synthesis of sugar chain(s) according to any one of claims 2 to 9, wherein monosaccharide skeletons represented by $A^1$, $A^2$, and $A^3$ are the monosaccharide skeletons of mannose, glucose, galactose, xylitose, glucosamine, galactosamine, glucuronic acid, fructose, or sialic acid.

**11.** A monosaccharide library used for carring out the method of solid-phase synthesis of sugar chain(s) according to any one of claims 1 to 10, which comprises at least one monosaccharide derivative selected from the group consisting of
phenyl 1-thio-3,4,6-tri-O-benzyl-β-D-mannopyranoside,
phenyl 1-thio-2,4,6-tri-O-benzyl-β-D-mannopyranoside,
phenyl 1-thio-2,3,6-tri-O-benzyl-β-D-mannopyranoside,
phenyl 1-thio-2,3,4-tri-O-benzyl-β-D-mannopyranoside,
phenyl 1-thio-3,4,6-tri-O-benzyl-β-D-galactopyranoside,
phenyl 1-thio-2,4,6-tri-O-benzyl-β-D-galactopyranoside,
phenyl 1-thio-2,3,6-tri-O-benzyl-β-D-galactopyranoside,
phenyl 1-thio-2,3,4-tri-O-benzyl-β-D-galactopyranoside,
phenyl 2,3-di-O-benzyl-1-thio-β-D-xylopyranoside,
phenyl 3,4-di-O-benzyl-1-thio-β-D-xylopyranoside,
phenyl 2,4-di-O-benzyl-1-thio-β-D-xylopyranoside,
phenyl 1-thio-2,3-di-O-benzyl-β-L-fucopyranoside,
phenyl 2-azido-4,6-di-O-benzyl-2-deoxy-1-thio-β-D-galactopyranoside,
phenyl 2-azido-3,6-di-O-benzyl-2-deoxy-1-thio-β-D-galactopyranoside,
phenyl 2-azido-3,4-di-O-benzyl-2-deoxy-1-thio-β-D-galactopyranoside,
phenyl 3,4,6-tri-O-benzyl-1-thio-β-D-glucopyranoside,
phenyl 2,4,6-tri-O-benzyl-1-thio-β-D-glucopyranoside,
phenyl 2,3,6-tri-O-benzyl-1-thio-β-D-glucopyranoside,
phenyl 2,3,4-tri-O-benzyl-1-thio-β-D-glucopyranoside,
phenyl 2-azido-4,6-di-O-benzyl-2-deoxy-1-thio-β-D-glucopyranoside,
phenyl 2-azido-3,4-di-O-benzyl-2-deoxy-1-thio-β-D-glucopyranoside,
phenyl 2-azido-3,6-di-O-benzyl-2-deoxy-1-thio-β-D-glucopyranoside,
methyl(phenyl 1-thio-2,3-di-O-benzyl-β-D-glucopyranoside)uronate,
methyl(phenyl 5-acetamide-4,7,8,9-tetra-O-acetyl-3,5-dideoxy-2-thio -β-D-glycero-D-galacto-2-nonulopyranoside) onate,
methyl(phenyl 5-acetamide-4,7,8,9-tetra-O-acetyl-3,5-dideoxy-2-thio -α-D-glycero-D-galacto-2-nonulopyranoside)onate,
methyl(phenyl 5-acetamide-3,5-dideoxy-2-thio-β-D-glycero-D-galacto -2-nonulopyranoside)onate,
methyl(phenyl 5-acetamide-3,5-dideoxy-2-thio-α-D-glycero-D-galacto -2-nonulopyranoside)onate,
and compounds obtained by substituting a phenylthio group with a fluorine group in the aforementioned compounds.

**12.** A method for carrying out a solid-phase reaction in a pore of a solid material with a size necessary for capillary phenomenon, which is **characterized in that** the reaction is carried out in a state where an excess amount of liquid-phase does not exist on the outer surface of the solid material.

**13.** The method for carrying out a solid-phase reaction according to claim 12, which is **characterized in that** the reaction is carried out by diffusive mixing.

**14.** The method for carrying out a solid-phase reaction according to claim 12 or 13, wherein the solid material is a resin particle having a pore therein.

**15.** The method for carrying out a solid-phase reaction according to any one of claims 12 to 14, wherein the solid-phase reaction is a chemical reaction or a biochemical reaction.

**16.** The method for carrying out a solid-phase reaction according to any one of claims 12 to 15, wherein the solid-phase reaction is a sugar chain synthesis reaction.

**17.** The method for carrying out a solid-phase reaction according to any one of claims 12 to 16, wherein the solid-phase reaction is the solid-phase synthesis reaction of sugar chain(s) according to any one of claims 1 to 10.

**18.** A library, which is composed of sugar chains consisting of all combinations of 3 types of sugars selected from the monosaccharides existing in a living body, which are synthesized by the method of solid-phase synthesis of sugar chain(s) according to any one of claims 1 to 10 or the solid-phase reaction method according to claim 17.

**19.** The library according to claim 18, wherein the monosaccharides existing in a living body are mannose, glucose, galactose, xylitose, glucosamine, galactosamine, glucuronic acid, fructose, or sialic acid.

**20.** A reaction device used for carrying out the solid-phase reaction method according to any one of claims 12 to 17, which comprises at least the following (1) and (2):

(1) a reaction unit for carrying out a solid-phase reaction, which has an introduction unit for injecting or discharging a liquid-phase and a space for accommodating a solid material; and
(2) a temperature-controlling unit for controlling the temperature of the reaction unit.

FIG. 1

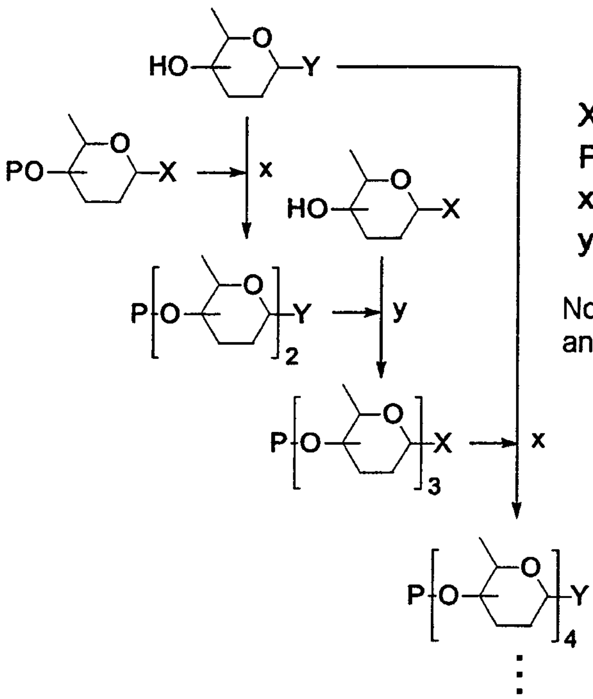


**X,Y:** leaving group
**P:** protecting group
**x:** activating condition to X
**y:** activating condition to Y

Note: Y is stable under condition x, and X is stable under condition y.

FIG. 2

| | 2-OH | 3-OH | 4-OH | 6-OH |
|---|---|---|---|---|
| Man | | | | |
| Glc | | | | |
| Gal | | | | |
| Xyl | | | | |
| GlcN | | | | |
| GalN | | | | |

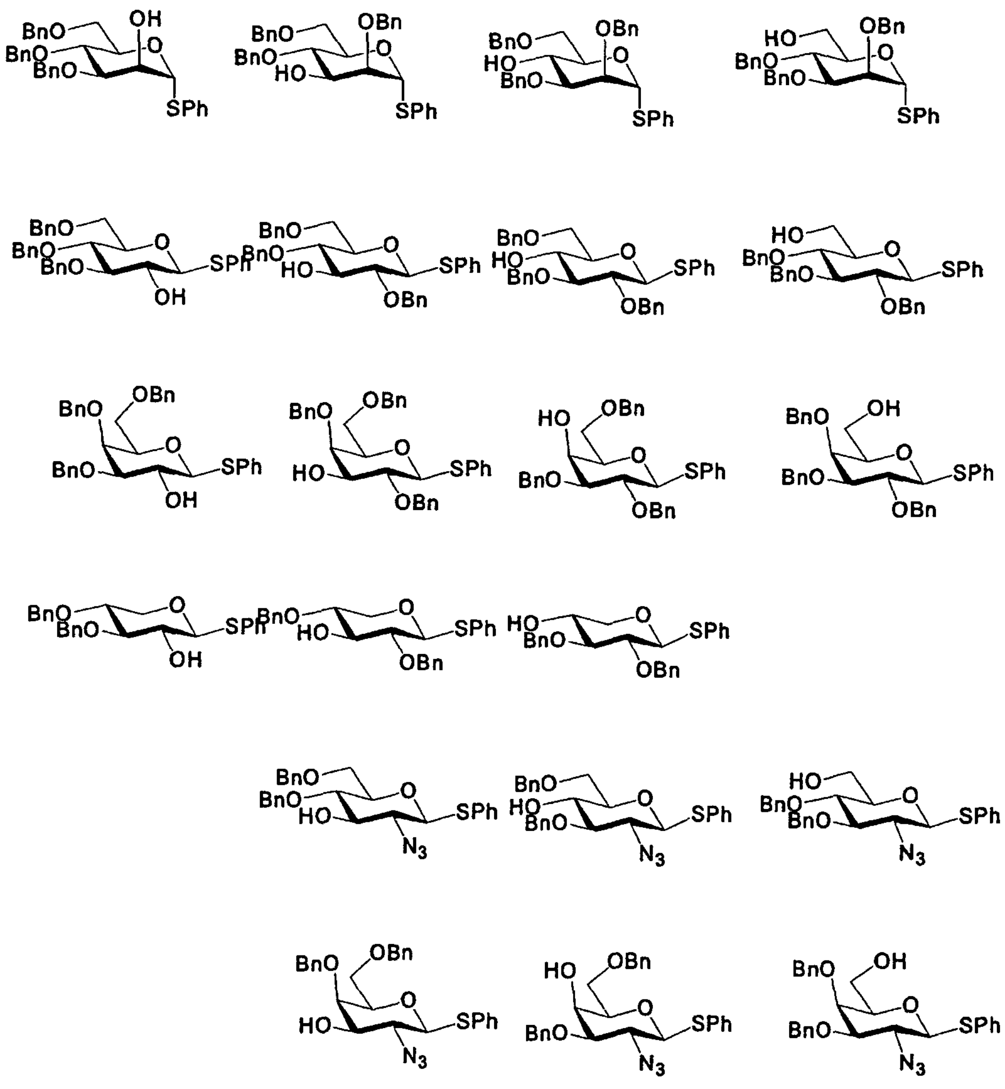

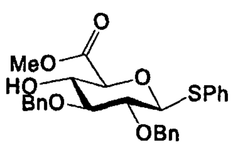

GlcA

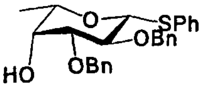

Fuc

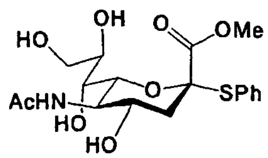

Sia

FIG. 3

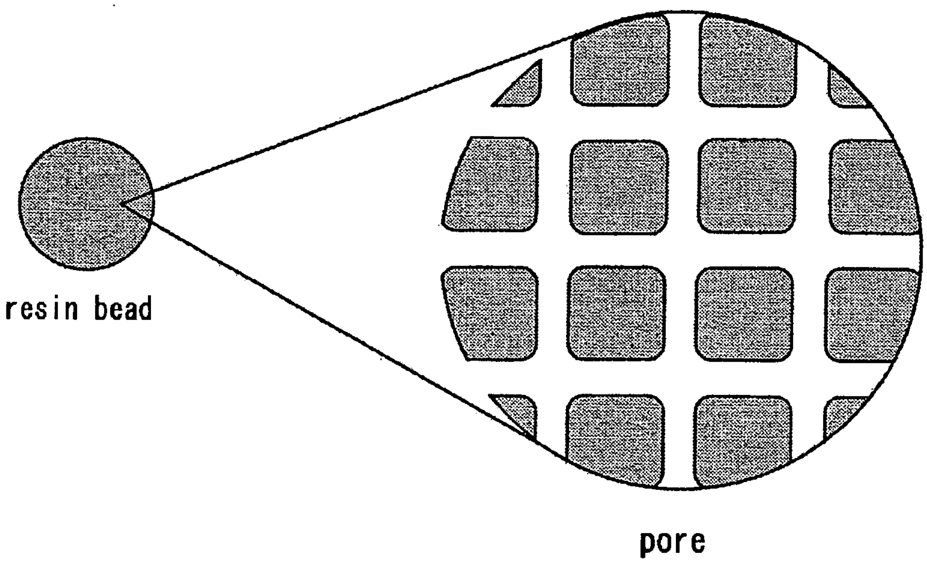
resin bead
pore

FIG. 4

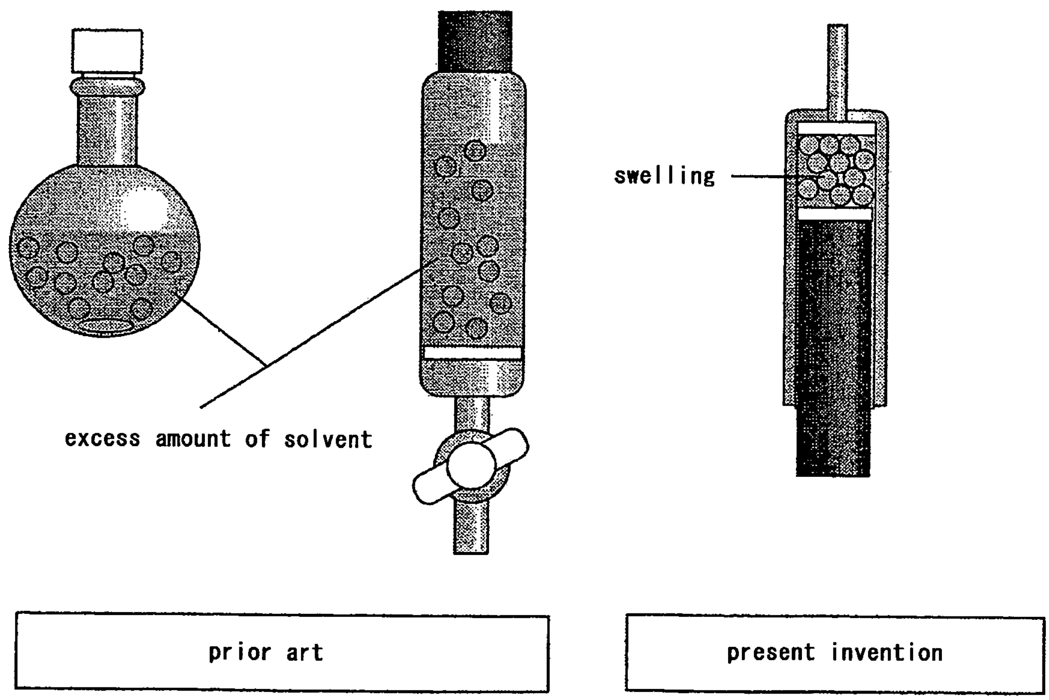
excess amount of solvent
swelling
prior art
present invention

FIG. 5

# sequence, position, stereoisomerism

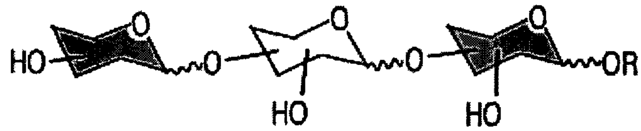

III

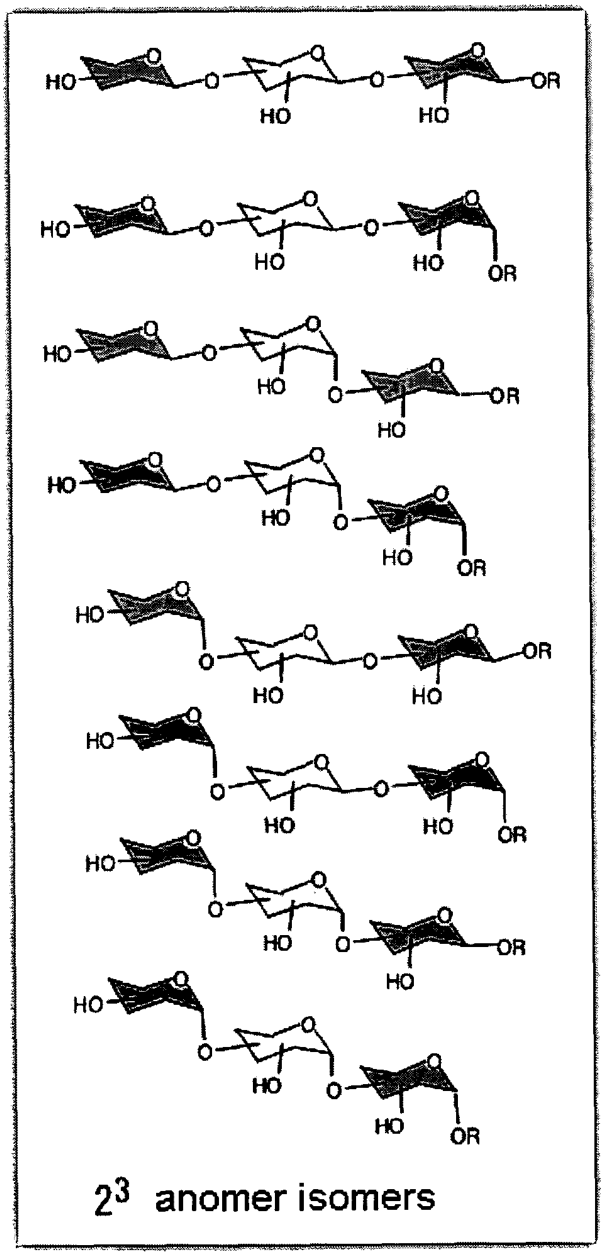

$2^3$ anomer isomers

FIG. 6

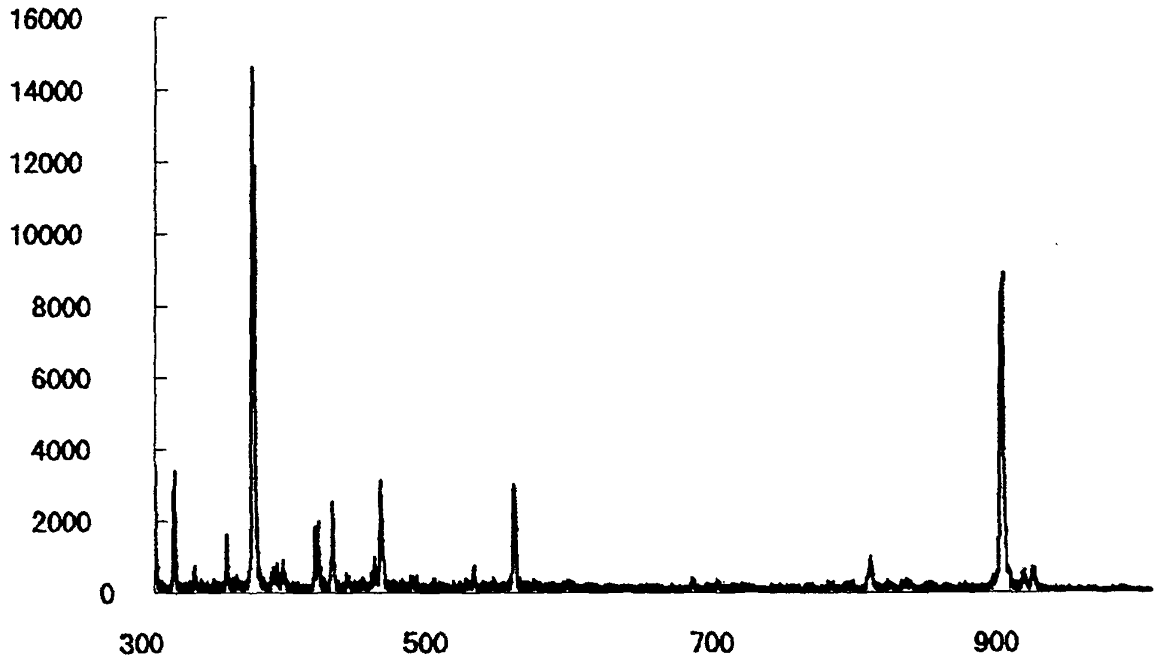
16000
14000
12000
10000
8000
6000
4000
2000
0
300
500
700
900

FIG. 7

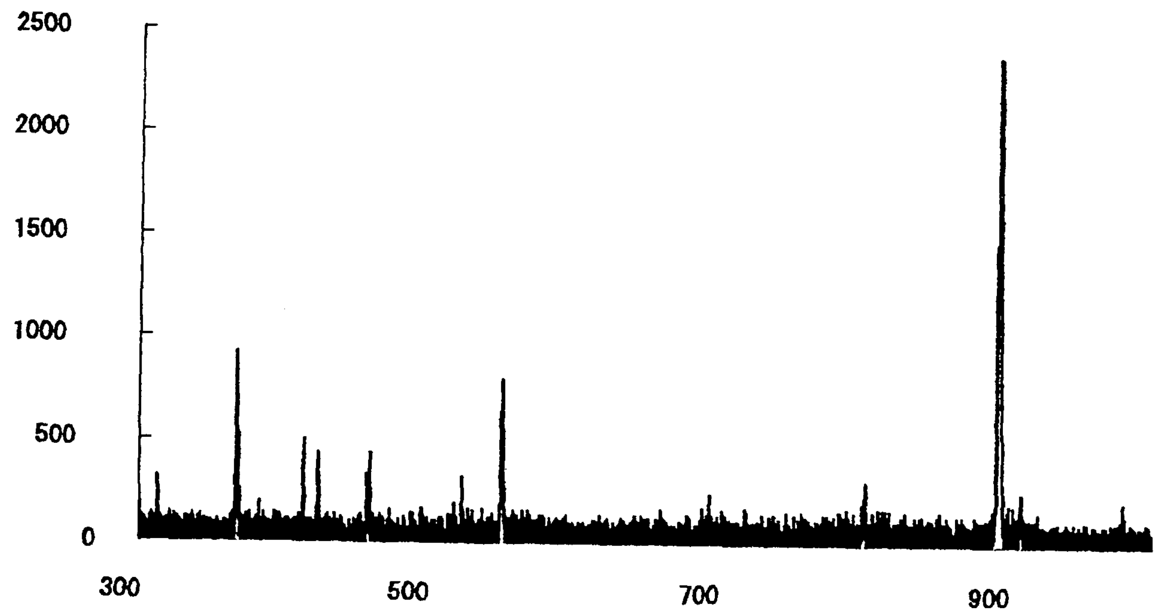
2500
2000
1500
1000
500
0
300
500
700
900

FIG. 8

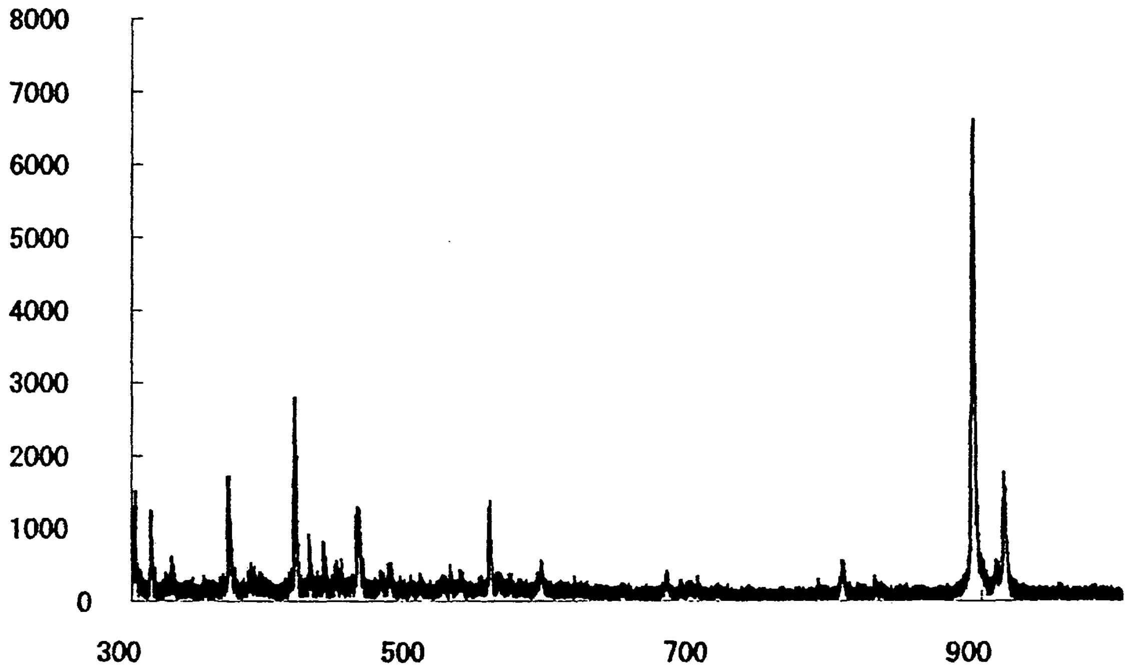
8000
7000
6000
5000
4000
3000
2000
1000
0
300
500
700
900

FIG. 9

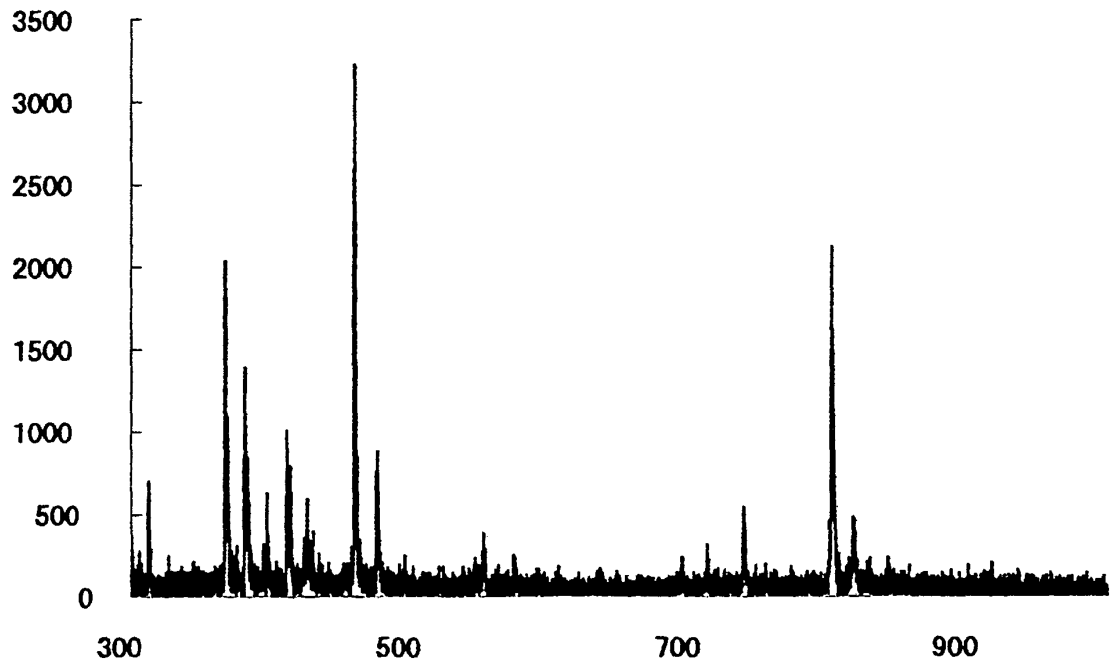
3500
3000
2500
2000
1500
1000
500
0
300
500
700
900

FIG. 10

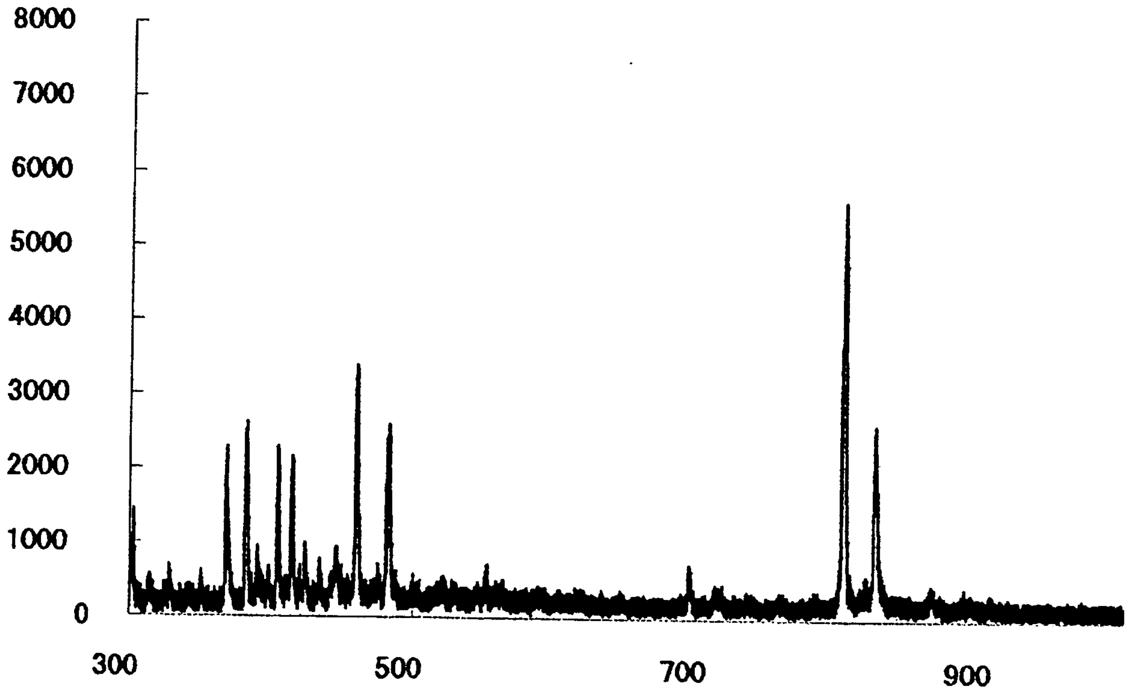
8000
7000
6000
5000
4000
3000
2000
1000
0
300
500
700
900

FIG. 11

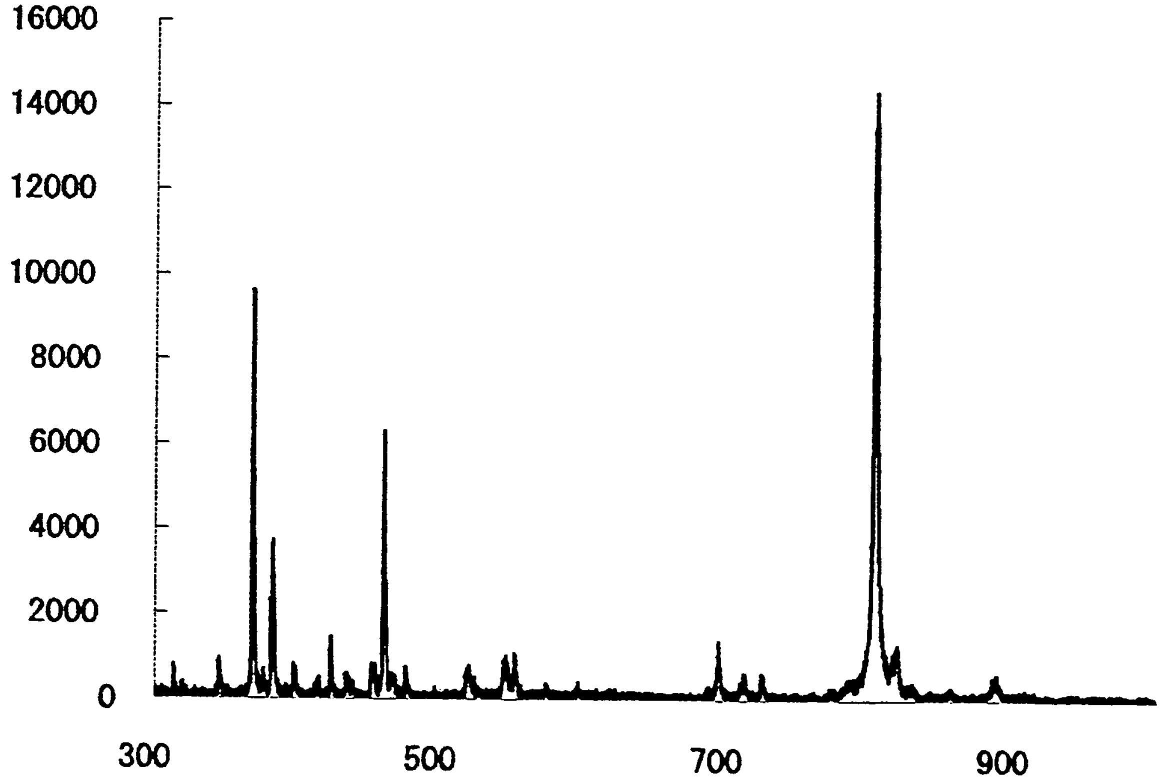
16000
14000
12000
10000
8000
6000
4000
2000
0
300
500
700
900

FIG. 12

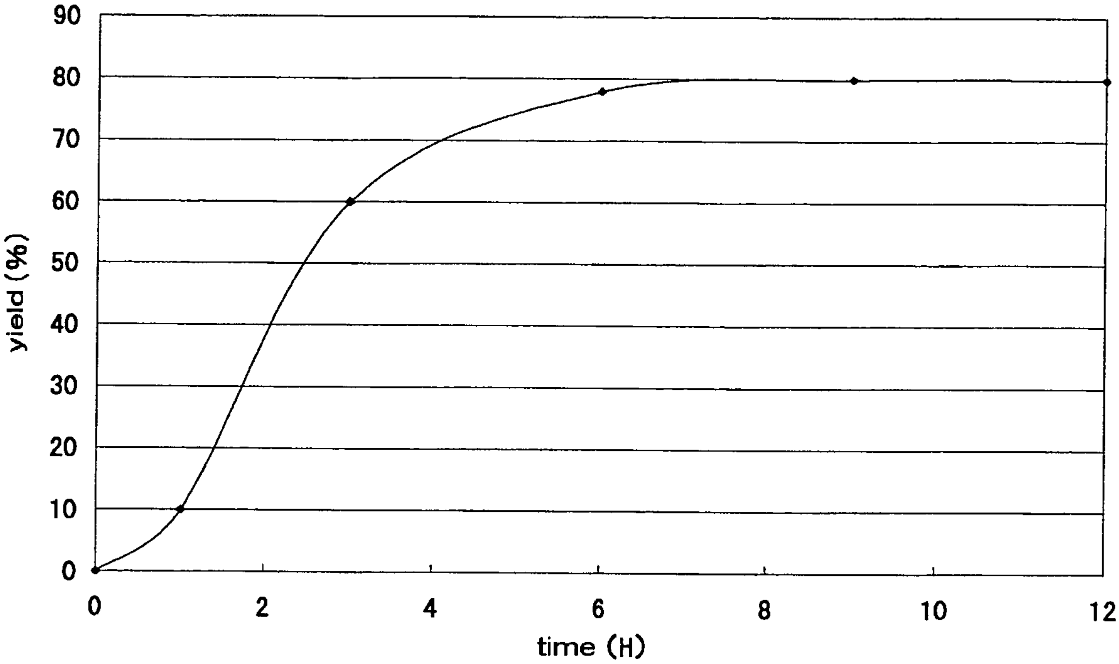
90
80
70
60
50
40
30
20
10
0
yield (%)
0
2
4
6
8
10
12
time (H)

FIG. 13

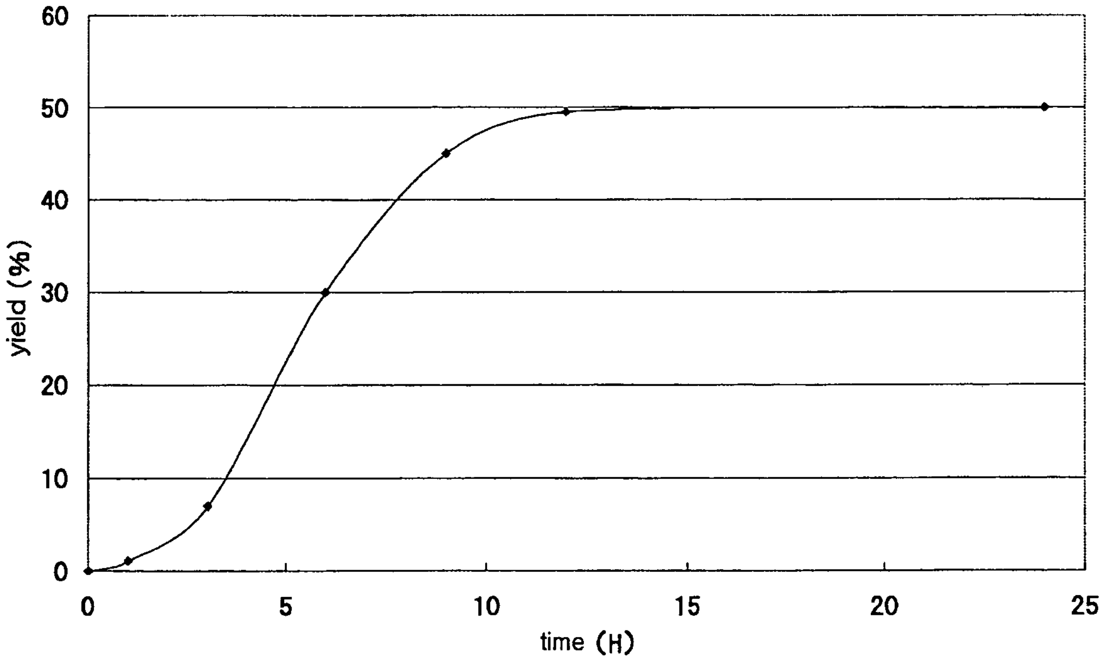
60
50
40
30
20
10
0
yield (%)
0
5
10
15
20
25
time (H)

FIG. 14

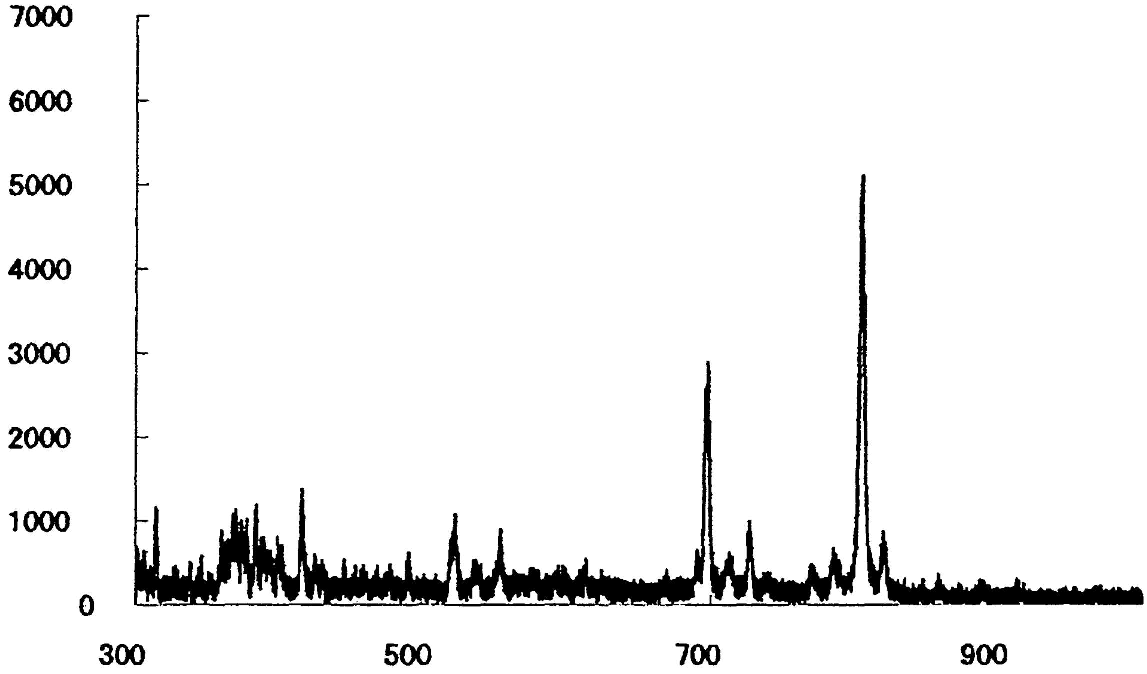
7000
6000
5000
4000
3000
2000
1000
0
300
500
700
900

FIG. 15

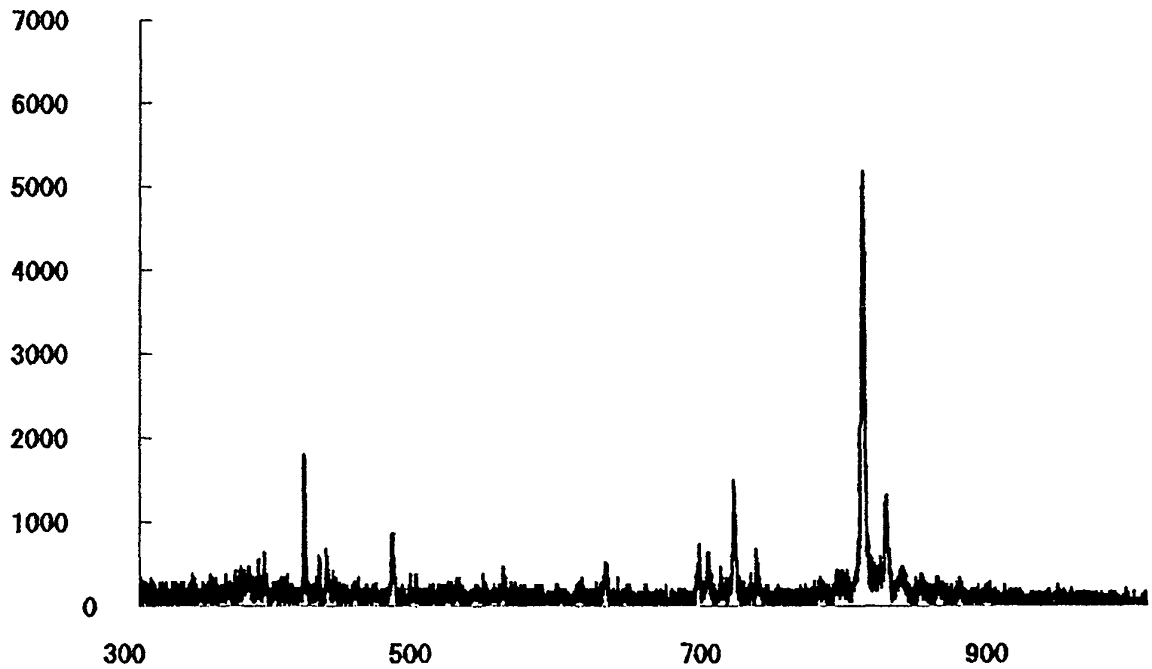
7000
6000
5000
4000
3000
2000
1000
0
300
500
700
900

FIG. 16

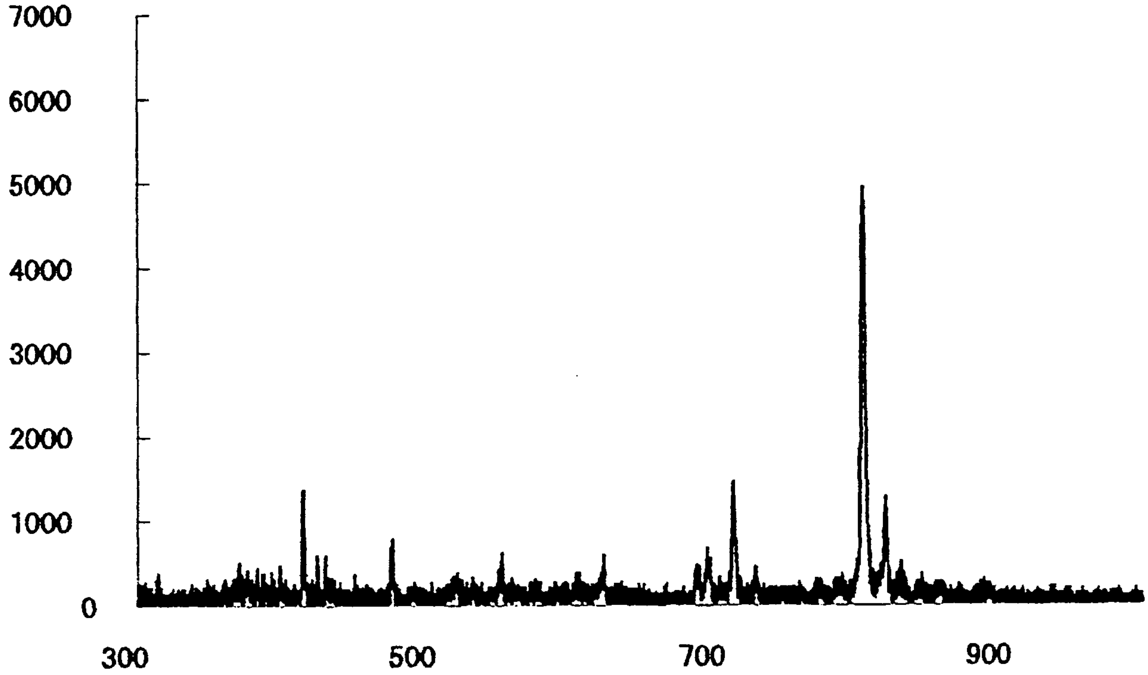
7000
6000
5000
4000
3000
2000
1000
0
300
500
700
900

FIG. 17

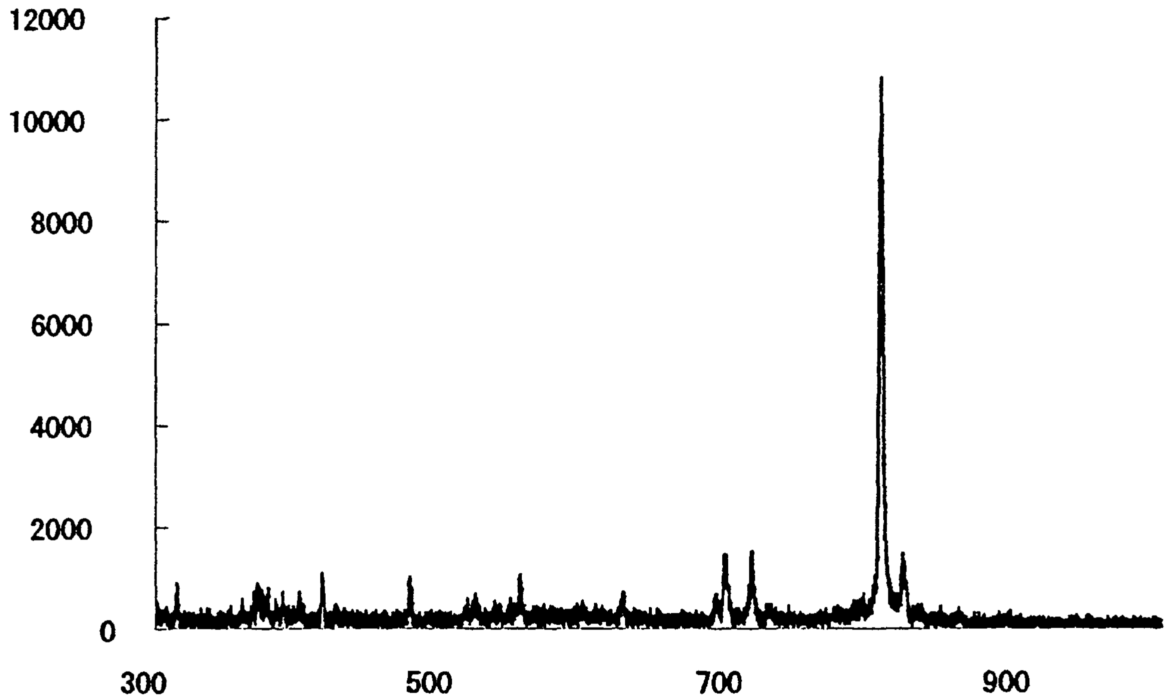
12000
10000
8000
6000
4000
2000
0
300
500
700
900

FIG. 18

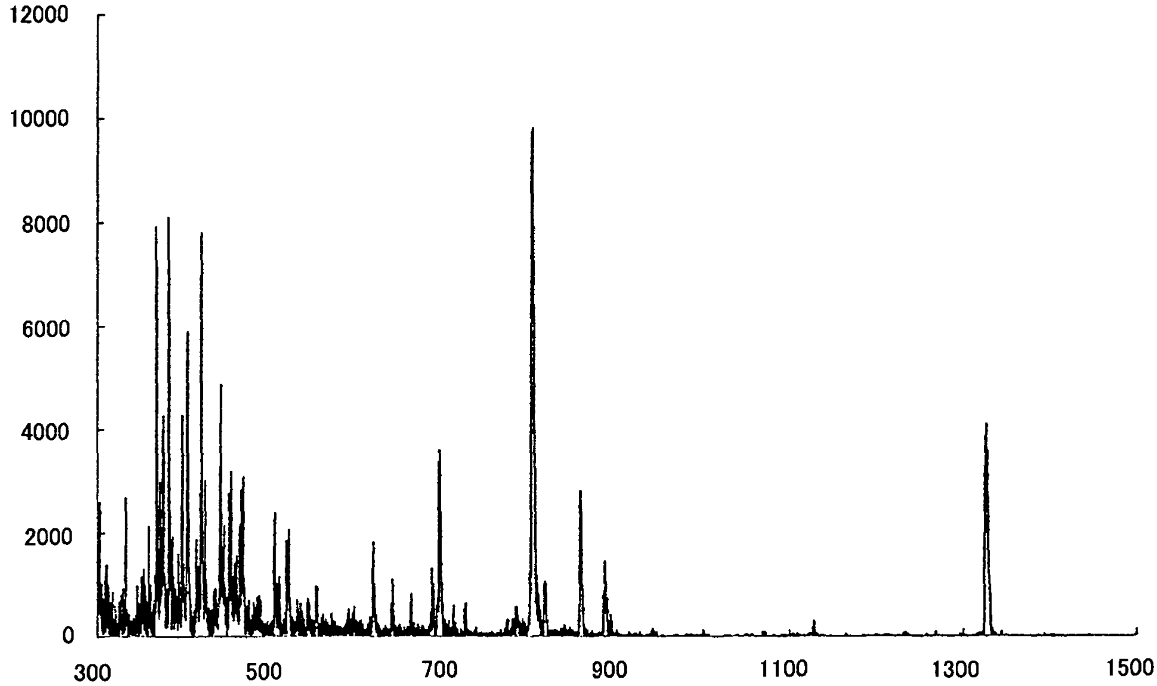
12000
10000
8000
6000
4000
2000
0
300
500
700
900
1100
1300
1500

FIG. 19

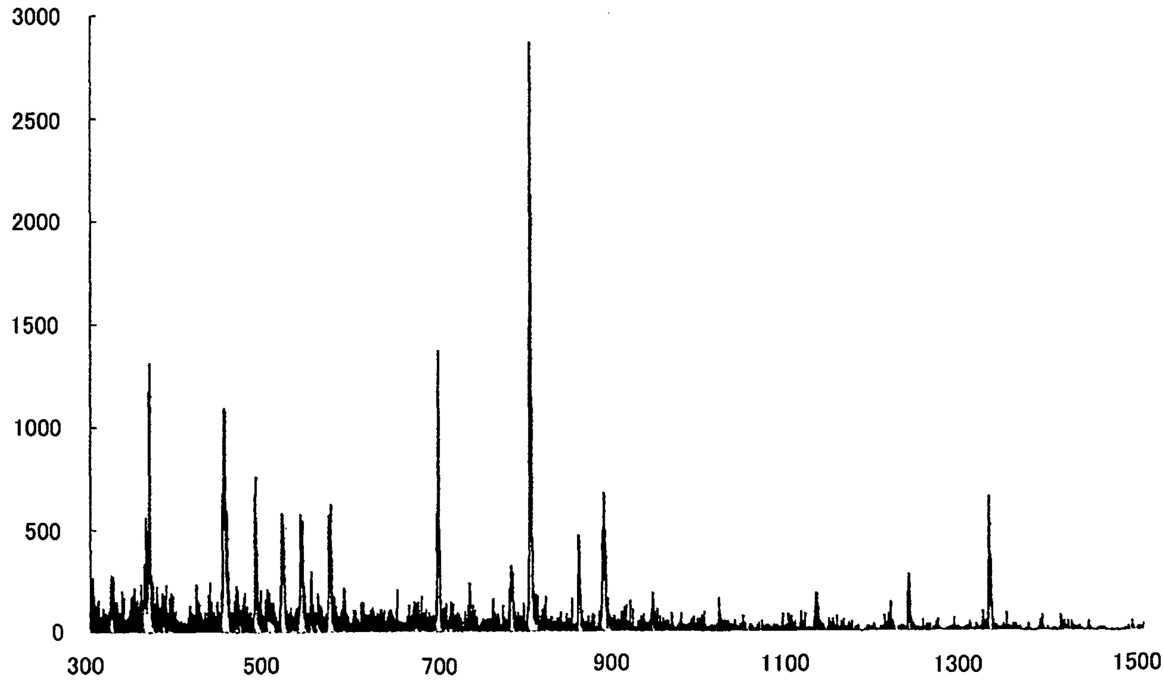
3000
2500
2000
1500
1000
500
0
300
500
700
900
1100
1300
1500

FIG. 20

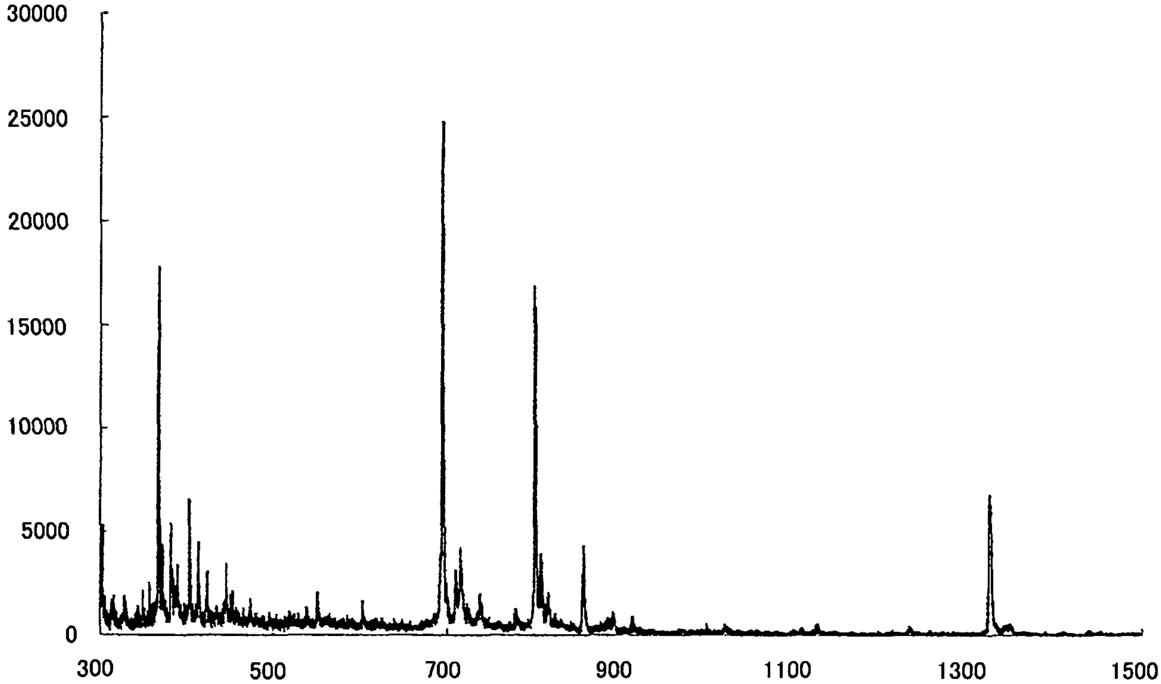
30000
25000
20000
15000
10000
5000
0
300
500
700
900
1100
1300
1500

FIG. 21

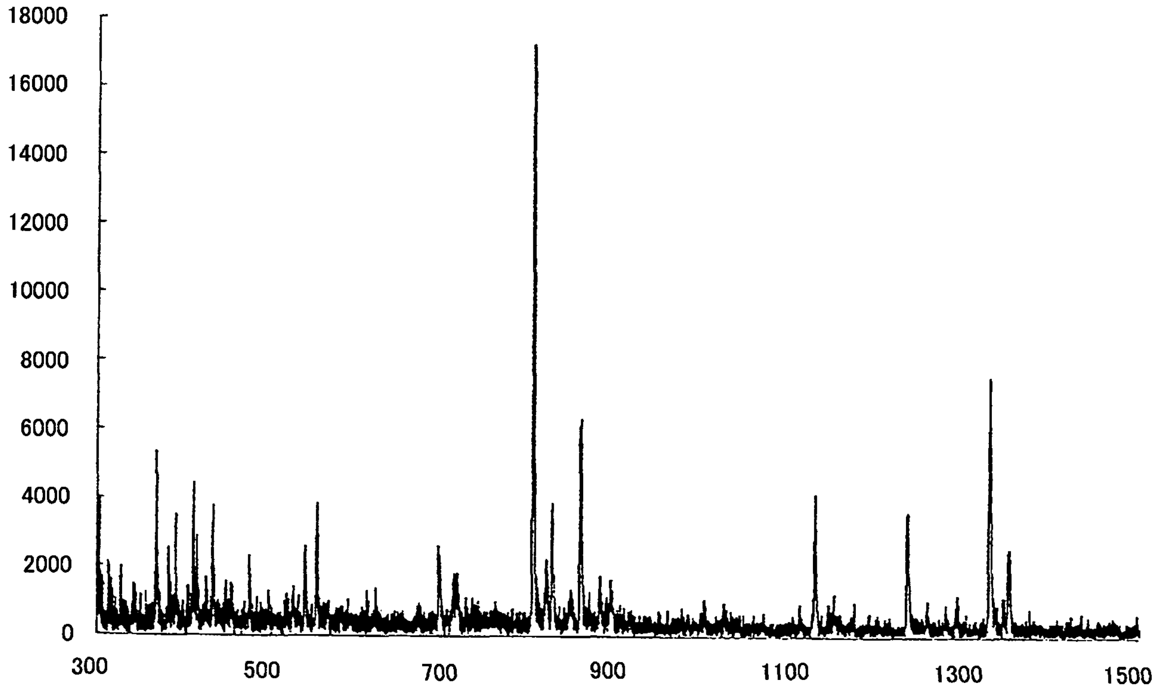
18000
16000
14000
12000
10000
8000
6000
4000
2000
0
300
500
700
900
1100
1300
1500

FIG. 22

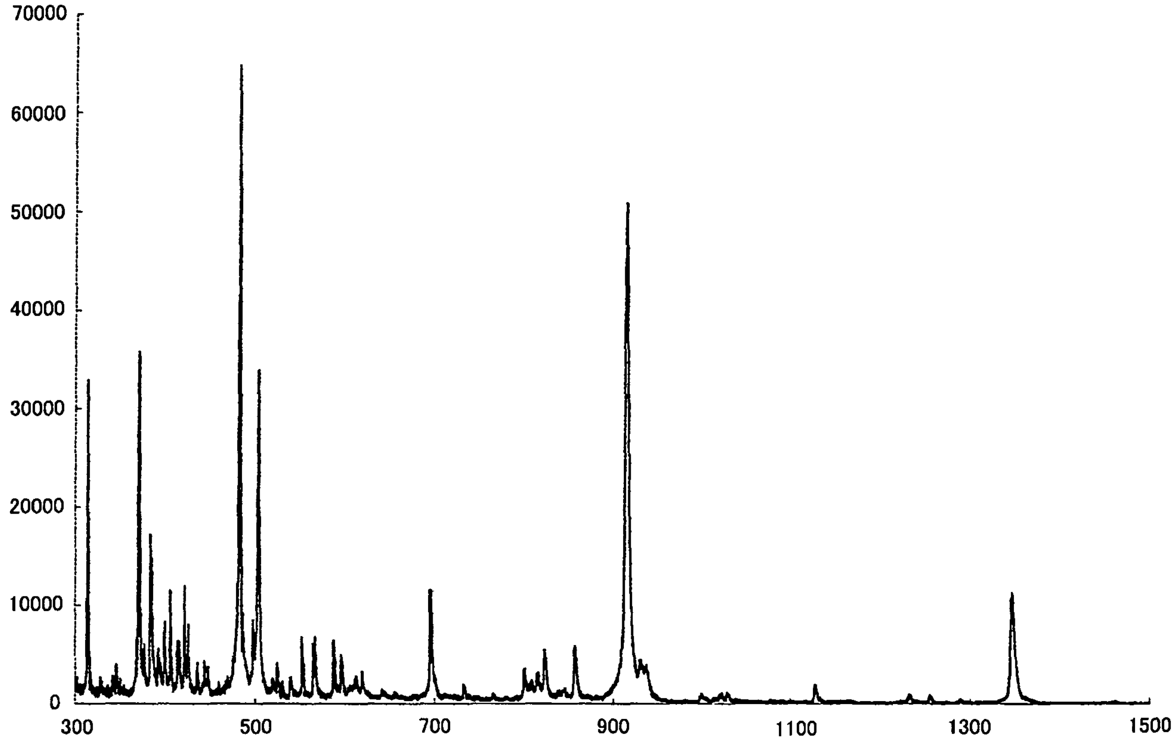
70000
60000
50000
40000
30000
20000
10000
0
300
500
700
900
1100
1300
1500

FIG. 23

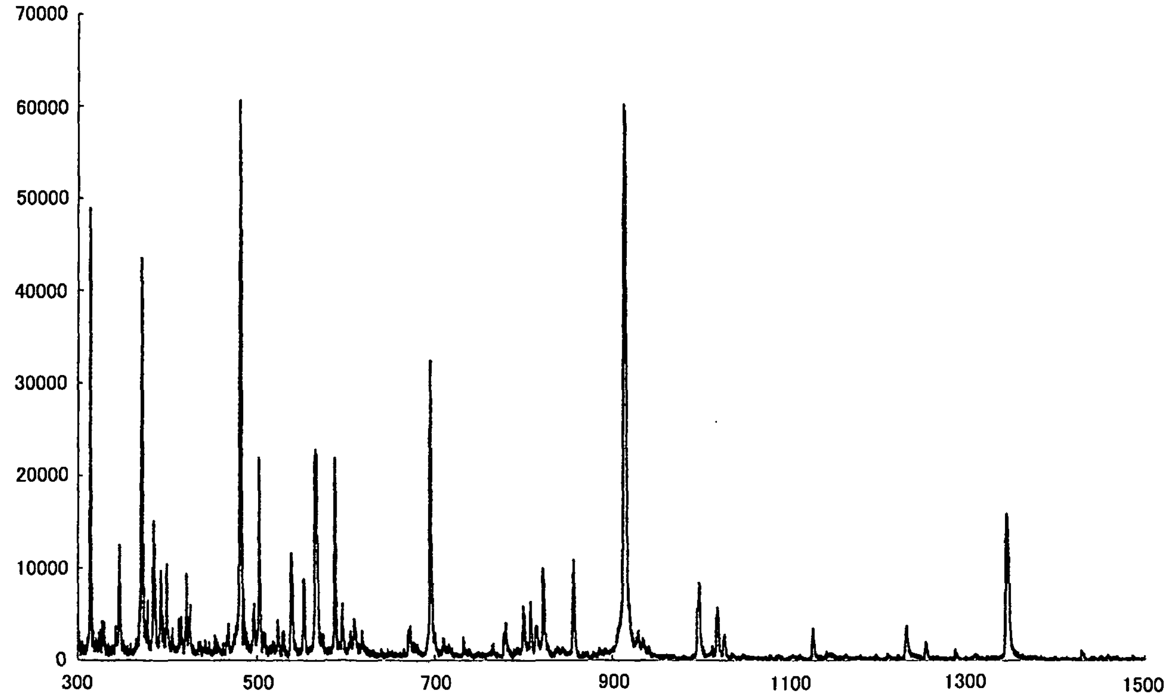
70000
60000
50000
40000
30000
20000
10000
0
300
500
700
900
1100
1300
1500

FIG. 24

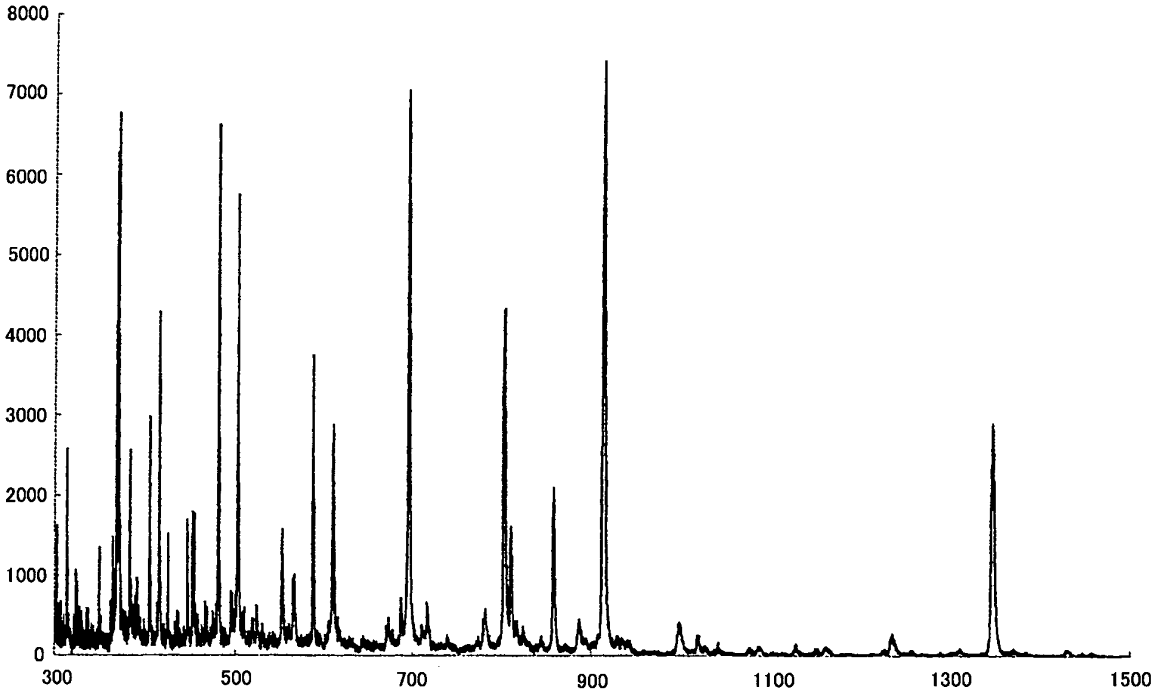
8000
7000
6000
5000
4000
3000
2000
1000
0
300
500
700
900
1100
1300
1500

FIG. 25

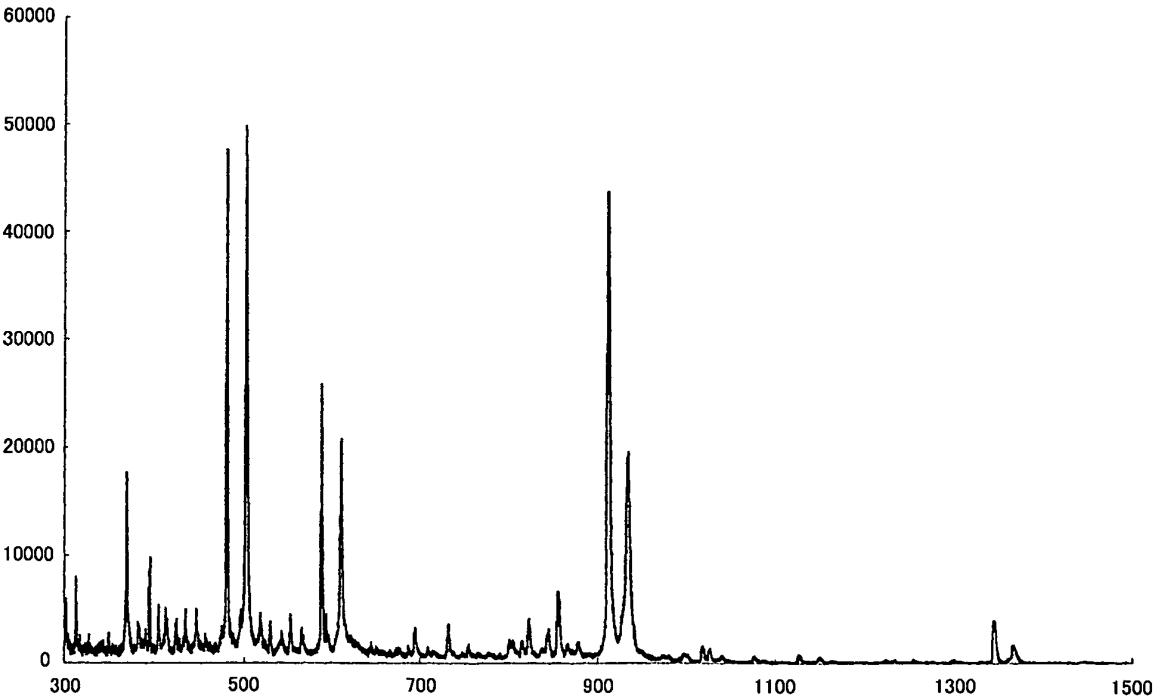
60000
50000
40000
30000
20000
10000
0
300
500
700
900
1100
1300
1500

**INTERNATIONAL SEARCH REPORT**

International application No.
PCT/JP2004/009523

A. CLASSIFICATION OF SUBJECT MATTER
Int.Cl[7] C07H1/00, 15/04, 15/18, 15/203, 3/06

According to International Patent Classification (IPC) or to both national classification and IPC

B. FIELDS SEARCHED

Minimum documentation searched (classification system followed by classification symbols)
Int.Cl[7] C07H1/00, 3/00-3/10, 15/00-15/26

Documentation searched other than minimum documentation to the extent that such documents are included in the fields searched

Electronic data base consulted during the international search (name of data base and, where practicable, search terms used)
WPI(STN), CAPLUS(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>A | Osamu KANIE et al., Orthogonal glycosylation strategy in synthesis of extended blood group B determinant, Tetrahedron Letters, 1996, Vol.37, pages 4551 to 4554; full text | 11<br>1-10,12-20 |
| X<br>A | Takashi, TAKAHASHI et al., Combinatorial synthesis of trisaccharides via solution-phase one-pot glycosylation, Tetrahedron Letters, 2000, Vol.41, pages 2599 to 2603; full text | 11,18,19<br>1-10,12-17, 20 |
| A | WO 02/016384 A2 (MASSACHUSETTS INSTITUTE OF TECHNOLOGY), 28 February, 2002 (28.02.02), & US 2002/085964 A1 & EP 1315559 A2 & AU 2001086539 A | 1-20 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 September, 2004 (10.09.04) | Date of mailing of the international search report<br>28 September, 2004 (28.09.04) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2004/009523

C (Continuation). DOCUMENTS CONSIDERED TO BE RELEVANT

| Category* | Citation of document, with indication, where appropriate, of the relevant passages | Relevant to claim No. |
|---|---|---|
| A | JP 8-511234 A (The Trustees of Princeton University), 26 November 1996 (26.11.96), & US 5639866 A & US 5635612 A & EP 686160 A1 & AU 9461382 A1 & IN 182498 A | 1-20 |

Form PCT/ISA/210 (continuation of second sheet) (January 2004)

**INTERNATIONAL SEARCH REPORT**

International application No.
PCT/JP2004/009523

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
(See extra sheet.)

1. ☒ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☒ No protest accompanied the payment of additional search fees.

Form PCT/ISA/210 (continuation of first sheet (2)) (January 2004)

**INTERNATIONAL SEARCH REPORT**

International application No.
PCT/JP2004/09523

Continuation of Box No.III of continuation of first sheet(2)

I. The special technical feature of the inventions as set forth in claims 1 to 10 relates to, in a method of solid phase synthesis of a sugar chain, the temperature of a reaction system is changed in accordance with a temperature-rising rate that has been determined under specific conditions. The invention as set forth in claim 11 relates to a monosaccharide library to be used in the method as set forth in claim 1. The inventions as set forth in claims 18 and 19 depending on claim 1 relate to a sugar chain library obtained by the method as set forth in claim 1.

II. The special technical feature of the inventions as set forth in claims 12 to 17 relates to a method of carrying out a solid phase reaction in a solid pore having such a size as inducing capillarity wherein the external surface of the solid is in the state of having no excessive liquid phase. The inventions as set forth in claims 18 and 19 depending on claim 12 relate to a sugar chain library obtained by the method as set forth in claim 12. The invention as set forth in claim 20 relates to an apparatus to be used for carrying out the method as set forth in claim 12.

Such being the case, it does not appear that there is a technical relationship between these invention groups I and II involving one or more of the same or corresponding special technical features and thus these groups of inventions are not considered as being so linked as to form a single general inventive concept.